(19) Europäisches Patentamt / European Patent Office / Office européen des brevets

(11) **EP 3 802 856 B1**

(12) **EUROPEAN PATENT SPECIFICATION**

(45) Date of publication and mention
of the grant of the patent:
**20.08.2025 Bulletin 2025/34**

(21) Application number: **19811297.1**

(22) Date of filing: **31.05.2019**

(51) International Patent Classification (IPC):
*C12Q 1/6827* (2018.01)     *C12Q 1/6876* (2018.01)
*C12Q 1/6883* (2018.01)     *G16B 50/20* (2019.01)
*G16H 50/30* (2018.01)

(52) Cooperative Patent Classification (CPC):
**G16H 50/20; C12Q 1/6883; G16B 20/00;**
**G16H 50/30;** C12Q 2600/136; C12Q 2600/154;
Y02A 90/10

(86) International application number:
**PCT/US2019/034829**

(87) International publication number:
**WO 2019/232320 (05.12.2019 Gazette 2019/49)**

(54) **DNA METHYLATION BIOMARKER OF AGING FOR HUMAN EX VIVO AND IN VIVO STUDIES**

DNA-METHYLIERUNGSBIOMARKER DER ALTERUNG FÜR EX-VIVO- UND
IN-VIVO-HUMANSTUDIEN

BIOMARQUEUR DU VIEILLISSEMENT BASÉ SUR LA MÉTHYLATION DE L'ADN POUR DES
ÉTUDES EX VIVO ET IN VIVO SUR L'HUMAIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2018 US 201862678730 P**

(43) Date of publication of application:
**14.04.2021 Bulletin 2021/15**

(73) Proprietor: **THE REGENTS OF THE UNIVERSITY
OF CALIFORNIA**
**Oakland, CA 94607-5200 (US)**

(72) Inventor: **HORVATH, Stefan**
**Los Angeles, California 90049 (US)**

(74) Representative: **Murgitroyd & Company**
**165-169 Scotland Street**
**Glasgow G5 8PL (GB)**

(56) References cited:
WO-A1-2014/146793     WO-A1-2018/027228
US-A1- 2009 099 037     US-A1- 2016 222 448
US-A1- 2016 222 448     US-A1- 2017 342 496

• CHRISTENSEN ET AL.: "Aging and
Environmental Exposures Alter Tissue-Specific
DNA Methylation Dependent upon CpG Island
Context", PLOS ONE, vol. 5, no. 8, 14 August
2009 (2009-08-14), pages 1 - 13, XP055659601
• ZUBAKOV ET AL.: "Estimating human age from
T-cell DNA rearrangements", CURRENT
BIOLOGY, vol. 20, no. 22, 23 November 2010
(2010-11-23), pages 970 - 971, XP028817175

EP 3 802 856 B1

**Description**

TECHNICAL FIELD

The invention relates to methods and materials for examining biological aging.

BACKGROUND OF THE INVENTION

Studies in invertebrates (yeast, worm, flies) have led to a long list of pharmacological agents that promise to intervene in different aspects of the aging process including stress response mimetics, anti-inflammatory interventions, epigenetic modifiers, neuroprotective agents, hormone treatments. While our arsenal of potential anti-aging interventions is brimming with highly promising candidates that delay aging in model organisms, it remains to be seen whether these interventions delay aging in human cells. To facilitate effective in vitro and *ex vivo* studies, there is a need for robust biomarkers of aging for human fibroblasts and other widely used cell types.

One potential biomarker that has gained significant interest in recent years is DNA methylation (DNAm). Chronological time has been shown to elicit predictable hypo- and hyper-methylation changes at many regions across the genome (see, e.g. Fraga et al., Trends in Genetics. 2007; 23(8):413-418, Rakyan et al., Genome research. 2010; 20(4):434-439, Teschendorff et al., Genome research. 2010; 20(4):440-446, Jung et al., BMC biology. 2015; 13(1):1-8 and Zheng et al., Epigenomics. 2016; 8(5):705-719). Several DNAm based biomarkers of aging have been developed (see, e.g., Bocklandt et al., PLoS One. 2011; 6(6): e14821, Garagnani et al., Aging Cell. 2012; 11(6):1132-1134, Hannum et al., Mol Cell. 2013; 49(2):359-367, Horvath, Genome Biol. 2013; 14(10):R115, Weidner et al., Genome Biol. 2014; 15(2):R24, Lin et al., Aging (Albany NY). 2016; 8(2):394-401, and Horvath et al., Nat Rev Genet. 2018) including the blood-based algorithm by Hannum (Hannum et al., Mol Cell. 2013; 49(2):359-367) and the multi-tissue algorithm by Horvath (Horvath, Genome Biol. 2013; 14(10):R115). These epigenetic age estimators exhibit statistically significant associations with many age-related diseases and conditions (see, e.g., Horvath et al., Proc Natl Acad Sci U S A. 2014; 111(43):15538-15543, Marioni et al., Genome Biol. 2015; 16(1):25, Marioni et al., Int J Epidemiol. 2015; 44(4):1388-1396, Horvath S, Garagnani P, Bacalini MG, Pirazzini C, Salvioli S, Gentilini D, Di Blasio AM, Giuliani C, Tung S, Vinters HV and Franceschi C. Accelerated epigenetic aging in Down syndrome. Aging Cell. 2015; 14(3):491-495, Horvath et al., J Infect Dis. 2015; 212(10):1563-1573, Levine et al., Aging (Albany NY). 2015; 7(9):690-700, Levine et al., Aging (Albany NY). 2015; 7(12):1198-1211, Levine et al.,. Proc Natl Acad Sci U S A. 2016; 113(33):9327-9332, Chen et al., Aging (Albany NY). 2016; 8(9):1844-1865, Quach et al., Aging (Albany NY). 2017; 9(2):419-446, Dugue et al., Int J Cancer. 2017, Simpkin et al., Int J Epidemiol. 2017; 46(2):549-558, and Maierhofer et al., Aging (Albany NY). 2017; 9(4):1143-1152).

Recently developed DNA methylation-based biomarkers allow one to estimate the epigenetic age of an individual (see, e.g., Bocklandt et al., PLoS One. 2011; Hannum, Mol Cell. 2013; Horvath, Genome Biol. 2013; 14(R115); and Weidner, Genome Biol. 2014). For example, the pan tissue epigenetic clock, which is based on 353 dinucleotide markers, known as CpGs (-C-phosphate--G-), can be used to estimate the age of most human cell types, tissues, and organs (Horvath, Genome Biol. 2013; 14(R115)). The estimated age, referred to as "DNA methylation age" (DNAm age). correlates with chronological age when methylation is assessed in sorted cell types (CD4+ T cells, monocytes, B cells, glial cells, neurons), tissues, and organs including whole blood, brain, breast, kidney, liver, lung, and saliva. Other reports described DNAm-based biomarkers that pertain to a single tissue (e.g. saliva or blood). Recent studies suggested that DNAm-based biomarkers of age capture aspects of biological age. For example, we and others have previously shown that individuals whose DNAm age was greater than their chronological age, i.e. individuals who exhibited epigenetic "age acceleration". were at an increased risk for death from all causes, even after accounting for known risk factors (see, e.g., Marioni et al., Genome Biol. 2015; 16(1):25, Christiansen et al., Aging Cell. 2015, and Perna et al., Clinical Epigenetics. 2016; 8(1):1-7).

WO2018027228 discloses a method for detection of age of an individual by analysing the methylation pattern of specific markers in a tissue sample from an individual, such as a blood sample, by using bisulfite treatment.

Also US2016222448 discloses a method of determining the age by analysing biological sample, such as blood sample, for a methylation pattern of specific markers using bisulfite treatment, as the age indicator.

There is a need for improved methods of observing phenomena associated with aging, independent of chronological age and traditional risk factors of mortality.

SUMMARY OF THE INVENTION

Although biological age is an intuitive concept, there is considerable debate in the literature on how to measure it. Here we describe a new DNA methylation based biomarker that accurately measures the age of human fibroblasts, keratinocytes, buccal cells, endothelial cells, skin, dermis, epidermis, saliva, lymphoblastoid cells, and blood samples. The biomarker is well suited for studying whether a given intervention increases, slows, or even reverses aging in *ex vivo* studies such as fibroblast-, keratinocyte-, endothelial-, or lymphoblastoid cell culture systems. For example, we demonstrate that cell population doubling levels are generally positively associated with epigenetic aging, rapamycin slows epigenetic aging in dividing keratinocytes, and human TERT immortalization does not slow epigenetic aging in dividing fibroblasts and endothelial cells.

The invention disclosed herein provides a novel and powerful estimator of the age of cells that is applicable to human cell types that are widely used in vitro studies and *ex vivo* studies (including fibroblasts, keratinocytes, endothelial cells). Its accuracy with respect to estimating age far exceeds existing molecular measurements including existing DNAm based biomarkers. Further, the biomarker also stands out in terms of its accuracy for measuring age based on blood samples, buccal swabs, skin samples, dermis, epidermis. Our epidemiological studies demonstrate that an age adjusted measure of DNAm age in blood also predicts human lifespan.

Embodiments of the invention include methods of observing the effects of one or more test agents on epigenetic aging in human cells. Typically, these methods comprise combining the test agent(s) with human cells (e.g. for specified period of time such as at least one day, one week or one month), and then observing methylation status in all of the 391 methylation markers of SEQ ID NO: 1-SEQ ID NO: 391 in genomic DNA from the human cells. These methods then compare the observations from human cells exposed to the test agent with observations of the methylation status in all of the 391 methylation markers of SEQ ID NO: 1-SEQ ID NO: 391 in genomic DNA from control human cells not exposed to the test agent such that effects of the test agent on epigenetic aging of human cells is observed. Optionally, the test agent is a compound having a molecular weight less than 3,000, 2,000, 1,000 or 500 g/mol, a polypeptide, a polynucleotide or the like. In certain embodiments of the invention, the cells are primary keratinocytes obtained from multiple donors. Typically, the methods observe human cells *in vitro* in cell culture studies.

Apart from cell culture studies, the biomarker can be used to accurately measure the age of an individual based on DNA extracted from skin, dermis, epidermis, blood, saliva, buccal swabs, and endothelial cells. Thus, the biomarker can also be used for forensic and biomedical applications involving human specimens. The biomarker stands out with respect to its ability to accurately estimating the age of an individual based on skin cells, buccal cells, blood, or endothelial cells. It applies to the entire age span from samples from newborns (e.g. cord blood samples) to centenarians.

Embodiments of the invention provide useful biomarkers for *ex vivo* studies of anti-aging interventions, thus allowing interventions to be quickly evaluated based on real-time measures of aging, rather than human clinical studies. Embodiments of the invention are also useful for applications in personalized medicine, as it allows for evaluation of accelerated aging effects based on DNA measurements. Embodiments of the invention can also be used for forensic applications involving human specimens. Similarly, embodiments of the invention can be used, for example, for age assessment in applicants seeking asylum. In particular, refugees seek asylum in different countries. Many applicants without proper paper work (lack of passport and birth certificate) claim to be younger than 18 since minor status confers advantages. The age estimator is highly accurate in adolescents based on a buccal swab, saliva sample, or blood sample. Thus, embodiments of the invention can be used to corroborate or refute the age claim of an asylum seeker.

Embodiments of the invention include methods of observing biomarkers in skin and blood cells that correlate with an age of an individual, the method comprising observing the individual's methylation status in all of the 391 methylation markers identified herein, so that biomarkers associated with the age of the individual are observed. Typically, the skin and blood cells are human fibroblasts, keratinocytes, buccal cells, endothelial cells, lymphoblastoid cells, and/or cells obtained from blood skin, dermis, epidermis or saliva. Embodiments of this method further comprise using the observations to estimate the age of the individual (e.g. using a regression analysis or the like). In some embodiments, the age of the individual is estimated using a weighted average of methylation markers within the set of 391 methylation markers. Certain embodiments of the invention include comparing the estimated age with the actual age of the individual so as to obtain information on health and/or life expectancy of the individual. Typically, methylation is observed by a process comprising treatment of genomic DNA from the population of cells from the individual with bisulfite to transform unmethylated cytosines of CpG dinucleotides in the genomic DNA to uracil and/or hybridizing genomic DNA obtained from the individual with 391 complementary sequences disposed in an array on a substrate.

In typical embodiments of the invention, the age estimate is calculated by aggregating the DNAm levels of 391 locations in the genome (known as cytosine-phosphate-guanines or CpGs). To use the epigenetic biomarker, one typically needs to extract DNA from cells or fluids, e.g. human fibroblasts, keratinocytes, buccal cells, skin samples, dermis, epidermis, blood cells, endothelial cells. Next, one needs to measure DNA methylation levels in the underlying signature of 391 CpGs (epigenetic markers) that are being used in the mathematical algorithm. The algorithm leads to an "age" estimate (for each sample or human subject). The higher the value, the older the cell or tissue sample. These recently developed DNA methylation-based biomarkers allow one to estimate the epigenetic age of an individual (see, e.g. Fraga et al., Trends in Genetics. 2007; 23(8):413-418, Rakyan et al., Genome research. 2010; 20(4):434-439, Teschendorff et al., Genome research. 2010; 20(4):440-446 and Jung et al., BMC biology. 2015; 13(1):1-8). For example, the "epigenetic clock", developed by Horvath, which is based on methylation levels of 353 CpGs, can be used to estimate the age of most human cell types, tissues, and organs (see, e.g., Teschendorff et al., Genome research. 2010; 20(4):440-446).

Other objects, features and advantages of the present invention will become apparent to those skilled in the art from the following detailed description. It is to be understood, however, that the detailed description and specific examples, while indicating some embodiments of the present invention, are given by way of illustration and not limitation. Many changes and modifications within the scope of the present invention may be made, and the invention includes all such modifications, in so far that fall within the scope defined by the appended claims.

BRIEF DESCRIPTION OF THE DRAWINGS

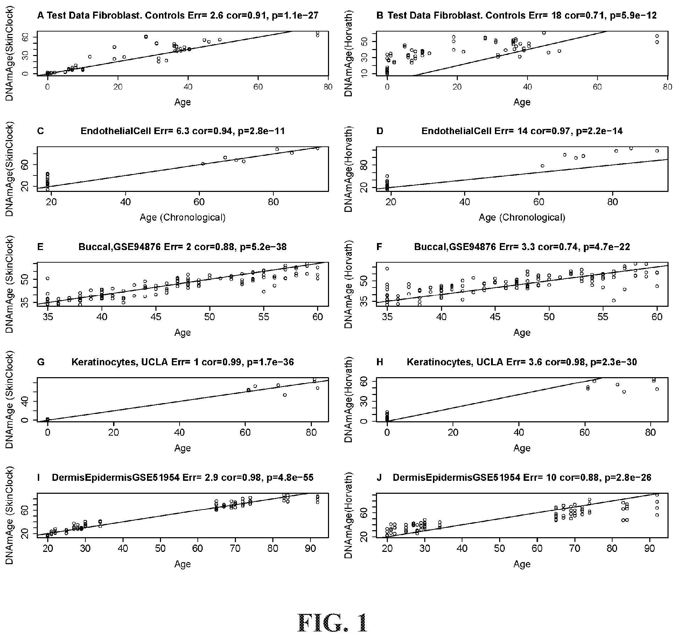

**Figure 1. Age estimation accuracy of the skin & blood clock in fibroblasts, keratinocytes, and endothelial cells.** Panels on the left-hand side and right-hand side, relate chronological age (x-axis) to DNAmAge estimates (y-axis) from the skin & blood clock (A,C,E,G,I) and the pan-tissue clock (Horvath 2013), respectively. Each row corresponds to a different tissue/cell type. DNA methylation data from A,B) fibroblasts, C,D) microvascular endothelial cells, E,F) buccal epithelial cells, G,H) keratinocytes, I,J) dermis/epidermis samples. Each panel reports the Pearson correlation coefficient and the error (defined as median absolute deviation between DNAm age and chronological age).

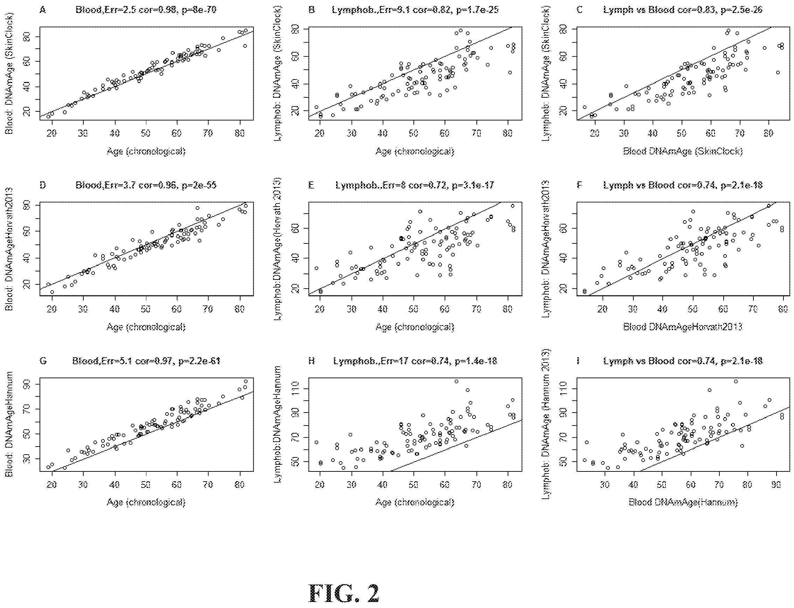

**Figure 2. Comparison of DNAm age estimators in whole blood and lymphoblastoid cell line data.** The rows correspond to 3 different age estimators: A,B,C) the novel skin & blood clock, D,E,F) the pan-tissue clock (Horvath 2013), G,H,I) Hannum clock. Panels in the first and second column report the accuracy in blood (A,D,C) and lymphoblastoid cell lines (B,E,H), respectively. Panels in the third column (C,F,I) report the relationship between DNAm age estimates in blood (x-axis) versus those in lymphoblastoid cell lines (y-axis). Panels report Pearson correlation coefficient and the estimation error, which is defined as median absolute deviation between the DNAm age estimate and chronological age. The lymphoblastoid cell lines were generated from the same individuals for whom whole blood was assessed, which facilitated the comparison in the third column.

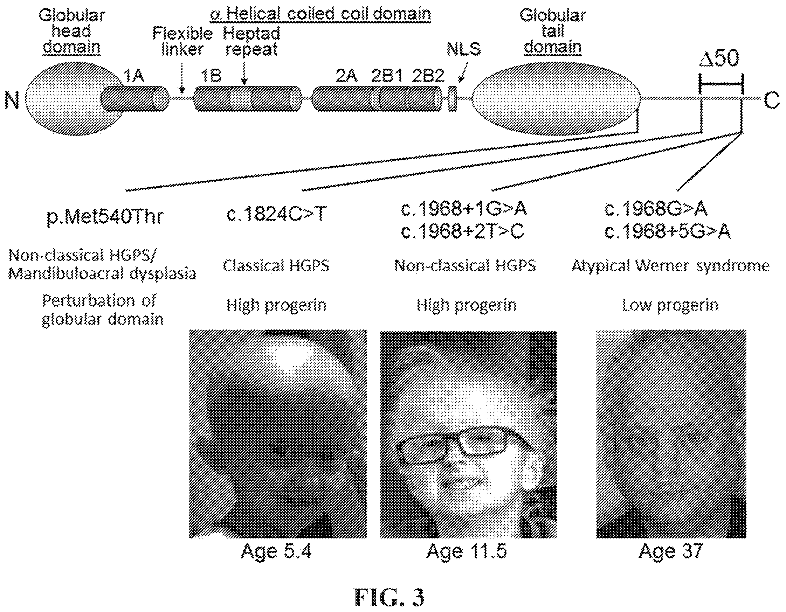

**Figure 3. LMNA mutations in progeria patients.** The diagram shows the structure of lamin A. It consists of globular head domain, linker regions, $\alpha$-helical coiled coil domain and globular tail domain. Locations of the progeria LMNA mutations in this study were shown with clinical phenotype and molecular mechanism of mutant lamin A protein, as previously reported in [34] (p.Met540Thr). [29] (c.1824C>T), [30] (c.1968+1G>A), [31] (c.1968+2T>C), and [36] (c.2968G>A and c.1968+5G>A). Δ50 indicates the region of deletion in progerin, also present in ZMPSTE24 mutant progeria [32].

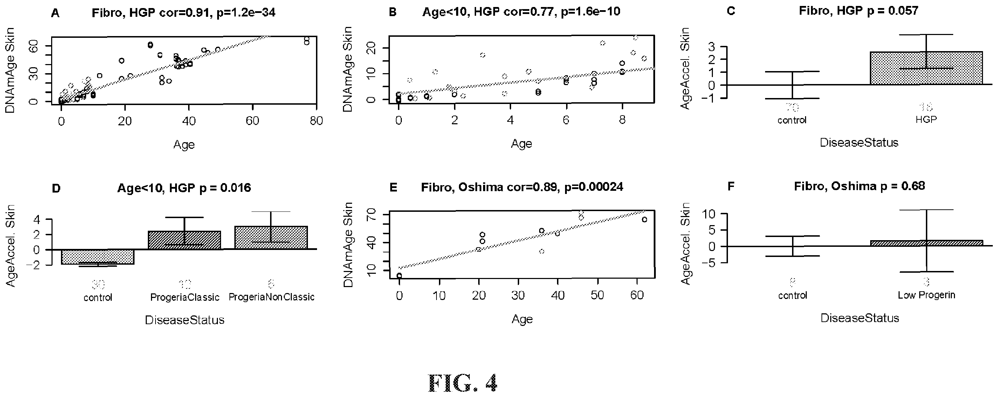

**Figure 4. Skin & blood clock analysis of fibroblasts from HGP individuals of the Progeria Research Foundation.** A,B) The new skin & blood clock was used to estimate DNAm age (y-axis) in fibroblasts from HGP individuals and controls. A) All individuals. B) Children younger than 10 years old). Dots are colored by disease status: red=classical progeria, green=non-classical progeria, black=controls. The grey line corresponds to a regression line through control individuals. The epigenetic age acceleration effect for each individual (point) corresponds to the vertical distance to the black regression line. The fact that red and green points tend to lie above the grey line indicates that HGP cases exhibit suggestive accelerated epigenetic aging effect. C) Mean epigenetic age acceleration (y-axis) versus HGP status. By definition, the mean age acceleration measure in controls is zero. The title of the bar plots also reports a P-value from a nonparametric group comparison test (Kruskal Wallis test). Each bar plot reports 1 SE.

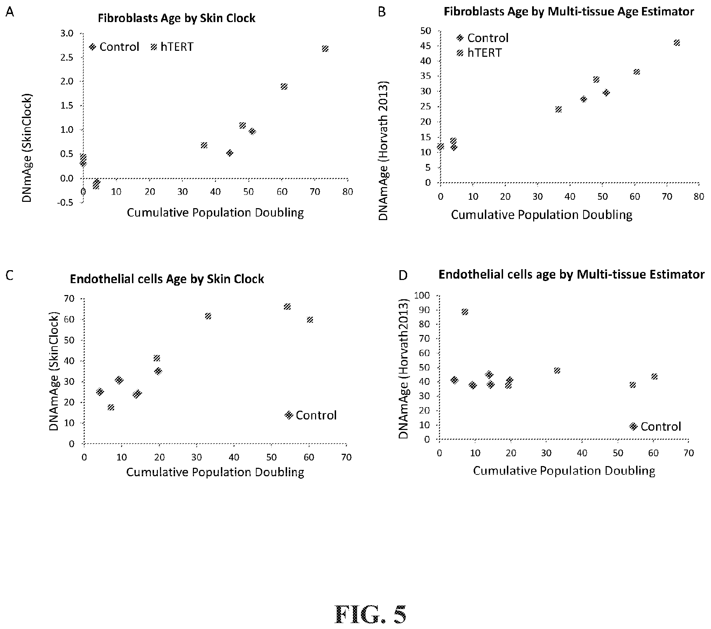

**Figure 5. DNAm age versus population doubling levels.** Each panel reports a DNAm age estimate (y-axis) versus cumulative population doubling level, respectively. Plots in the left panel and right panel correspond to the new skin & blood clock (A,C) and the pan-tissue clock (B,D). respectively. A,B) The growth of human primary fibroblasts from neonatal foreskin samples measured was measured as population doublings (x-axis). A,B) Neonatal foreskin samples (age zero). C,D) Results for endothelial cells from an adult individual. Dots are colored by hTERT status (red=hTERT expression, blue=control).

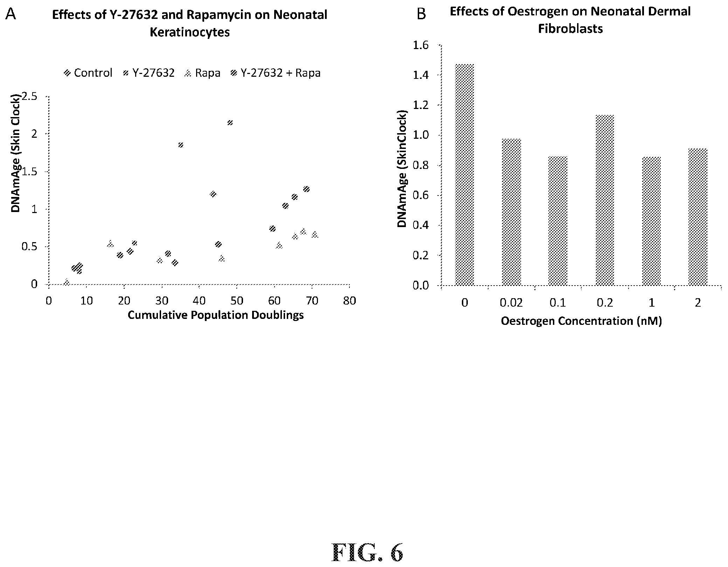

**Figure 6. Ex *vivo* study of compounds that may accelerate or decelerate epigenetic aging.** A) Effect of Y-27632 and rapamycin treatment on neonatal keratinocytes measured by the skin & blood clock. B) Effect of oestrogen on neonatal dermal fibroblasts.

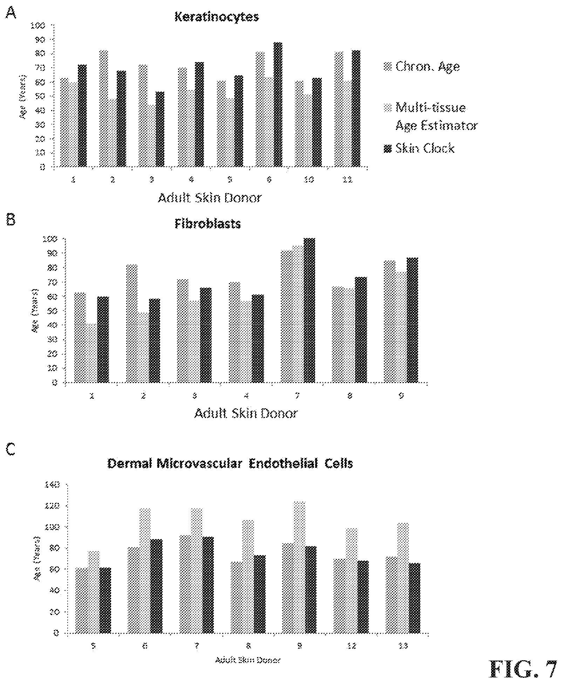

**Figure 7. Comparing the new skin & blood clock with the pan-tissue age estimator in different cell types.** The y-axis reports chronological age estimates based on DNA methylation levels from A) keratinocytes, B) fibroblasts and c) microvascular endothelial cells. The x-axis corresponds to different donors whose chronological ages are indicated by the orange bars. The age estimates of the skin & blood clock and the pan-tissue clock are colored in brown and green, respectively.

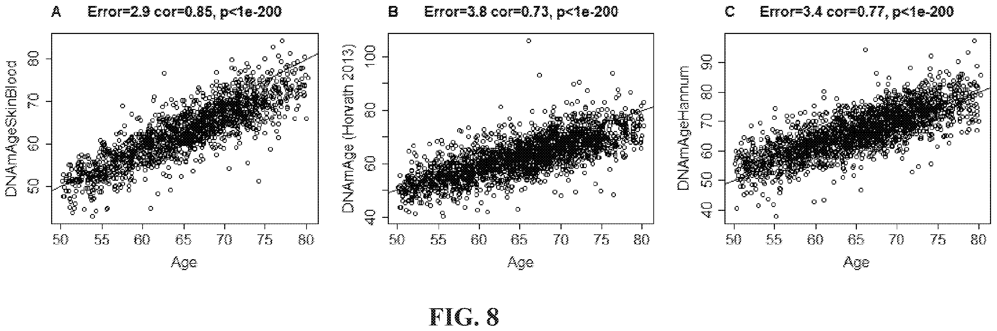

**Figure 8. Accuracy of different DNAm age estimators in blood from the WHI.** Age at blood draw (x-axis) versus DNAm age estimates from A) the novel skin & blood clock, B) the pan-tissue DNAm age estimator (Horvath 2013), C) DNAm age estimator by Hannum (2013). The DNA methylation data from participants of the Women's Health Initiative are described in [21, 49]. The error is defined as the median absolute deviation between chronological age and the age estimate.

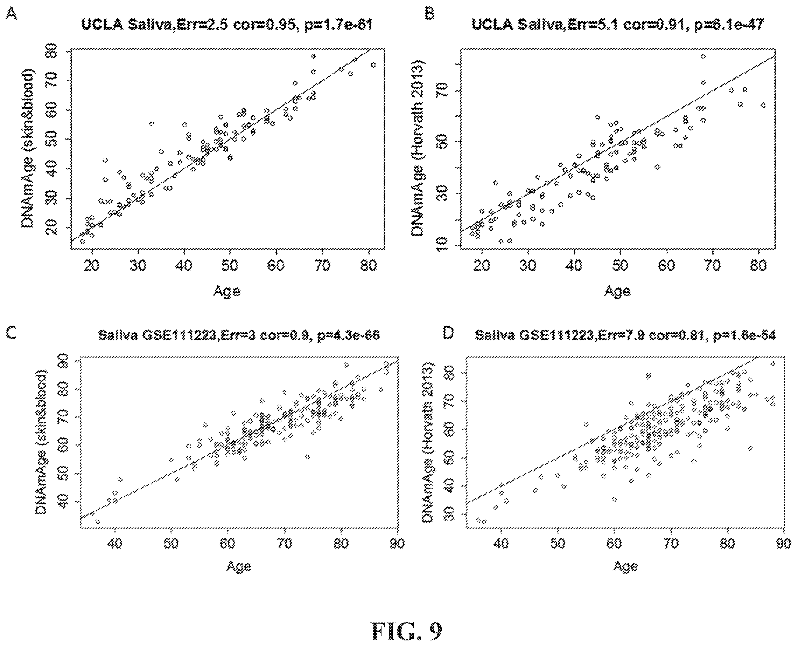

**Figure 9. Accuracy of different DNAm age estimators in two different saliva data sets.** Age at the collection of saliva samples (via a spit cup) (x-axis) versus DNAm age estimates from A,C) the novel skin & blood clock, B,D) the pan-tissue DNAm age estimator (Horvath 2013). The error is defined as the median absolute deviation between chronological age and the age estimate. Panels on the first and second row correspond to A,B) an Illumina 450K DNA methylation data set from UCLA and C,D) a publicly available DNA methylation data set (Gene Expression Omnibus identifier GSE111223) described in Horvath and Ritz 2015 [50].

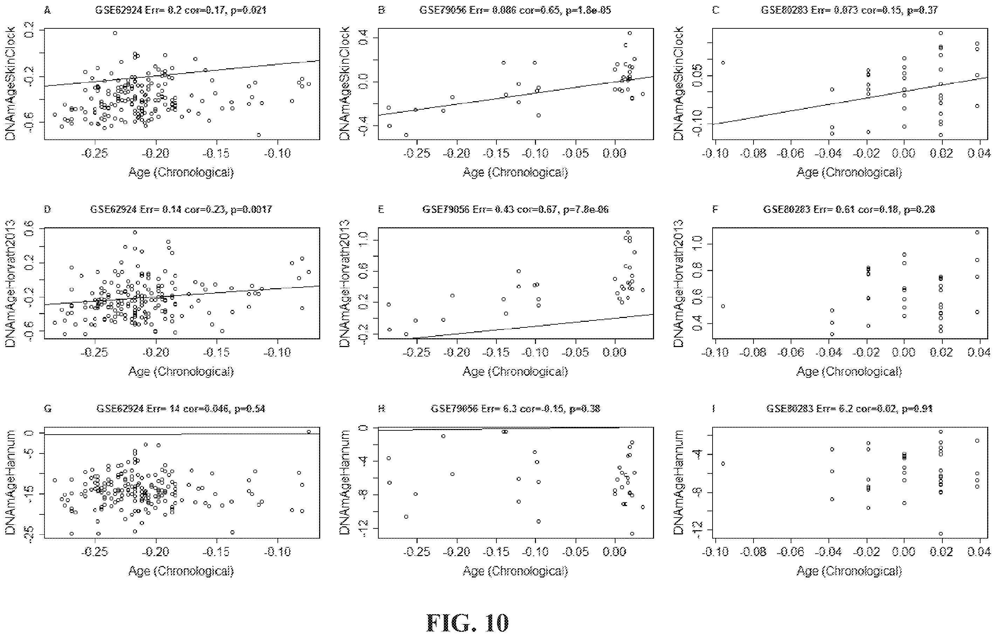

**Figure 10. Gestational age versus different DNAm age estimates from blood.** Age blood draw in units of years (x-axis) versus DNAm age estimates from A,B,C) the novel skin & blood clock, D,E,F) the pan-tissue DNAm age estimator (Horvath 2013), and G,H,I) the Hannum (2013) clock. Gestational Week was translated into units of years using the following formula Age=(Gestational Week-39)/52. The error is defined as the median absolute deviation between chronological age and the age estimate. Panels in the different columns correspond to three publicly available data sets: A,D,E) GEO identifier GSE62924 [51], B,E,H) Nashville birth cohort (GSE79056 [52],) C,F,I) Victorian Infant Collaborative Study GSE80283.

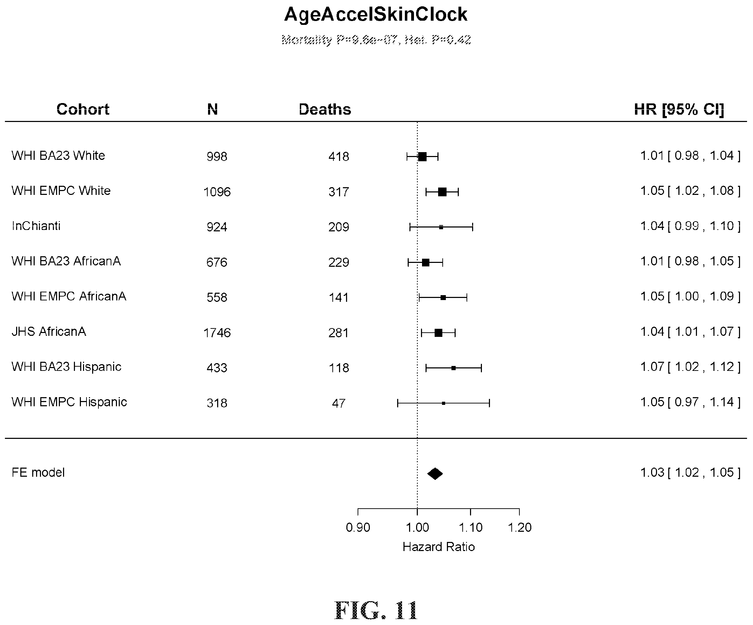

**Figure 11.** Univariate Cox regression meta-analysis of all-cause mortality (time to death). A univariate Cox regression model was used to relate the censored survival time (time to all-cause mortality) to epigenetic age acceleration (according to the skin & blood clock). The rows correspond to the different cohorts/racial groups. Each row depicts the hazard ratio and a 95% confidence interval. The coefficient estimates from the respective studies were meta-analyzed using a fixed-effect model weighted by inverse variance (implemented in the "metafor" R package [53]. The meta analysis p values (red sub-title) is highly significant p=9.6E-7. The p-value of the heterogeneity test (Cochran's Q-test) is not significant because the cohort-specific estimates do not differ substantially.

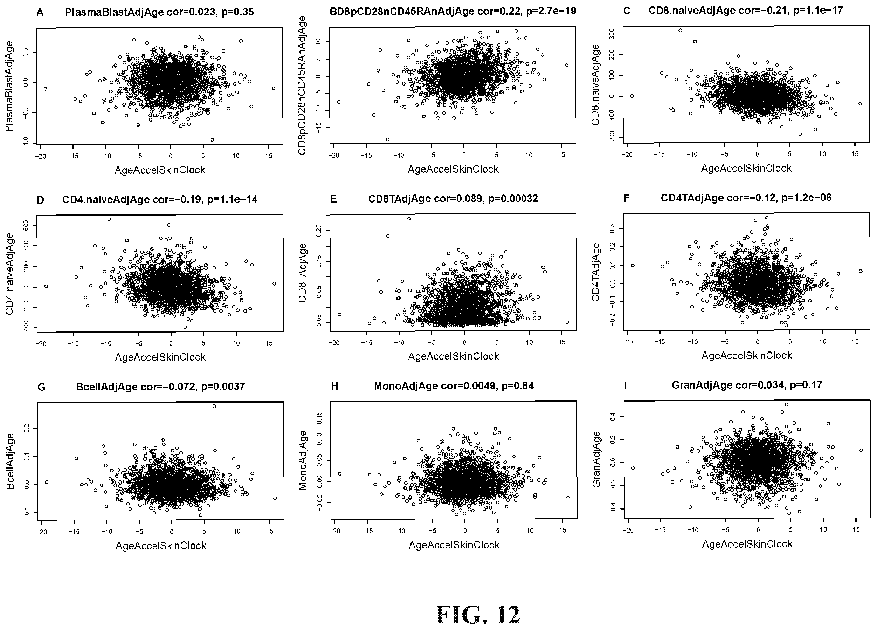

**Figure 12.** Relationship between epigenetic age acceleration and age adjusted estimates of various blood cell counts in the WHI (BA 23). Epigenetic age acceleration in blood (according to the skin & blood clock) versus age adjusted estimates of A) plasma blasts, B) exhausted CD8+ T cells, C) naive CD8+ T cells, D) naive CD4+ T cells, E) CD8+ T cells, F) CD4+ T cells, G) B cells, H) monocytes, I) granulocytes (mostly neutrophils). The blood cell counts were imputed based on the DNA methylation data using the Houseman method (Houseman 2012)[48] and the Horvath method (Horvath 2015)[17].

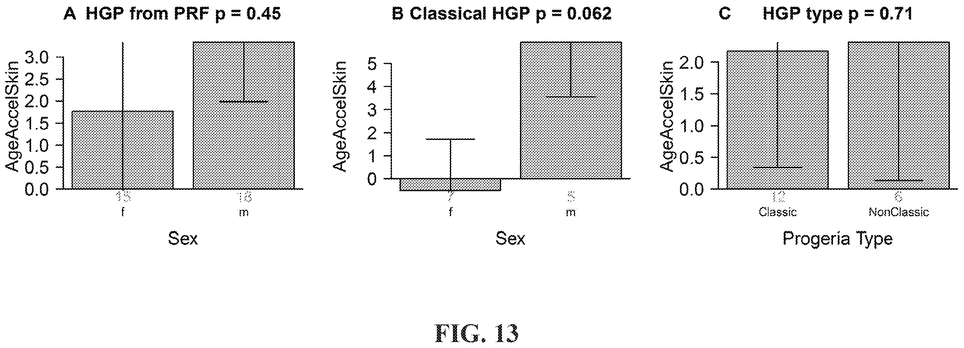

**Figure 13.** Detailed analysis of HGP fibroblast samples from the Progeria Research Foundation (PRF) . A) Sex (x-axis) versus epigenetic age acceleration in all HGP samples from the PRF (Table 2). B) Sex versus epigenetic age acceleration in classical HGP samples. C) Epigenetic age acceleration does not relate to progeria type (classical versus non-classical). Each bar plot reports the findings from a non-parametric group comparison test (Kruskal Wallis test). Each bar plot depicts the mean value of age acceleration and one standard error (error bars).

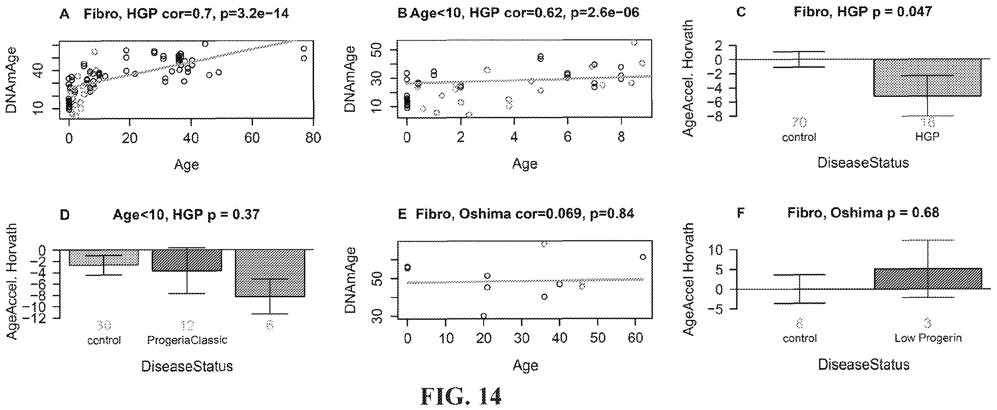

**Figure 14. Pan-tissue clock analysis of fibroblasts from HGP individuals of the Progeria Research Foundation.** A,B) The pan-tissue clock (Horvath 2013) was used to estimate DNAm age (y-axis) in fibroblasts from HGP individuals and controls. A) All individuals. B) Children younger than 10 years old). Dots are colored by disease status: red=classical progeria, green=non-classical progeria, black=controls. The grey line corresponds to a regression line through control individuals. The epigenetic age acceleration effect for each individual (point) corresponds to the vertical distance to the black regression line. The fact that red and green points tend to lie above the grey line indicates that HGP cases exhibit suggestive accelerated epigenetic aging effect. C) Mean epigenetic age acceleration (y-axis) versus HGP status. By definition, the mean age acceleration measure in controls is zero. The title of the bar plots also reports a P-value from a nonparametric group comparison test (Kruskal Wallis test). Each bar plot reports 1 standard error.

## DETAILED DESCRIPTION OF THE INVENTION

In the description of embodiments, reference may be made to the accompanying figures which form a part hereof, and in which is shown by way of illustration a specific embodiment in which the invention may be practiced. It is to be understood that other embodiments may be utilized, and structural changes may be made without departing from the scope of the present invention, which is defined in the appended claims. Many of the techniques and procedures described or referenced herein are well understood and commonly employed by those skilled in the art. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

All publications mentioned herein are to disclose and describe aspects, methods and/or materials in connection with the cited publications. For example, U.S. Patent Publication 20150259742, U.S. Patent App. No. 15/025,185, titled "METHOD TO ESTIMATE THE AGE OF TISSUES AND CELL TYPES BASED ON EPIGENETIC MARKERS", filed by Stefan Horvath; U.S. Patent App. No. 14/119,145, titled "METHOD TO ESTIMATE AGE OF INDIVIDUAL BASED ON EPIGENETIC MARKERS IN BIOLOGICAL SAMPLE", filed by Eric Villain et al.; and Hannum et al. "Genome-Wide Methylation Profiles Reveal Quantitative Views Of Human Aging Rates." Molecular Cell. 2013; 49(2):359-367; Matsuyama et al., "Epigenetic clock analysis of human fibroblasts in vitro: effects of hypoxia, donor age, and expression of hTERT and SV40 largeT" AGING 2019, Vol. 11, 1-11, and patent US2015/0259742.

DNA methylation refers to chemical modifications of the DNA molecule. Technological platforms such as the Illumina Infinium microarray or DNA sequencing based methods have been found to lead to highly robust and reproducible measurements of the DNA methylation levels of a person. There are more than 28 million CpG loci in the human genome. Consequently, certain loci are given unique identifiers such as those found in the Illumina CpG loci database (see, e.g. Technical Note: Epigenetics, CpG Loci Identification ILLUMINA Inc. 2010). These CG locus designation identifiers are used herein. In this context, one embodiment of the invention is a method of obtaining information useful to observe biomarkers associated with a phenotypic age of an individual by observing the methylation status of all of the 391 methylation marker specific GC loci that are identified herein.

The term "epigenetic" as used herein means relating to, being, or involving a chemical modification of the DNA molecule. Epigenetic factors include the addition or removal of a methyl group which results in changes of the DNA methylation levels.

The term "nucleic acids" as used herein may include any polymer or oligomer of pyrimidine and purine bases, preferably cytosine, thymine, and uracil, and adenine and guanine, respectively. The present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "methylation marker" as used herein refers to a CpG position that is potentially methylated. Methylation typically occurs in a CpG containing nucleic acid. The CpG containing nucleic acid may be present in, e.g., in a CpG island. a CpG doublet, a promoter, an intron, or an exon of gene. For instance, in the genetic regions provided herein the potential methylation sites encompass the promoter/enhancer regions of the indicated genes. Thus, the regions can begin upstream of a gene promoter and extend downstream into the transcribed region.

The term "gene" as used herein refers to a region of genomic DNA associated with a given gene. For example, the region can be defined by a particular gene (such as protein coding sequence exons, intervening introns and associated expression control sequences) and its flanking sequence. It is, however, recognized in the art that methylation in a particular region is generally indicative of the methylation status at proximal genomic sites. Accordingly, determining a methylation status of a gene region can comprise determining a methylation status of a methylation marker within or flanking about 10 bp to 50 bp, about 50 to 100 bp, about 100 bp to 200 bp, about 200 bp to 300 bp, about 300 to 400 bp, about 400 bp to 500 bp, about 500 bp to 600 bp, about 600 to 700 bp, about 700 bp to 800 bp, about 800 to 900 bp, 900 bp to 1kb, about 1 kb to 2 kb, about 2 kb to 5 kb, or more of a named gene, or CpG position.

The phrase "selectively measuring" as used herein refers to methods wherein only a finite number of methylation marker or genes (comprising methylation markers) are measured rather than assaying essentially all potential methylation marker (or genes) in a genome. For example, in some aspects, "selectively measuring" methylation markers or genes comprising such markers can refer to measuring more than (or no more than) 300, 200, 100, 75, 50, 25, or 10 different methylation markers or genes comprising methylation markers.

The invention described herein provides novel and powerful predictors of life expectancy, mortality, and morbidity based on DNA methylation levels. In this context, it is critical to distinguish clinical from molecular biomarkers of aging. Clinical biomarkers such as lipid levels, blood pressure, blood cell counts have a long and successful history in clinical practice. By contrast, molecular biomarkers of aging are rarely used. However, this is likely to change due to recent breakthroughs in DNA methylation based biomarkers of aging. Since their inception in 2013, DNA methylation (DNAm) based biomarkers of aging promise to greatly enhance biomedical research, clinical applications, patient care, and even medical underwriting when it comes to life insurance policies and other financial products. They will also be more useful for clinical trials and intervention assessment that target aging, since they are more proximal to the biological changes that characterize the aging process compared to upstream clinical read outs of health and disease status.

The profitability of a life insurance product directly depends on the accurate assessment of mortality risk because the costs of life insurance (to the insurance company) are directly proportional to the number of deaths in a given category. Thus, any improvement in assessing mortality risk and in improving the basic classification will directly translate into cost savings. For the reasons noted above, DNA methylation (DNAm) based biomarkers of aging are useful for predicting mortality. Consequently, they are useful the life insurance industry due to their ability to increase the accuracy of medical underwriting. DNAm measurements can provide a host of complementary information that can inform the medical underwriting process. In this context, the DNAm based biomarkers and associated method disclosed herein can be used both to estimate biological age, as well as to directly predict/prognosticate mortality.

The disclosure presented herein surrounding the prediction of mortality and morbidity show that these combinations of clinical and DNAm based biomarkers are highly robust and informative for a range of applications. DNAm age can not only be used to directly predict/prognosticate mortality but also relate to a host of age related conditions such as heart disease risk, cancer risk, dementia status, cardiovascular disease and various measures of frailty. Further embodiments and aspects of the invention are discussed below.

## ILLUSTRATIVE ASPECTS AND EMBODIMENTS OF THE INVENTION

Chronological time has been shown to elicit predictable hypo- and hyper-methylation changes at many regions across the genome [1-5], and as a result, the first generation of DNAm based biomarkers of aging were developed to predict chronological age [6-11]. The blood-based age estimator by Hannum (2013) [8] and the pan-tissue estimator by Horvath (2013) [9] produce age estimates (DNAm age) are widely used in epidemiological studies [12, 13]. After adjusting a DNAm age estimate for chronological age, one arrives at a measure of epigenetic age acceleration. Positive values of epigenetic age acceleration (indicative of faster epigenetic aging) exhibit statistically significant associations with many age-related diseases and conditions [12-25].

As indicated by its name, the pan-tissue age estimator applies to all sources of DNA (except for sperm) [9]. Despite its many successful applications, the pan-tissue DNAm age estimator, for reasons yet to be elucidated, performs sub-optimally when applied to fibroblast samples [9]. This is particularly frustrating because fibroblasts are widely used in *ex vivo* studies of various interventions. As a case in point, the Progeria Research Foundation provides fibroblast lines derived from skin biopsies from patients with Hutchinson Gilford Progeria Syndrome (HGPS) for use in research. It is therefore necessary to address this challenge and develop epigenetic biomarkers of aging that are highly accurate and equally compatible with fibroblasts and other readily accessible human cells. In spite of clear acceleration of phenotypic aging in HGPS, this is not mirrored in epigenetic age measurements by current DNA methylation-based estimators [9]. While this could be due to a genuinely interesting distinction between epigenetic and phenotypic aging, it could also be due an anomaly arising from the incompatibility between current age estimators and fibroblasts. The discernment between the two possibilities requires an age estimator that is best-suited for measuring epigenetic age of fibroblasts very accurately. Sharing this challenge and aim, is the need for an age estimator that is highly compatible with cells that are used routinely in *ex vivo* experiments. In particular, keratinocytes, fibroblasts and microvascular endothelial cells are readily isolated from skin biopsies for experimental use. The ability to accurately measure and track their epigenetic age in culture would be a boost to testing and screening compounds with anti-aging properties that can potentially work in humans. This would alleviate several high challenging features inherent in carrying out such tests in humans, such as the great length of time required to determine effect, the high susceptibility of such trails to life-style differences, the inability to control against confounders and the enormous cost that it entails. Hence, an *ex vivo* system that incorporates human cells and a highly sensitive and precise epigenetic clock compatible with these cells will undoubtedly accelerate the screening and detection of compounds that stops or slow the rate of human aging.

Here, we describe a novel powerful epigenetic age estimator (called the skin & blood clock) that outperforms existing DNAm-based biomarkers when it comes to estimating the chronological ages of human donors of fibroblasts, keratinocytes, endothelial cells, skin cells, lymphoblastoid cells, blood, and saliva samples. Embodiments of this invention include methods of observing biomarkers in human skin and/or blood cells that correlate with an age of an individual. These methods typically comprise obtaining genomic DNA from human skin and/or blood cells derived from the individual; observing the individual's genomic DNA cytosine methylation status in all of the 391 methylation markers of SEQ ID NO: 1- SEQ ID NO: 391 (typically wherein said observing comprises performing a bisulfite conversion process on the genomic DNA so that cytosine residues in the genomic DNA are transformed to uracil, while 5-methylcytosine residues in the genomic DNA are not transformed to uracil); comparing the CG locus methylation observed in the individual to the CG locus methylation observed in genomic DNA from human skin and/or blood cells derived from a group of individuals of known ages; and then correlating the CG locus methylation observed in the individual with the CG locus methylation and known ages in the group of individuals so that biomarkers in human skin and/or blood cells that correlate with an age of an individual such that biomarkers in human skin and/or blood cells that correlate with an age of an individual are observed.

As noted above, embodiments of the present invention relate to methods for estimating the biological age of an individual human tissue or cell type sample based on measuring DNA Cytosine-phosphate-Guanine (CpG) methylation markers that are attached to DNA. In a general embodiment of the invention, a method is disclosed comprising a first step of choosing a source of DNA such as specific biological cells (e.g. T cells in blood) or tissue sample (e.g. blood) or fluid (e.g. saliva). In a second step, genomic DNA is extracted from the collected source of DNA of the individual for whom a biological age estimate is desired. In a third step, the methylation levels of the methylation markers near the specific clock CpGs are measured. In an optional fourth step, a statistical prediction algorithm can be applied to the methylation levels to predict the age. One basic approach is to form a weighted average of the CpGs, which is then transformed to DNA methylation (DNAm) age using a calibration function. As used herein, "weighted average" is a linear combination calculated by giving values in a data set more influence according to some attribute of the data. It is a number in which each quantity included in the linear combination is assigned a weight (or coefficient), and these weightings determine the relative importance of each quantity in the linear combination.

DNA methylation of the methylation markers (or markers close to them) can be measured using various approaches, which range from commercial array platforms (e.g. from IlluminaTM) to sequencing approaches of individual genes. This includes standard lab techniques or array platforms. A variety of methods for detecting methylation status or patterns have been described in, for example U.S. Pat. Nos. 6,214,556, 5,786,146, 6,017,704, 6,265,171, 6,200,756, 6,251,594, 5,912,147, 6,331,393, 6,605,432, and 6,300,071 and US Patent Application Publication Nos. 20030148327, 20030148326, 20030143606, 20030082609 and 20050009059. For a review of some methylation detection methods, see, Oakeley, E. J., Pharmacology & Therapeutics 84:389-400 (1999). Available methods include, but are not limited to: reverse-phase HPLC, thin-layer chromatography, SssI methyltransferases with incorporation of labeled methyl groups, the chloracetaldehyde reaction, differentially sensitive restriction enzymes, hydrazine or permanganate treatment (m5C is cleaved by permanganate treatment but not by hydrazine treatment), sodium bisulfite, combined bisulphate-restriction analysis, and methylation sensitive single nucleotide primer extension.

The methylation levels of a subset of the DNA methylation markers disclosed herein are assayed (e.g. using an IlluminaTM DNA methylation array, or using a PCR protocol involving relevant primers). To quantify the methylation level, one can follow the standard protocol described by IlluminaTM to calculate the beta value of methylation, which equals the fraction of methylated cytosines in that location. The invention can also be applied to any other approach for quantifying DNA methylation at locations near the genes as disclosed herein. DNA methylation can be quantified using many currently available assays which include, for example:

a) Molecular break light assay for DNA adenine methyltransferase activity is an assay that is based on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.

b) Methylation-Specific Polymerase Chain Reaction (PCR) is based on a chemical reaction of sodium bisulfite with DNA that converts unmethylated cytosines of CpG dinucleotides to uracil or UpG, followed by traditional PCR. However, methylated cytosines will not be converted in this process, and thus primers are designed to overlap the CpG site of interest, which allows one to determine methylation status as methylated or unmethylated. The beta value can be calculated as the proportion of methylation.

c) Whole genome bisulfite sequencing, also known as BS-Seq, is a genome-wide analysis of DNA methylation. It is based on the sodium bisulfite conversion of genomic DNA, which is then sequencing on a Next-Generation Sequencing (NGS) platform. The sequences obtained are then re-aligned to the reference genome to determine methylation states of CpG dinucleotides based on mismatches resulting from the conversion of unmethylated cytosines into uracil.

d) The HpaII tiny fragment Enrichment by Ligation-mediated PCR (HELP) assay is based on restriction enzymes' differential ability to recognize and cleave methylated and unmethylated CpG DNA sites.

e) Methyl Sensitive Southern Blotting is similar to the HELP assay but uses Southern blotting techniques to probe gene-specific differences in methylation using restriction digests. This technique is used to evaluate local methylation near the binding site for the probe.

f) ChIP-on-chip assay is based on the ability of commercially prepared antibodies to bind to DNA methylation-associated proteins like MeCP2.

g) Restriction landmark genomic scanning is a complicated and now rarely-used assay is based upon restriction enzymes' differential recognition of methylated and unmethylated CpG sites. This assay is similar in concept to the HELP assay.

h) Methylated DNA immunoprecipitation (MeDIP) is analogous to chromatin immunoprecipitation. Immunoprecipitation is used to isolate methylated DNA fragments for input into DNA detection methods such as DNA microarrays (MeDIP-chip) or DNA sequencing (MeDIP-seq).

i) Pyrosequencing of bisulfite treated DNA is a sequencing of an amplicon made by a normal forward primer but a biotinylated reverse primer to PCR the gene of choice. The Pyrosequencer then analyses the sample by denaturing the DNA and adding one nucleotide at a time to the mix according to a sequence given by the user. If there is a mismatch, it is recorded and the percentage of DNA for which the mismatch is present is noted. This gives the user a percentage methylation per CpG island.

In certain embodiments of the invention, the genomic DNA is hybridized to a complimentary sequence (e.g. a synthetic polynucleotide sequence) that is coupled to a matrix (e.g. one disposed within a microarray). Optionally, the genomic DNA is transformed from its natural state via amplification by a polymerase chain reaction process. For example, prior to or concurrent with hybridization to an array, the sample may be amplified by a variety of mechanisms, some of which may employ PCR. See, for example, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159, 4,965,188, and 5,333,675. The sample may be amplified on the array. See, for example, U.S. Pat. No. 6,300,070, which is incorporated herein by reference.

In addition to using art accepted modeling techniques (e.g. regression analyses), embodiments of the invention can include a variety of art accepted technical processes. For example, in certain embodiments of the invention, a bisulfite conversion process is performed so that cytosine residues in the genomic DNA are transformed to uracil, while 5-methylcytosine residues in the genomic DNA are not transformed to uracil. Kits for DNA bisulfite modification are commercially available from, for example, MethylEasyTM (Human Genetic SignaturesTM) and CpGenomeTM Modification Kit (ChemiconTM). See also, WO04096825A1, which describes bisulfite modification methods and Olek et al. Nuc. Acids Res. 24:5064-6 (1994), which discloses methods of performing bisulfite treatment and subsequent amplification. Bisulfite treatment allows the methylation status of cytosines to be detected by a variety of methods. For example, any method that may be used to detect a SNP may be used, for examples, see Syvanen, Nature Rev. Gen. 2:930-942 (2001). Methods such as single base extension (SBE) may be used or hybridization of sequence specific probes similar to allele specific hybridization methods. In another aspect the Molecular Inversion Probe (MIP) assay may be used.

EXAMPLES

### EXAMPLE 1: EPIGENETIC CLOCK FOR SKIN AND BLOOD CELLS APPLIED TO HUTCHINSON GILFORD PROGERIA AND EX VIVO STUDIES

**DNA methylation data sets**

We analyzed both novel and existing DNA methylation data sets that were generated on the Illumina Infinium platform (Table 1). DNA was extracted from human fibroblasts, keratinocytes, buccal cells, endothelial cells, blood, and saliva. We analyzed data from two Illumina platforms (Infinium 450K and the EPIC array, also known as the 850K array) to ensure that the resulting estimator would apply to the latest Illumina platform (the EPIC array).

**The DNAm age estimator for skin and blood**

To ensure an unbiased validation of the test data, we used only the training data to define the DNAm age estimator. As detailed in Methods, a transformed version of chronological age was regressed on methylation states of CpGs using a penalized regression model (elastic net). The elastic net regression model automatically selected 391 CpGs (Table 5). We refer to the 391 CpGs as (epigenetic) clock CpGs since their weighted average (formed by the regression coefficients) amounts to a highly accurate epigenetic aging clock.

In the following, we will demonstrate that the resulting age estimator (referred to as skin & blood clock) performs remarkably well across a wide spectrum of cells that are widely used in *ex vivo* studies. The new skin & blood clock even outperforms the pan-tissue clock (Horvath 2013) in all metrics of accuracy (age correlation, median error) in fibroblasts, microvascular endothelial cells, buccal epithelial cells, keratinocytes, and dermis/epidermis samples (Figure 1 and Figure 7). As indicated by its name, the new skin & blood clock is also a highly accurate age estimator of blood methylation data, where it provides more accurate age estimates than the widely used estimators by Horvath (2013) and Hannum (2013)[8] (Figure 2A,D,G and Figure 8). Further, it outperforms the Horvath and Hannum DNAm age estimators when applied to lymphoblastoid cell lines (Figure 2B,E,H), i.e. B cells that have been immortalized using EBV transformation. Interestingly, the DNAm age of blood is highly correlated with the DNAm age estimate of the lymphoblastoid cell line collected from the same donor at the same time (r=0.83, Figure 2C). The skin & blood clock accurately estimates age in two different saliva DNA methylation data sets (age correlations r=0.9 and r=0.95) and outperforms the pan-tissue DNAm age estimator in these data (Figure 9). The skin & blood clock also applies to cord blood samples as can be seen from the fact that it accurately estimates gestational age in three different data (with correlations ranging from r=0.15 to r=0.66, Figure 10).

Similar to what has been observed with previous age estimators, epigenetic age acceleration in blood (according to the skin & blood clock) is highly predictive of time to all-cause mortality (p=9.6E-7) according to a univariate Cox regression model fixed effects meta-analysis across multiple epidemiological cohort studies (Figure 11).

Epigenetic age acceleration measured by the skin & blood clock is only weakly correlated with, or affected by blood cell type counts, as is evident from the analyses of postmenopausal women from the Women's Health Initiative (Figure 12). The strongest correlations are observed with exhausted CD8+ T cells (r=0.22), naive CD8+ T cells (r=-0.21), and naive CD4+T cells (r=-0.19, Figure 12B-D). These correlations suggest that individuals with positive epigenetic age accelerations exhibit an adaptive immune system that is older than expected.

**Epigenetic age of fibroblasts from Hutchinson Gilford Progeria fibroblasts**

Segmental progeroid syndromes such as Down syndrome and Werner syndrome have been found to exhibit epigenetic age acceleration according to the pan-tissue clock [16, 25]. A severe developmental disorder (known as syndrome X) whose patients exhibit dramatically delayed development (seemingly eternal toddler-like state) was not associated with epigenetic age acceleration in blood tissue [26].

Cases of the Hutchinson Gilford Progeria (HGP) and the Atypical Werner Syndrome (AWS) can be caused by different progeroid mutations of the LMNA gene (Figure 3). It is not yet known whether HGPS patients, who generally appear normal at birth but exhibit a "failure-to-thrive" syndrome, exhibit positive or negative epigenetic age acceleration. HGPS is associated with many clinical manifestations of accelerated aging including loss of subcutaneous fat, joint contractures, and a striking aged appearance during the first to third years of life [27]. Virtually all HGPS patients die of myocardial infarction at a median age of 14.6 year [28]. Classical HGPS is caused by a recurrent heterozygous pathogenic variant, c.1824C>T in exon 11 of the *LMNA* gene, which activates a cryptic splice site and causes a 50-amino acid in-frame deletion (Δ50) [29]. The resulting abnormal protein, termed progerin, lacks the proteolytic site for an essential but transient post-translational modification by the ZMPSTE24 metalloprotease. This causes retention of the C-terminal farnesylated moiety, resulting in aberrant nuclear structure and function [29]. Non-classical HGPS mutations at the exon 11 and intron 11 boundary, including c.1968+1G>A [30] and c.1968+2T>C [31], can also activate the cryptic splice site, leading to the accumulation of progerin and an infantile-onset HGPS phenotype. Biallelic ZMPSTE24 mutations also cause accumulations of famesylated lamin A and various degree of progeroid phenotypes, depending on the residual enzymatic activity of ZMPSTE24 [32, 33]. In rare instances, a homozygous amino acid substitution of lamin A can present with a phenotype similar to HGPS or mandibuloacral dysplasia, as described in cases with [p.Met540Thr; p.Met540Thr] [34] and [p.Thr528Met; p.Met540Thr] [35].

A small subset of cases of Atypical Werner syndrome (AWS) (those with some features of Werner syndrome, without mutations in *WRN* or altered expressions of the WRN protein) may be caused by accumulations of low levels of progerin [36, 37]. Pathogenic LMNA variants found AWS include c.1968G>A and c.1968+5G>A [36]. While there is a general genotype-phenotype correlation between the amount of progerin and the severity of the disease, the amounts and structures of progerin can vary among those who carry the same *LMNA* splice mutation, and the severity of the disease can vary among patients within the same family [36, 37].

The original pan-tissue DNAm age estimator does not find positive age acceleration in HGPS individuals (Table 4). By contrast, the application of the novel skin & blood clock showed that while DNAm age is highly correlated with chronological age in fibroblasts, those from HGP cases exhibited accelerated epigenetic aging (Figure 4). The epigenetic age acceleration effects become particularly pronounced after adjusting for differences in cell population doubling levels and when restricting the analysis to children who are less than 10 years old (p=0.00021, Table 3). There is a non-significant trend of increased methylation age in Atypical Werner Syndrome cases with low levels of progerin. The median age of death of classical HGPS is ~14.6 years [38], while that of AWS patients with low levels of progerin range from 37 to 60s [36]. Since classical HGPS leads only to a nominally significant epigenetic age acceleration effect, it is perhaps not surprising that Atypical Werner Syndrome (which presents with a lower progerin concentration, Figure 3) is not associated with greater magnitude of epigenetic age acceleration.

Although non-classical HGPS are often presented at later ages, they can nevertheless be diagnosed at ages that are slightly younger than patients with classical HGPS [27]. It should indeed be noted that the cases examined in this study (see Methods for mutation details). have exceptionally early manifestations - as early as birth or fewer than 5 months of age. Interestingly, their DNA methylation age acceleration is comparable and consistent with that of classical HGPS, which as mentioned is an early onset progeria condition (Figure 4D).

Detailed results for the lines of skin fibroblasts provided by the Progeria Research Foundation are presented in Table 2. The skin & blood clock provides marginally significant evidence (p=0.062) that fibroblast samples from boys with classical HGP are epigenetically older than those from girls with classical HGP but no sex effect can be observed after pooling classical and non-classical HGP samples (Figure 13).

It is to be further noted that the small epigenetic age acceleration of HGPS fibroblasts revealed by the skin & blood clock, escapes detection when measurements were carried out with the pan-tissue clock; indeed the opposite appears to be the case (Figure 14C). Evidently, manifestation of such changes in fibroblasts is dependent upon the choice of the age estimator that is used.

**_Ex vivo_ studies of anti-aging interventions**

While it may appear obvious that the skin & blood clock is superior in terms of compatibility with fibroblasts, it is necessary to verify and validate this deduction by applying this clock to non-progeria fibroblasts and other cell types. To this end, fibroblasts derived from non-progeria neonatal foreskins are ideal as they pose minimal to no confounding factors that could alter their age. While the skin & blood clock correctly estimated the neonatal fibroblast cells to be of ages close to zero years, the pan-tissue age estimator leads to age estimates larger than 10 years (Figure 5AB). Analyses of other skin cell types namely, keratinocytes and microvascular endothelial cells derived from neonatal foreskins also revealed greater accuracy of the skin & blood clock. This conclusion continues to hold true even when the analyses were extended to isogenic skin cells derived from adult tissues (Figure 7).

Having established the robustness of the skin & blood clock in measuring age of cells isolated from human tissues, we proceeded to test the applicability of the clock on human cells cultured *ex vivo*. As observed previously using the pan-tissue age estimator, the skin & blood clock revealed that human fibroblasts cultured *ex vivo* undergo epigenetic aging. However, unlike the former, the DNAm ages of the fibroblasts estimated by the new clock are consistent with those of the donors from whom the cells were obtained (Figure 5A). Furthermore, proliferation of human fibroblasts in culture, measured as population doubling, was observed to correlate with continual increase in DNAm age until cellular senescence. Importantly, hTERT-immortalized fibroblasts also exhibited similar progression of aging which continued unabated, indicating that hTERT immortalization does not halt epigenetic aging. These features which are also shared by human coronary artery endothelial cells, are revealed by the skin & blood clock, but no so by the pan-tissue age estimator (Figure 5).

By its ability to quantitatively track aging of human cells *ex vivo,* the skin & blood clock lends itself to be used in the development of an *ex vivo* human cell aging assay that can be used for testing and screening compounds with anti-aging or pro-aging effects. For example, we find suggestive evidence that rapamycin slows epigenetic aging in dividing keratinocytes whereas Y-2763 appears to increase epigenetic aging in neonatal keratinocytes (Figure 6A). Similarly, we find suggestive evidence that estrogen is associated with slower epigenetic aging in fibroblasts (Figure 6B). While these results are being validated with additional studies and will be reported separately with much greater details, they demonstrate the proof-of-concept that the resolution of the new skin & blood clock is sufficiently high and robust to allow the establishment of an assay that can detect, within a short time, compounds that affect human aging.

**Effect of lifestyle and demographic variables on blood aging**

To characterize further the nature of the skin & blood clock, we applied it to DNA methylation data from various human cohorts.

Similar to the previous epigenetic aging clock analyses of blood [22], the new skin & blood clock reveals that slow epigenetic aging in blood is associated with higher education, physical exercise, fish consumption, high carotenoid levels, beta carotene levels, and, to a lesser extent, with alcohol consumption (Table 5). Conversely, faster epigenetic aging in blood is associated C-reactive protein levels, body mass index, triglyceride, and insulin levels (Table 5). Collectively these characteristics demonstrate that although the new clock is highly and uniquely accurate for cells such as fibroblasts, it has not acquired this at the cost of losing any of the features shared amongst existing age estimators. This clock represents genuine added value in terms of epigenetic age estimation.

**DISCUSSION**

We present a new DNA methylation based biomarker (based on at least 10, 50, 100, 200, 300 or 391 CpGs disclosed herein) that accurately measures the age of human fibroblasts, keratinocytes, buccal cells, endothelial cells, skin and blood samples. The need for this became apparent when it was observed that the existing DNA methylation-based age estimators that are highly accurate in measuring ages of blood and many cell types of the body, perform poorly when applied to human fibroblasts and other skin cells. The implications of this anomaly extend beyond theoretical curiosity as it impacts on the reliability of conclusions drawn from epigenetic age analyses of skin cells. As a case in point, the apparent lack of epigenetic age acceleration of HGPS fibroblasts indicated by measurements using the pan-tissue age estimator was in doubt.

Skin cells are among the most common cell types employed in laboratories. This is owed largely to the ease by which cells such as keratinocytes, fibroblasts, microvascular endothelial cells can be isolated from skin, allowing cells from many donors to be easily acquired and used; a characteristic that is not afforded by internal organs. The ability to use these cells to investigate epigenetic age *ex vivo* is paramount if we are to identify constituents of the epigenetic clock and elucidate how they function together to drive the ticking of the clock.

The skin & blood clock that we derived is well-suited to meet all these needs. By applying it to fibroblasts from HGPS cases, we a significant epigenetic age acceleration effect after adjusting for fibroblast population doubling levels. For reason yet to be determined, the pan-tissue DNA methylation age estimator failed to detect this subtle increase in epigenetic age acceleration. It could be simply due to lower sensitivity or to a qualitative difference between the CpGs that constitute the different DNAm age estimators. In considering the modest increase in age acceleration of HGPS cells, it is worth noting that changes in the methylation state of clock CpGs in the early years of life already occur at a frenetic rate, which is approximately twenty-four times greater than that which takes place after the age of twenty (Horvath 2013). Hence, it is difficult to envisage and expect that the rate of epigenetic aging in HGPS cells from young donors could be very much greater in magnitude. This hypothesis can in theory be tested by measuring the epigenetic age of HGPS cells from patients older that twenty years of age, when the basal rate of normal epigenetic aging is significantly reduced, allowing for any age acceleration to become even more apparent. It is however difficult to achieve this as the median age of death of HGPS patients is approximately 14 years old. The ability of the skin & blood clock to nevertheless detect the modest increase in age acceleration in young HGPS patient fibroblasts attests to its sensitivity.

In addition to resolving the conundrum of HGPS described above, the skin & blood clock outperforms widely used existing biomarkers when it comes to accurately measuring the age of an individual based on DNA extracted from skin, dermis, epidermis, blood, saliva, buccal swabs, and endothelial cells. Thus, the biomarker can also be used for forensic and biomedical applications involving human specimens. The biomarker applies to the entire age span - from newborns (e.g. cord blood samples) to centenarians.

Furthermore, the skin & blood clock confirms the effect of lifestyle and demographic variables on epigenetic aging. Essentially it highlights a very strong trend of accelerated epigenetic aging with sub-clinical indicators of poor health. Conversely, reduced aging rate is correlated with known health-improving features such as physical exercise, fish consumption, high carotenoid levels etc. (Table 5). As with the other age predictors, the skin & blood clock is also able to predict time to death. Collectively, these features show that while the skin & blood clock is clearly superior in its performance on skin cells, it crucially retained all the other features that are common to other existing age estimators.

The performance of the skin & blood clock is equally impressive when applied to ex vivo cell culture system. Studies with fibroblasts and endothelial cells revealed that cell proliferation (as measured by population doublings) is significantly associated with increased DNAm age even in hTERT immortalized cells which is consistent with other studies [39, 40].

We have coupled the skin & blood clock with human primary cell cultures to generate an *ex vivo* human cell aging assay that is highly sensitive. This assay is able to detect epigenetic aging of a few years, in a few months. The benefits of this assay are self-evident. The two most obvious are its potential use to test and screen for potential pharmaceuticals that can alter the rate of epigenetic aging, and its use to test and detect potential age-inducing hazards in the arena of health protection.

Many of our key results are critically dependent upon the choice of a DNAm age estimator, i.e., they could only be observed with the new skin & blood clock assay. For example, the original pan-tissue clock could not detect an age acceleration effect due to HGPS nor could they reveal an anti-aging effect of rapamycin. Looking ahead, there are likely to be valuable applications of this more robust epigenetic clock for the evaluation of clinical trials of pharmaceutical interventions in segmental progeroid syndromes. For example, the most recent clinical trial of a farnesyltransferase inhibitor, lonafarnib, for the treatment of HGPS was able to significantly lower mortality rates (6.3% death in the treated group vs 27% death in the matched untreated group after 2.2 years of follow-up) [28]. We are likely to see additional such clinical trials. For example, *in vitro* studies of the effects of rapamycin or another mTOR inhibitor, metformin, showed a reduction of progerin accumulation accompanied by the amelioration of cellular HGPS phenotypes [41, 42]. Reactivation of the antioxidant NRF2 was also shown to alleviate cellular defects of HGPS in an animal model [43]. It would be interesting to examine whether these drugs affect DNA methylation patterns in fibroblasts or other cell types.

Due to its superior accuracy, we expect that this novel set of epigenetic biomarkers will be useful for both *ex vivo* studies involving cultures of various somatic cell types, including fibroblasts, keratinocytes, and endothelial cells, as well as *in vivo* studies utilizing samples of peripheral blood and biopsies of skin.

## METHODS

### Definition of DNAm age using a penalized regression model

Using the training data sets, SH used a penalized regression model (implemented in the R package glmnet [44]) to regress a calibrated version of chronological age on the CpG probes that a) were present both on the Illumina 450K and EPIC platforms. The alpha parameter of glmnet was chosen as 0.5 (elastic net regression) and the lambda value was chosen using cross-validation on the training data. DNAm age was defined as predicted age.

### Processing of DNA methylation data sets

The raw DNA methylation data were normalized using the noob normalization method when raw "idat" files were available [45].

### Fibroblasts from the Progeria Research Foundation

Fibroblast cell lines were from cases with classic mutations, non-classical mutations and parental controls as detailed in Table 2. The following citations provide additional details on individual cases: *LMNA* c.1968+1G>A heterozygote (Moulson et al., 2007)[30], *LMNA* c.1968+2T>C heterozygote (Bar et al., 2017)[31], *LMNA* p.Met540Thr homozygotes (Bai et al., 2014)[34] and compound heterozygotes of ZMPSTE24 p.Pro248Leu and p.Trp450* (Ahmad et al., 2010)[32]. As detailed in Table 2, we generated DNA methylation data from the following cell lines that are described on the PRF webpage: PSADFN086, PSADFN257, PSADFN317, PSADFN318, PSADFN392, HGADFN003, HGADFN169, HGADFN143, HGADFN167, HGADFN271, HGADFN164, HGADFN178, HGADFN122, HGADFN127, HGADFN155, HGADFN188, HGADFN367, HGFDFN369, PRF319P8, PSFDFN319, PSFDFN327, PSFDFN394, PSFDFN319, HGMDFN090, HGMDFN368, PSMDFN320, HGMDFN368, PSMDFN320, PSMDFN326, PSMDFN346, PSMDFN393, HGFDFNDNA168.

**Control samples**

To avoid batch effect in the DNA methylation data, we generated control fibroblast samples for concurrent assays with fibroblasts from patients with HGPS. The control fibroblasts have been described in [46]. Cell fibroblast cell lines ranging in age from three days to 96 years were obtained from the NIA Aging Cell Repository at the Coriell Institute for Medical Research. The Coriell ID designations were:, RRID#: AG08498, RRID:CVCL_1Y51, AG07095, RRID:CVCL_0N66, AG11732, RRID:CVCL_2E35, AG04060, RRID:CVCL_2A45, AG04148, RRID:CVCL_2A55, AG04349, RRID:CVCL_2A62, AG04379, RRID:CVCL_2A72, AG04056, RRID:CVCL_2A43, AG04356, RRID:CVCL_2A69, AG04057, RRID:CVCL_2A44, AG04055, RRID:CVCL_2A42, AG13349, RRID:CYCL_2G05, AG13129, RRID:CVCL_2F55, AG12788, RRID:CVCL_L632, AG07725, RRID:CVCL_2C46, AG04064, RRID:CVCL_L624, AG04059, RRID:CVCL_L623, AG09602, RRID:CVCL_L607, AG16409, RRID:CVCL_V978, AG06234, RRID:CVCL_2B66, AG04062, RRID:CVCL_2A47, AG08433, RRID:CVCL_L625, AG16409, RRID:CVCL_V978, GM00302, RRID:CVCL_7277, AG01518, RRID:CVCL_F696, AG06234, RRID:CVCL_2B66.
Mycoplasma contamination is routinely ruled out for all cell cultures using LINE and PCR-based techniques. None of the cell lines we have used are among those listed the International Cell Line Authentication Committee (ICLAC) as commonly misidentified cell lines. Fibroblast cell lines were cultured and expanded in DMEM media (high glucose, Invitrogen) supplemented with 10% or 15% fetal bovine serum (Gibco), sodium pyruvate, non-essential amino acids, GlutaMAX (Invitrogen), Pen/Strep solution, and Beta-mercaptoethanol. Fibroblast cell lines were expanded to a population doubling level (PDL) of ~19-21. The formula used to calculate PDL was PDL = 3.32*log (cells harvested/cells seeded) + previous PDL. Cell aliquots of early passages of all cell lines were kept frozen at -150°C in the above culture medium with additional 40% FBS and 10% DMSO.

**Blood methylation data from different cohorts**

Blood methylation data and cohorts have been described in [21, 47]. A number of validation studies were used to test associations between DNAm Clock Age and various aging-related traits.

**Estimation of blood cell counts based on DNAm levels**

We estimate blood cell counts using two different software tools. First, Houseman's estimation method [48] was used to estimate the proportions of CD8+ T cells, CD4+ T, natural killer, B cells, and granulocytes (mainly neutrophils). Second, the Horvath blood cell estimation method, implemented in the advanced analysis option of the epigenetic clock software [9, 17], was used to estimate the percentage of exhausted CD8+ T cells (defined as CD28-CD45RA-), the number (count) of naïve CD8+ T cells (defined as CD45RA+CCR7+) and plasmablasts. We and others have shown that the estimated blood cell counts have moderately high correlations with corresponding flow cytometric measures [48, 49].

TABLES 1-3

| No. | Data Source | Use | n | Source | Median Age (Range) |
|-----|-------------|-----|---|--------|---------------------|
| 1 | existing, Portales-Casamar 2016, GSE80261 | Train | 216 | Buccal | 11 (5,18) |
| 2 | existing, Berko 2014, GSE50759 | Train | 96 | Buccal | 7 (1,28) |
| 3 | novel, blood methylation | Train | 278 | whole blood | 69 (2,92) |

| 4 | existing, Yang 2017, GSE104471 | Train | 72 | Epithelium | 30 (24,74) |
|---|---|---|---|---|---|
| 5 | existing, Ivanov 2016, GSE77136 | Train | 21 | Fibroblast | 33 (0.1,85) |
| 6 | existing, Wagner 2014, GSE52026 | Train | 10 | Fibroblast | 37 (23,63) |
| 7 | novel fibroblasts | Train | 48 | Fibroblast | 50 (0.42,94) |
| 8 | novel, Cell Applications | Train | 11 | Fibroblast | 56 (7,94) |
| 9 | existing, Borman 2016, SkinE-MTAB-4385 | Train | 108 | Skin | 49.25 (18,78) |
| 10 | existing, cord blood, GSE79056 | Train | 36 | cord blood | 0 (-0.28,0.04) |
| 11 | existing, Jessen 2016, GSE94876 | Test | 120 | Buccal | 46 (35,60) |
| 12 | Lussier 2018, GSE109042 | Test | 53 | Buccal | 10 (3.5,18) |
| 13 | existing, Vandiver 2015, GSE51954 | Test | 78 | Dermis+Epidermis | 65 (20,92) |
| 14 | novel, Kenneth Raj | Test | 23 | Endothelial | 19 (19,19) |
| 15 | novel, Kenneth Raj | Test | 44 | Endothelial | 19 (17,26) |
| 16 | novel, Kenneth Raj | Test | 48 | Fibroblast | 0 (0,0) |
| 17 | novel, Kenneth Raj | Test | 48 | Fibroblast | 0 (0,0) |
| 18 | novel, Progeria Research Foundation+Commercial | Test | 88 | Fibroblast | 8 (0,77) |

| | | | | | |
|---|---|---|---|---|---|
| | vendors | | | | |
| 19 | novel, Junko Oshima | Test | 11 | Fibroblast | 36 (0,62) |
| 20 | novel, Kenneth Raj | Test | 43 | Keratinocyte | 0 (0,0) |
| 21 | novel, Blood methylation Inf 450 | Test | 100 | Whole Blood | 53 (19,82) |
| 22 | novel, Lymphoblastoid cell | Test | 100 | Lymphoblast | 53 (19,82) |
| 23 | novel, Saliva methylation | Test | 120 | Saliva | 44 (18, 81) |
| 24 | existing, Horvath 2015, GSE111223 | Test | 229 | Saliva | 68 (36,88) |
| 25 | existing, cord blood, GSE62924 | Test | 38 | cord blood | 0 (-.10,0.04) |
| 26 | existing, cord blood, GSE80283 | Test | 183 | cord blood | -0.22(-0.3,-0.1) |

**Table 1. DNA methylation data.** The rows correspond to Illumina DNA methylation data sets. The table reports the data set number, relevant citation (first author and publication year), public availability (for example, Gene Expression Omnibus identifier), sample size (n), source of the DNA (for example, tissue), median age, age range (minimum and maximum age),. The column 'Use' reports whether the data set was used as a training set or test set.

| Cell-line ID | Progeria | Mutation | Sex | Age | DNAmAge SkinClock | AgeAcc elSkinC |
|---|---|---|---|---|---|---|
| | | | | | | |

| | | | | | | lock |
|---|---|---|---|---|---|---|
| PSADFN086 | NonClassic | LM Exon 11 c.1968+1G>A | m | 0.58 | 0.39 | -3.49 |
| PSADFN257 | NonClassic | LM Exon 10 homozygous c.1619 T>C (p.Met540Thr) | m | 1.83 | 4.44 | -0.51 |
| PSADFN257.replicate | NonClassic | LM Exon 10 homozygous c.1619 T>C (p.Met540Thr) | m | 1.8 | 4.84 | -0.08 |
| PSADFN317 | NonClassic | ZMPste24 Exon 6 heterozygous c.743C>T(p.Pro248Leu)and Exon 10 heterozygous c.1349G>A (p.Trp450Stop) | m | 3.8 | 8.86 | 2.23 |
| PSADFN318 | NonClassic | ZMPste24 Exon 6 heterozygous c.743 C>T(p.Pro248Leu)and Exon 10 heterozygous c.1349G>A (p.Trp450Stop) | m | 0.4 | 7.48 | 3.75 |
| PSADFN392 | NonClassic | LM Exon 11 c.1968+2T>C | m | 7.3 | 21.61 | 11.99 |
| HGADFN003 | Classic | LM Exon 11 heterozygous c.1824C>T | m | 2 | 3.39 | -1.70 |
| HGADFN169 | Classic | LM Exon 11 heterozygous c.1824C>T | m | 8.5 | 23.73 | 13.08 |
| HGADFN143 | Classic | LM Exon 11 heterozygous c.1824C>T | m | 8.8 | 15.61 | 4.71 |
| HGADFN167 | Classic | LM Exon 11 heterozygous c.1824C>T | m | 8.4 | 17.88 | 7.32 |
| HGADFN271 | Classic | LM Exon 11 heterozygous | m | 1.3 | 10.73 | 6.24 |

| | | c.1824C>T | | | | |
|---|---|---|---|---|---|---|
| HGADFN164 | Classic | LM Exon 11 heterozygous c.1824C>T | f | 4.66 | 10.64 | 3.28 |
| HGADFN178 | Classic | LM Exon 11 heterozygous c.1824C>T | f | 6.92 | 4.36 | -4.93 |
| HGADFN122 | Classic | LM Exon 11 heterozygous c.1824C>T | f | 5 | 6.96 | -0.70 |
| HGADFN127 | Classic | LM Exon 11 heterozygous c.1824C>T | f | 3.8 | 2.10 | -4.53 |
| HGADFN155 | Classic | LM Exon 11 heterozygous c.1824C>T | f | 1.1 | 0.59 | -3.73 |
| HGADFN188 | Classic | LM Exon 11 heterozygous c.1824C>T | f | 2.3 | 1.23 | -4.11 |
| HGADFN367 | Classic | LM Exon 11 heterozygous c.1824C>T | f | 3 | 17.10 | 11.16 |

**Table 2. Epigenetic clock results for fibroblast samples from the progeria research foundation**. The columns report the cell line identifier, the disease status, mutational analysis, sex, age, DNAm age estimate (based on the skin & blood clock), and the measure of age acceleration (defined as residual from a regression line). Classic HGP cases exhibit the following mutation: LMNA Exon 11, heterozygous c.1824C>T (p.Gly608Gly). By contrast, non-classic HGP cases exhibit mutations elsewhere in the LMNA gene.

| Outcome: DNAmAge (SkinClock) | | | | | | |
|---|---|---|---|---|---|---|
| | Data: All, n=88 | | | Data: Age<10, n=44 | | |
| Covariate | Coef | St. Error | P-value | Estimate | SE | P-value |

| Intercept | -3.55 | 2.99 | 2.39E-1 | 7.34 | 2.97 | 1.84E-2 |
|---|---|---|---|---|---|---|
| Age | 1.64 | 1.29E-1 | 3.44E-20 | -5.90E-1 | 8.33E-1 | 4.84E-1 |
| Age^2 | -1.07E-2 | 2.08E-3 | 2.14E-6 | 2.40E-1 | 9.58E-2 | 1.70E-2 |
| Fibroblast Population Doubl. Level | 4.46E-1 | 1.65E-1 | 8.52E-3 | -1.20E-1 | 1.32E-1 | 3.71E-1 |
| HGP.Disease | 4.81 | 2.27 | 3.76E-2 | 5.18 | 1.25 | 2.12E-4 |

**Table 3. Multivariate regression model analysis of HGP based on the novel skin & blood clock.** DNAm age is regressed on chronological age, the square of age, the population doubling level of the fibroblast cell culture, and HGP disease status. The table reports estimates of the regression coefficients and corresponding standard errors, Wald test P-values. The last row reports the age acceleration associated with HGP disease status. The left panel and right panel report the results for all n=88 fibroblast samples and for n=44 samples from children (younger than 10 years old), respectively.

**Statistical Methods**

As for the multi-tissue DNAm age estimator (Horvath 2013) [9], the dependent variable, chronological age, was transformed before carrying out an elastic net regression analysis. Toward this end, the following function F for transforming age was used:

- 

$$F(age)=log(age+1)-log(adult.age+1) \text{ if } age<=adult.age.$$

- 

$$F(age)=(age-adult.age)/(adult.age+1) \text{ if } age>adult.age.$$

The parameter "adult.age" was set to 20. Note that F satisfies the following desirable properties: it
- i) is a continuous, monotonically increasing function (which can be inverted),
- ii) has a logarithmic dependence on age until adulthood (here set at 20 years),
- iii) has a linear dependence on age after adulthood (here set to 20),
- iv) is defined for negative ages (i.e. prenatal samples) by adding 1 (year) to age in the logarithm,
- v) it has a continuous first derivative (slope function). In particular the slope at age=adult.age is given by 1/(adult.age+1).

An elastic net regression model (implemented in the glmnet R function) was used to regress a transformed version of age on the beta values in the training data. The glmnet function requires the user to specify two parameters (alpha and beta). Since I used an elastic net predictor, alpha was set to 0.5. But the lambda value of was chosen by applying a 10 fold cross validation to the training data (via the R function cv.glmnet). The elastic net regression results in a linear regression model whose coefficients $b_0$, $b_1$, . . . , $b_{391}$ relate to transformed age as follows

$$F(\text{chronological age})=b_0+b_1 CpG_1+ \ldots +b_{391}CpG_{391}+\text{error}$$

Based, on the coefficient values from the regression model, DNAmAge is estimated as follows

$$DNAmAge = inverse.F(b_0 + b_1 CpG_1 + \ldots + b_{391} CpG_{391})$$

where inverse.F(.) denotes the mathematical inverse of the function F(.) and is specified as follows.

- 

$$anti.F(x) = (1 + adult.age) * exp(x) - 1 \text{ if } x < 0$$

- 

$$anti.F(x) = (1 + adult.age) * x + adult.age \text{ if } x >= 0$$

- and the parameter adult.age was chosen to be 20.

Thus, the regression model can be used to predict to transformed age value by simply plugging the beta values of the selected CpGs into the formula.

| Outcome: Pan-tissue DNAmAge (Horvath 2013) | | | | | | |
|---|---|---|---|---|---|---|
| | Data: All, n=88 | | | Data: Age<10, n=44 | | |
| | Coef | SE | P-value | Coef | SE | P-value |
| Age | 1.10 | 1.57E-1 | 8.85E-10 | 1.82 | 2.02 | 0.37 |
| Age^2 | -9.98E-3 | 2.54E-3 | 1.86E-4 | 5.37E-2 | 2.32E-1 | 0.82 |
| PopulationDoublingLevel | -4.75E-1 | 2.01E-1 | 2.07E-2 | -4.25E-1 | 3.19E-1 | 0.192 |
| HGP.Disease | -2.83 | 2.77 | 3.10E-1 | -2.88 | 3.02 | 0.35 |

**Table 4. Multivariate linear regression analysis of HPG using the pan-tissue DNAm age estimator (Horvath 2013).** DNAm age is regressed on chronological age, population doubling levels, and HGP disease status. The table reports estimates of the regression coefficients and corresponding standard errors, Wald test P-values. The last row reports the age acceleration associated with HGP disease status. The left panel and right panel report the results for all n=88 fibroblast samples and for n=44 samples from children (younger than 10 years old), respectively.

| | | median | AASkin | | | IEAA | | EEAA | | |
|---|---|---|---|---|---|---|---|---|---|---|
| | | | bicor | p | n | bicor | p | bicor | p | n |
| Diet | log10(Total energy) | 3.18 | -0.04 | 0.09 | 2100 | 0.00 | 0.96 | -0.02 | 0.19 | 3687 |
| | Carbohydrate | 48.65 | 0.00 | 0.88 | 2100 | 0.02 | 0.29 | 0.00 | 0.96 | 3687 |
| | Protein | 16.36 | -0.03 | 0.14 | 2100 | -0.02 | 0.15 | -0.03 | 0.10 | 3687 |
| | Fat | 34.95 | 0.03 | 0.22 | 2100 | 0.00 | 0.97 | 0.02 | 0.15 | 3687 |
| | log10(1+Red meat) | 0.21 | 0.01 | 0.76 | 2100 | 0.03 | 0.10 | 0.02 | 0.28 | 3687 |
| | log10(1+Poultry) | 0.05 | -0.04 | 0.08 | 2100 | -0.05 | 5E-3 | -0.03 | 0.06 | 3687 |
| | log10(1+Fish) | 0.03 | -0.06 | 5E-3 | 2100 | -0.02 | 0.29 | -0.07 | 2E-5 | 3687 |
| | log10(1+Dairy) | 0.36 | -0.05 | 0.02 | 2100 | 0.00 | 0.99 | -0.02 | 0.29 | 3687 |
| | log10(1+Whole grains) | 0.30 | -0.01 | 0.56 | 2100 | 0.00 | 0.85 | -0.02 | 0.19 | 3687 |
| | log10(1+Nuts) | 0.01 | 0.02 | 0.41 | 2100 | 0.01 | 0.59 | -0.01 | 0.38 | 3687 |
| | log10(Fruits) | 0.16 | -0.04 | 0.08 | 2100 | 0.00 | 0.81 | -0.03 | 0.04 | 3687 |
| | log10(Vegetables) | 0.21 | -0.04 | 0.07 | 2100 | 0.00 | 0.98 | -0.04 | 0.01 | 3687 |
| Dietary biomarkers | Retinol | 0.58 | -0.10 | 0.14 | 224 | 0.02 | 0.46 | -0.01 | 0.69 | 2268 |
| | Mean carotenoids | 0.05 | -0.10 | 0.14 | 224 | -0.06 | 4E-3 | -0.13 | 2E-9 | 2267 |
| | Lycopene | 0.36 | -0.08 | 0.24 | 224 | -0.02 | 0.44 | -0.03 | 0.17 | 2268 |
| | log10(alpha-Carotene) | -1.26 | -0.07 | 0.30 | 224 | -0.04 | 0.04 | -0.11 | 9E-8 | 2268 |
| | log10(beta-Carotene) | -0.64 | -0.15 | 0.03 | 224 | -0.06 | 0.01 | -0.11 | 3E-7 | 2267 |
| | log10(Lutein+Zeaxanthin) | -0.72 | -0.08 | 0.22 | 224 | -0.04 | 0.09 | -0.09 | 1E-5 | 2268 |
| | log10(beta-Cryptoxanthin) | -1.12 | -0.03 | 0.67 | 224 | -0.06 | 2E-3 | -0.11 | 3E-7 | 2268 |
| | log10(alpha-Tocopherol) | 1.16 | -0.03 | 0.71 | 224 | -0.04 | 0.07 | -0.06 | 0.01 | 2268 |
| | log10(gamma-Tocopherol) | 0.27 | 0.06 | 0.36 | 224 | 0.08 | 2E-4 | 0.09 | 9E-6 | 2268 |
| Measurements | log10(C-reactive protein) | 0.47 | 0.06 | 5E-3 | 2073 | 0.08 | 6E-5 | 0.12 | 2E-10 | 2809 |
| | log10(Insulin) | 1.74 | 0.06 | 0.01 | 2051 | 0.07 | 2E-5 | 0.11 | 3E-12 | 4043 |
| | log10(Glucose) | 0.30 | 0.04 | 0.05 | 2091 | 0.06 | 8E-5 | 0.06 | 1E-4 | 4145 |
| | log10(Triglyceride) | 2.11 | 0.07 | 1E-3 | 2091 | 0.05 | 5E-4 | 0.07 | 6E-6 | 4149 |
| | Total cholesterol | 224.61 | 0.00 | 0.92 | 2091 | 0.03 | 0.04 | 0.01 | 0.62 | 4149 |
| | LDL cholesterol | 140.61 | -0.02 | 0.38 | 2057 | 0.03 | 0.06 | 0.01 | 0.41 | 4085 |
| | HDL cholesterol | 52.99 | -0.04 | 0.08 | 2091 | -0.04 | 0.01 | -0.09 | 1E-8 | 4146 |
| | log10(Creatinine) | -0.13 | 0.00 | 0.86 | 2041 | 0.01 | 0.74 | 0.02 | 0.26 | 2748 |
| | Systolic blood pressure | 128.87 | 0.01 | 0.57 | 2107 | 0.04 | 5E-3 | 0.07 | 4E-6 | 4165 |
| | Diastolic blood pressure | 75.69 | 0.01 | 0.75 | 2107 | 0.05 | 3E-3 | 0.04 | 0.01 | 4165 |
| | log10(Waist / hip ratio) | -0.09 | 0.05 | 0.01 | 2107 | 0.05 | 3E-3 | 0.09 | 2E-8 | 4165 |
| Demographics | BMI | 28.98 | 0.04 | 0.04 | 2107 | 0.08 | 1E-6 | 0.09 | 2E-8 | 4165 |
| | Education | 6.75 | -0.07 | 1E-3 | 2085 | -0.02 | 0.14 | -0.10 | 3E-10 | 4130 |
| | Income | 3.44 | -0.04 | 0.05 | 2033 | 0.00 | 0.79 | -0.06 | 1E-4 | 4041 |
| | log10(1+Exercise) | 0.82 | -0.06 | 0.01 | 2101 | -0.04 | 0.01 | -0.07 | 2E-5 | 4142 |
| | Current smoker | 0.00 | -0.01 | 0.79 | 2101 | 0.00 | 0.78 | -0.01 | 0.66 | 4142 |
| | log10(1+Alcohol) | 0.04 | -0.05 | 0.03 | 2100 | -0.02 | 0.22 | -0.07 | 3E-5 | 3687 |

**Table 5. Cross sectional correlations of various variables (diet, lifestyle, demographic) with epigenetic age acceleration in the WHI.** Correlations (bicor, biweight midcorrelation) between select variables and three measures of epigenetic age acceleration: AASkin=epiegnetic age acceleration based on the skin & blood clock, IEAA=intrinsic epigenetic age acceleration, EEAA=extrinsic epigenetic age acceleration. In addition to adjusting for chronologic age, IEAA also adjusts the Horvath (2013) estimator of DNAm age for blood cell count estimates, arriving at a measure that is unaffected by both variation in chronologic age and blood cell composition. EEAA, on the other hand, integrates known age-related changes in blood cell counts with a blood-based measure of epigenetic age before adjusting for chronologic age, making EEAA dependent on age-related changes in blood cell composition [21]. IEAA can be interpreted as a measure of cell-intrinsic aging and EEAA as a measure of immune system aging, where for both, a positive value indicates that the epigenetic age of an individual blood sample is higher than expected based on chronological age. The entries are colored according to their magnitude with positive correlations in red, negative correlations in blue, and statistical significance (p-values) in green. Blood biomarkers were measured from fasting plasma collected at baseline. Food groups and nutrients are inclusive, including all types and all preparation methods, e.g. folic acid includes synthetic and natural, dairy includes cheese and all types of milk. Variables are adjusted for race/ethnicity and dataset. The individual variables (rows) are explained in [22].

**Technical Details surrounding the DNAm age estimator for skin and blood samples**

This section contains technical and statistical details surrounding the invention: "DNA methylation biomarker of aging for human *ex vivo* and *in vivo* studies".

<u>Definition of DNAm Age according to the skin & blood clock</u>

As for the multi-tissue DNAm age estimator (Horvath, S. DNA methylation age of human tissues and cell types. Genome Biol 14, R115, doi:10.1186/gb-2013-14-10-r115 (2013)), the dependent variable, chronological age, was transformed before carrying out an elastic net regression analysis. Toward this end, the following function F for transforming age was used:

▪

$$F(age)=\log(age+1)-\log(adult.age+1) \text{ if } age<=adult.age.$$

▪

$$F(age)=(age-adult.age)/(adult.age+1) \text{ if } age>adult.age.$$

The parameter "adult.age" was set to 20. Note that F satisfies the following desirable properties: it

- i) is a continuous, monotonically increasing function (which can be inverted),
- ii) has a logarithmic dependence on age until adulthood (here set at 20 years),
- iii) has a linear dependence on age after adulthood (here set to 20),
- iv) is defined for negative ages (i.e. prenatal samples) by adding 1 (year) to age in the logarithm,
- v) it has a continuous first derivative (slope function). In particular the slope at age=adult.age is given by 1/(adult.age+1).

An elastic net regression model (implemented in the glmnet R function) was used to regress a transformed version of age on the beta values in the training data. The glmnet function requires the user to specify two parameters (alpha and beta). Since I used an elastic net predictor, alpha was set to 0.5. But the lambda value of was chosen by applying a 10 fold cross validation to the training data (via the R function cv.glmnet). The elastic net regression results in a linear regression model whose coefficients $b_0$, $b_1$, . . . , $b_{391}$ relate to transformed age as follows

$$F(\text{chronological age}) = b_0 + b_1 CpG_1 + \ldots + b_{391} CpG_{391} + \text{error}$$

Based, on the coefficient values from the regression model, DNAmAge is estimated as follows

$$DNAmAge = \text{inverse.} F(b_0 + b_1 CpG_1 + \ldots + b_{391} CpG_{391})$$

where inverse.F(.) denotes the mathematical inverse of the function F(.) and is specified as follows.

-

$$\text{anti.} F(x) = (1 + \text{adult.age}) * \exp(x) - 1 \text{ if } x < 0$$

-

$$\text{anti.} F(x) = (1 + \text{adult.age}) * x + \text{adult.age if } x >= 0$$

- and the parameter adult.age was chosen to be 20.

Thus, the regression model can be used to predict to transformed age value by simply plugging the beta values of the selected CpGs into the formula.

Steps for measuring the DNAmAge based on the Skin & Blood clock

Step 1: Collect human fibroblasts, keratinocytes, buccal cells, endothelial cells, skin, dermis, epidermis, saliva, blood, urine, or other sources of DNA.

Many options exist for collecting or culturing cell samples, e.g. punch biopsy for skin samples, buccal swabs for buccal cells, spit cup for saliva or buccal samples.

Blood tubes collected by venipunture: Blood tubes collected by venipuncture will result in a large amount of high quality DNA from a relevant tissue. The invention applies to DNA from whole blood, or peripheral blood mononuclear cells or even sorted blood cell types.

Dried blood spots can be easily collected by a finger prick method. The resulting blood droplet can be put on a blood card, e.g. http://www.lipidx.com/dbs-kits/.

Step 2: Extract human DNA and generate DNA methylation data

Measure cytosine DNA methylation levels. Several approaches can be used for measuring DNA methylation including sequencing, bisulfite sequencing, arrays, pyrosequencing, liquid chromatography coupled with tandem mass spectrometry.

Our invention applies to any platform used for measuring DNA methylation data. In particular, it can be used in conjunction with the latest Illumina methylation array platform the EPIC array or the older platforms (Infinium 450K array or 27K array). The coefficient values used pertain to the "beta values" whose values lie between 0 and 1 but it could be easily adapted to other metrics of assessing DNA methylation, e.g. "M values".

Step 3: Estimate the DNAmAge based on Skin & Blood clock estimate

The DNAm Age (Skin & Blood) estimate is estimated in two steps.

First, one can optionally form a weighted linear combination of 391 CpGs.

Second, the weighted average of the 391 CpGs can be transformed using a monotonically increasing function so that it is in units of years.

DNAmAge=anti.F(WeightedAverage) where function anti.F() is given by

■

$$\text{anti.F}(x) = (1 + \text{adult.age}) * \exp(x) - 1 \text{ if } x < 0$$

■

$$\text{anti.F}(x) = (1 + \text{adult.age}) * x + \text{adult.age} \text{ if } x >= 0$$

■ and the parameter adult.age was chosen to be 20.

■

■ This application references a number of different publications as indicated throughout the specification by reference numbers. Lists of these different publications ordered according to these reference numbers can be found above and below.

■

■ REFERENCES

■ The following references are cited in, and pertain to, the disclosure immediately above this section but not Example 2 below.

■ 1. Fraga MF and Esteller M. Epigenetics and aging: the targets and the marks. Trends in Genetics. 2007; 23(8):413-418.

■ 2. Rakyan VK, Down TA, Maslau S, Andrew T, Yang TP, Beyan H, Whittaker P, McCann OT, Finer S, Valdes AM, Leslie RD, Deloukas P and Spector TD. Human aging-associated DNA hypermethylation occurs preferentially at bivalent chromatin domains. Genome research. 2010; 20(4):434-439.

■ 3. Teschendorff AE, Menon U, Gentry-Maharaj A, Ramus SJ, Weisenberger DJ, Shen H, Campan M, Noushmehr H, Bell CG, Maxwell AP, Savage DA, Mueller-Holzner E, Marth C, et al. Age-dependent DNA methylation of genes that are suppressed in stem cells is a hallmark of cancer. Genome research. 2010; 20(4):440-446.

■ 4. Jung M and Pfeifer GP. Aging and DNA methylation. BMC biology. 2015; 13(1):1-8.

■ 5. Zheng SC, Widschwendter M and Teschendorff AE. Epigenetic drift, epigenetic clocks and cancer risk. Epigenomics. 2016; 8(5):705-719.

■ 6. Bocklandt S, Lin W, Sehl ME, Sanchez FJ, Sinsheimer JS, Horvath S and Vilain E. Epigenetic predictor of age. PLoS One. 2011; 6(6):e14821.

■ 7. Garagnani P, Bacalini MG, Pirazzini C, Gori D, Giuliani C, Mari D, Di Blasio AM, Gentilini D, Vitale G, Collino S, Rezzi S, Castellani G, Capri M, et al. Methylation of ELOVL2 gene as a new epigenetic marker of age. Aging Cell. 2012; 11(6):1132-1134.

■ 8. Hannum G, Guinney J, Zhao L, Zhang L, Hughes G, Sadda S, Klotzle B, Bibikova M, Fan JB, Gao Y, Deconde R, Chen M, Rajapakse I, et al. Genome-wide methylation profiles reveal quantitative views of human aging rates. Mol Cell. 2013; 49(2):359-367.

■ 9. Horvath S. DNA methylation age of human tissues and cell types. Genome Biol. 2013; 14(10):R115.

■ 10. Weidner CI, Lin Q, Koch CM, Eisele L, Beier F, Ziegler P, Bauerschlag DO, Jockel KH, Erbel R, Muhleisen TW, Zenke M, Brummendorf TH and Wagner W. Aging of blood can be tracked by DNA methylation changes at just three CpG sites. Genome Biol. 2014; 15(2):R24.

■ 11. Lin Q, Weidner CI, Costa IG, Marioni RE, Ferreira MR, Deary IJ and Wagner W. DNA methylation levels at individual age-associated CpG sites can be indicative for life expectancy. Aging (Albany NY). 2016; 8(2):394-401.

■ 12. Horvath S and Raj K. DNA methylation-based biomarkers and the epigenetic clock theory of ageing. Nat Rev Genet. 2018.

■ 13. Nwanaji-Enwerem JC, Weisskopf MG and Baccarelli AA. Multi-tissue DNA methylation age: Molecular relationships and perspectives for advancing biomarker utility. Ageing Res Rev. 2018; 45:15-23.

■ 14. Horvath S, Erhart W, Brosch M, Ammerpohl O, von Schonfels W, Ahrens M, Heits N, Bell JT, Tsai PC, Spector TD, Deloukas P, Siebert R, Sipos B, et al. Obesity accelerates epigenetic aging of human liver. Proc Natl Acad Sci U S A. 2014; 111(43):15538-15543.

■ 15. Marioni R, Shah S, McRae A, Chen B, Colicino E, Harris S, Gibson J, Henders A, Redmond P, Cox S, Pattie A, Corley J, Murphy L, et al. DNA methylation age of blood predicts all-cause mortality in later life. Genome Biol. 2015; 16(1):25.

■ 16. Horvath S, Garagnani P, Bacalini MG, Pirazzini C, Salvioli S, Gentilini D, Di Blasio AM, Giuliani C, Tung S, Vinters HV and Franceschi C. Accelerated epigenetic aging in Down syndrome. Aging Cell. 2015; 14(3):491-495.

■ 17. Horvath S and Levine AJ. HIV-1 Infection Accelerates Age According to the Epigenetic Clock. J Infect Dis. 2015; 212(10):1563-1573.

■ 18. Horvath S, Mah V, Lu AT, Woo JS, Choi OW, Jasinska AJ, Riancho JA, Tung S, Coles NS, Braun J, Vinters HV and Coles LS. The cerebellum ages slowly according to the epigenetic clock. Aging (Albany NY). 2015; 7(5):294-306.

■ 19. Levine ME, Hosgood HD, Chen B, Absher D, Assimes T and Horvath S. DNA methylation age of blood predicts future onset of lung cancer in the women's health initiative. Aging (Albany NY). 2015; 7(9):690-700.

■ 20. Levine ME, Lu AT, Chen BH, Hernandez DG, Singleton AB, Ferrucci L, Bandinelli S, Salfati E, Manson JE, Quach A, Kusters CD. Kuh D, Wong A, et al. Menopause accelerates biological aging. Proc Natl Acad Sci U S A. 2016; 113(33):9327-9332.

■ 21. Chen BH, Marioni RE, Colicino E, Peters MJ, Ward-Caviness CK, Tsai PC, Roetker NS, Just AC, Demerath EW, Guan W, Bressler J, Fornage M, Studenski S, et al. DNA methylation-based measures of biological age: meta-analysis predicting time to death. Aging (Albany NY). 2016; 8(9):1844-1865.

■ 22. Quach A, Levine ME, Tanaka T, Lu AT, Chen BH, Ferrucci L, Ritz B, Bandinelli S, Neuhouser ML, Beasley JM, Snetselaar L, Wallace RB, Tsao PS, et al. Epigenetic clock analysis of diet, exercise, education, and lifestyle factors. Aging (Albany NY). 2017; 9(2):419-446.

■ 23. Dugue PA, Bassett JK, Joo JE, Jung CH, Ming Wong E, Moreno-Betancur M, Schmidt D, Makalic E, Li S, Severi G, Hodge AM, Buchanan DD, English DR, et al. DNA methylation-based biological aging and cancer risk and survival: Pooled analysis of seven prospective studies. Int J Cancer. 2017.

■ 24. Simpkin AJ, Howe LD, Tilling K, Gaunt TR, Lyttleton O, McArdle WL, Ring SM, Horvath S, Smith GD and Relton CL. The epigenetic clock and physical development during childhood and adolescence: longitudinal analysis from a UK birth cohort. Int J Epidemiol. 2017; 46(2):549-558.

■ 25. Maierhofer A, Flunkert J, Oshima J, Martin GM, Haaf T and Horvath S. Accelerated epigenetic aging in Werner syndrome. Aging (Albany NY). 2017; 9(4):1143-1152.

■ 26. Walker RF, Liu JS, Peters BA, Ritz BR, Wu T, Ophoff RA and Horvath S. Epigenetic age analysis of children who seem to evade aging. Aging (Albany NY). 2015; 7(5):334-339.

• 27. Merideth MA, Gordon LB, Clauss S, Sachdev V, Smith AC, Perry MB, Brewer CC, Zalewski C, Kim HJ, Solomon B, Brooks BP, Gerber LH, Turner ML, et al. Phenotype and course of Hutchinson-Gilford progeria syndrome. N Engl J Med. 2008; 358(6):592-604.

■ 28. Gordon LB, Kleinman ME, Massaro J, D'Agostino RB, Sr., Shappell H, Gerhard-Herman M, Smoot LB, Gordon CM, Cleveland RH, Nazarian A, Snyder BD, Ullrich NJ. Silvera VM, et al. Clinical Trial of the Protein Farnesylation Inhibitors Lonafamib, Pravastatin, and Zoledronic Acid in Children With Hutchinson-Gilford Progeria Syndrome. Circulation. 2016; 134(2):114-125.

■ 29. Eriksson M, Brown W, Gordon L, Glynn M, Singer J, Scott L, Erdos M, Robbins C, Moses T, Berglund P, Dutra A, Pak E, Durkin S, et al. Recurrent de novo point mutations in lamin A cause Hutchinson-Gilford progeria syndrome. Nature. 2003; 423(6937):293-298.

■ 30. Moulson CL, Fong LG, Gardner JM, Farber EA, Go G, Passariello A, Grange DK, Young SG and Miner JH. Increased progerin expression associated with unusual LMNA mutations causes severe progeroid syndromes. Hum Mutat. 2007; 28(9):882-889.

■ 31. Bar DZ, Arlt MF, Brazier JF, Norris WE, Campbell SE, Chines P, Larrieu D, Jackson SP, Collins FS, Glover TW and Gordon LB. A novel somatic mutation achieves partial rescue in a child with Hutchinson-Gilford progeria syndrome. J Med Genet. 2017; 54(3):212-216.

■ 32. Ahmad Z, Zackai E, Medne L and Garg A. Early onset mandibuloacral dysplasia due to compound heterozygous mutations in ZMPSTE24. Am J Med Genet A. 2010; 152A(11):2703-2710.

■ 33. Barrowman J, Wiley PA, Hudon-Miller SE, Hrycyna CA and Michaelis S. Human ZMPSTE24 disease mutations: residual proteolytic activity correlates with disease severity. Hum Mol Genet. 2012; 21(18):4084-4093.

- 34. Bai S, Lozada A, Jones MC, Dietz HC, Dempsey M and Das S. Mandibuloacral Dysplasia Caused by LMNA Mutations and Uniparental Disomy. Case Rep Genet. 2014; 2014:508231.
- 35. Verstraeten VL, Broers JL, van Steensel MA, Zinn-Justin S, Ramaekers FC, Steijlen PM, Kamps M, Kuijpers HJ, Merckx D, Smeets HJ. Hennekam RC, Marcelis CL and van den Wijngaard A. Compound heterozygosity for mutations in LMNA causes a progeria syndrome without prelamin A accumulation. Hum Mol Genet. 2006; 15(16):2509-2522.
- 36. Hisama FM, Lessel D, Leistritz D, Friedrich K, McBride KL, Pastore MT, Gottesman GS, Saha B, Martin GM, Kubisch C and Oshima J. Coronary artery disease in a Werner syndrome-like form of progeria characterized by low levels of progerin, a splice variant of lamin A. Am J Med Genet A. 2011; 155A(12):3002-3006.
- 37. Barthelemy F, Navarro C, Fayek R, Da Silva N, Roll P, Sigaudy S, Oshima J, Bonne G, Papadopoulou-Legbelou K, Evangeliou AE, Spilioti M, Lemerrer M, Wevers RA, et al. Truncated prelamin A expression in HGPS-like patients: a transcriptional study. Eur J Hum Genet. 2015; 23(8):1051-1061.
- 38. Gordon LB, Massaro J, D'Agostino RB, Sr., Campbell SE, Brazier J, Brown WT, Kleinman ME and Kieran MW. Impact of farnesylation inhibitors on survival in Hutchinson-Gilford progeria syndrome. Circulation. 2014; 130(1):27-34.
- 39. Lu AT, Xue L, Salfati EL, Chen BH, Ferrucci L, Levy D, Joehanes R, Murabito JM, Kiel DP, Tsai PC, Yet I, Bell JT, Mangino M, et al. GWAS of epigenetic aging rates in blood reveals a critical role for TERT. Nat Commun. 2018; 9(1):387.
- 40. Lowe D, Horvath S and Raj K. Epigenetic clock analyses of cellular senescence and ageing. Oncotarget. 2016; 7(8):8524-8531.
- 41. Cao K, Graziotto JJ, Blair CD, Mazzulli JR, Erdos MR, Krainc D and Collins FS. Rapamycin reverses cellular phenotypes and enhances mutant protein clearance in Hutchinson-Gilford progeria syndrome cells. Sci Transl Med. 2011; 3(89):89ra58.
- 42. Park SK and Shin OS. Metformin alleviates ageing cellular phenotypes in Hutchinson-Gilford progeria syndrome dermal fibroblasts. Exp Dermatol. 2017; 26(10):889-895.
- 43. Kubben N, Zhang W, Wang L, Voss TC, Yang J, Qu J, Liu GH and Misteli T. Repression of the Antioxidant NRF2 Pathway in Premature Aging. Cell. 2016; 165(6):1361-1374.
- 44. Friedman J, Hastie T and Tibshirani R. Regularization Paths for Generalized Linear Models via Coordinate Descent. Journal of Statistical Software. 2010; 33(1):1-22.
- 45. Triche TJ, Weisenberger DJ, Van Den Berg D, Laird PW and Siegmund KD. Low-level processing of Illumina Infinium DNA Methylation BeadArrays. Nucleic Acids Research. 2013; 41(7):e90-e90.
- 46. Hub CJ, Zhang B, Victor MB, Dahiya S, Batista LF, Horvath S and Yoo AS. Maintenance of age in human neurons generated by microRNA-based neuronal conversion of fibroblasts. Elife. 2016; 5:e18648.
- 47. Levine ME, Lu AT, Quach A, Chen BH, Assimes TL, Bandinelli S, Hou L, Baccarelli AA, Stewart JD, Li Y, Whitsel EA, Wilson JG, Reiner AP, et al. An epigenetic biomarker of aging for lifespan and healthspan. Aging (Albany NY). 2018.
- 48. Houseman E, Accomando W, Koestler D, Christensen B, Marsit C, Nelson H, Wiencke J and Kelsey K. DNA methylation arrays as surrogate measures of cell mixture distribution. BMC Bioinformatics. 2012; 13(1):86.
- 49. Horvath S, Gurven M, Levine ME, Trumble BC, Kaplan H, Allayee H, Ritz BR, Chen B, Lu AT, Rickabaugh TM, Jamieson BD, Sun D, Li S, et al. An epigenetic clock analysis of race/ethnicity, sex, and coronary heart disease. Genome Biol. 2016; 17(1):171.
- 50. Horvath S and Ritz BR. Increased epigenetic age and granulocyte counts in the blood of Parkinson's disease patients. Aging (Albany NY). 2015; 7(12):1130-1142.
- 51. Rojas D, Rager JE, Smeester L, Bailey KA, Drobna Z, Rubio-Andrade M, Styblo M, Garcia-Vargas G and Fry RC. Prenatal arsenic exposure and the epigenome: identifying sites of 5-methylcytosine alterations that predict functional changes in gene expression in newborn cord blood and subsequent birth outcomes. Toxicol Sci. 2015; 143(1):97-106.
- 52. Knight AK, Craig JM, Theda C, Bakvad-Hansen M, Bybjerg-Grauholm J, Hansen CS, Hollegaard MV, Hougaard DM, Mortensen PB, Weinsheimer SM, Werge TM, Brennan PA, Cubells JF, et al. An epigenetic clock for gestational age at birth based on blood methylation data. Genome Biology. 2016; 17(1):206.
- 53. Viechtbauer W. Conducting Meta-Analyses in R with the metafor Package. J Statistical Software. 2010; 36(3):1-48.

## EXAMPLE 2: RAPAMYCIN RETARDS EPIGENETIC AGEING IN KERATINOCYTES INDEPENDENTLY OF ITS EFFECT ON REPLICATIVE SENESCENCE, PROLIFERATION AND DIFFERENTIATION

The advent of epigenetic clocks has prompted questions about the place of epigenetic ageing within the current understanding of ageing biology. It was hitherto unclear whether epigenetic ageing represents a distinct mode of ageing or a manifestation of a known characteristic of ageing. We report here that epigenetic ageing is not affected by replicative senescence, telomere length, somatic cell differentiation, cellular proliferation rate or frequency. It is instead retarded by rapamycin, the potent inhibitor of the mTOR complex which governs many pathways relating to cellular metabolism. Rapamycin however, is also an effective inhibitor of cellular senescence. Hence cellular metabolism underlies two independent arms of ageing - cellular senescence and epigenetic ageing. The demonstration that a compound that targets metabolism can slow epigenetic ageing provides a long-awaited point-of-entry into elucidating the molecular pathways that underpin the latter. Lastly, we report here an *in vitro* assay, validated in humans, that recapitulates human epigenetic ageing that can be used to investigate and identify potential interventions that can inhibit or retard it.

One of the biggest challenges in ageing research is the means of measuring age independently of time. This need becomes particularly clear when we wish to evaluate the effects of drugs or compounds on ageing, where the use of time as a measure of age is clearly inappropriate. In recent years, several age-estimators known as epigenetic clocks have been developed, which are based on methylation states of specific CpGs, some of which become increasingly methylated, while others decreasingly methylated with age (Horvath and Raj, 2018). Age estimated by these clocks is referred to as epigenetic age or more precisely, DNA methylation age (DNAm age). The "ticking" of these clocks is constituted by methylation changes that occur at specific CpGs of the genome. Significantly, the increased rate by which these specific methylation changes occur is associated with many age-related health conditions (Horvath, 2013, Horvath and Raj, 2018, Horvath et al., 2018, Horvath et al., 2014, Horvath et al., 2015a, Horvath et al., 2016a, Horvath et al., 2016b, Horvath et al., 2015b, Horvath and Ritz, 2015), indicating that epigenetic clocks, capture biological ageing (epigenetic ageing) at least to some extent. The numerous epigenetic clocks that have been independently developed (Hannum et al., 2013, Weidner et al., 2014, Eipel et al., 2016, Koch and Wagner, 2011, Bocklandt et al., 2011, Hernandez et al., 2011, Florath et al., 2014) differ in accuracy, biological interpretation and applicability, whereby some epigenetic clocks are compatible only to some tissues such as blood. In this regard, the pan-tissue epigenetic clock (Horvath, 2013) stands out because it is applicable to virtually all tissues of the body, with the exception of sperm. It estimates the same epigenetic age for different post-mortem tissues (except the cerebellum and female breast) from the same individual (Horvath, 2013, Horvath et al., 2015b). Although the pan-tissue epigenetic clock performs extremely well with *in vivo* cell samples, its accuracy was not as good with fibroblasts and other *in vitro* cell samples. We addressed this recently by developing an even more accurate multi-tissue age estimator, which we refer to as skin & blood clock (Horvath et al., 2018), which is applicable for *in vivo* as well as *in vitro* samples of human fibroblasts, keratinocytes, buccal cells, blood cells, saliva and endothelial cells. *In vitro* human cell culture systems offer many advantages including tight control of growth conditions, nutrients, cell proliferation rates, detailed morphological analyses and genetic manipulation, all of which are impractical or inappropriate in human cohort studies. Hence the availability of an *in vivo* epigenetic clock, such as the skin & blood clock that can also be used for *in vitro* experiments is an important and significant step towards uncovering the molecular mechanisms that underpin epigenetic ageing.

Although the molecular mechanisms of epigenetic ageing remain largely uncharacterised, the cellular aspects however, have been explored to a greater albeit limited degree. The similar epigenetic ages detected amongst different tissue of the same body (Horvath, 2013, Horvath et al., 2015b) suggests that epigenetic age is not a measure of cellular proliferation since the rate and frequency of proliferation differ greatly between different tissues such as blood, which is highly proliferative and heart cells, which are post-mitotic. It is intuitive to make a connection between epigenetic ageing and senescent cells, which increases in number with age and which mediates phenotypic ageing (Horvath et al., 2015b, Munoz-Espin and Serrano, 2014). This attractive link however, was discounted by previous reports which clearly excluded DNA damage, telomere attrition and cellular senescence as drivers of epigenetic aging (Kabacik et al., 2018).

A way to further characterise epigenetic ageing is through the evaluation of validated anti-aging interventions on it. Such an intervention is the nutrient response pathway regulated by the mammalian target of rapamycin (mTOR) (Sharp et al., 2013, Betz and Hall, 2013, Cornu et al., 2013). Although originally developed as an immunosuppressant, rapamycin has emerged as one of the most impressive life-extending compounds (Ehninger et al., 2014). It has been repeatedly shown to extend the lives of different animal species including those of yeast (Powers et al., 2006), flies (Bjedov et al., 2010) and mice (Harrison et al., 2009, Zhang et al., 2014). The structure of rapamycin presents two major sites for potential interactions. The binding of one site to FKBP12 protein, allows its other site to bind and inhibit the mTOR kinase (Choi et al., 1996). This kinase is part of a complex that promotes cell growth, proliferation and cell survival (Stanfel et al., 2009, Johnson et al., 2013). This may be why mTOR activity is often elevated in cancer cells; the rationale behind its use as an anti-cancer drug (Ilagan and Manning, 2016). By inhibiting mTOR activity, rapamycin also recapitulates to some extent, the effect of calorie-restriction, which has also been repeatedly shown to prolong the lives of many different animal species (Heilbronn and Ravussin, 2003). As such, rapamycin is widely considered to be a promising anti-ageing intervention. Here we characterise epigenetic aging in primary human keratinocytes from multiple donors by testing their sensitivities to rapamycin and we observed that it can indeed mitigate epigenetic ageing independently of cellular senescence, proliferation, differentiation and telomere elongation.

**Results**

**Opposing effects of Rapamycin and ROCK inhibitor on keratinocyte proliferation**

The availability of an epigenetic clock, such as the skin & blood clock, which is applicable to cultured cells, allows epigenetic ageing to be studied beyond the purely descriptive nature afforded by epidemiological analyses alone. Towards this end, we have established *in vitro* epigenetic ageing systems using primary human cells. One of this is based on primary keratinocytes that are derived from healthy human skins. As previously reported by others, we observed that the proliferation rate of these cells, which is defined as the number of population doublings per unit of time, can be significantly altered by different compounds. Rapamycin, which is the primary focus of this investigation reduces cellular proliferation rate, while Y-27632, which inhibits Rho kinase (ROCK inhibitor) increases it, and a mixture of both modestly alleviates the repressive effect of rapamycin. The opposing effects of these compounds on keratinocyte proliferation present us with the opportunity to test whether cellular proliferation rate impacts epigenetic ageing while carrying out our primary aim of interrogating the effects of rapamycin on epigenetic ageing.

Effects of **Rapamycin and Y-27632 on epigenetic ageing**

Primary keratinocytes were isolated from human neonatal foreskins from three donors (Donor A, B and C) and were put in culture with standard media or media supplemented with rapamycin, Y-27632 or a cocktail of both of these compounds (methods). The cells were passaged continually and population doublings at each passage recorded. In time all cells, regardless of donor or treatment underwent replicative senescence, where they ceased to increase their numbers after at least 2 weeks in culture with regular replenishment of media. Interestingly, two of the three donor cells treated with rapamycin underwent further proliferation before replicative senescence, indicating that their proliferative capacity was increase. This was also observed with Y -27632-treated cells. DNA methylation profiles from a selection of passages of these cells were obtained and analysed with the skin & blood clock. It is clear that while Y-27632 did not impose any appreciable effect, rapamycin retarded epigenetic ageing of these cells. This is evident even when Y-27632 was present with rapamycin. These empirical observations demonstrate three fundamental features of epigenetic ageing. First, increased cellular proliferation rate, as instigated by Y-27632 does not affect epigenetic ageing. This echoes the conclusion derived from analyses of *in vivo* tissues, using the pan-tissue age estimator (Horvath, 2013) and confirmed by Yang et al.(Yang et al., 2016) who specifically derived a DNA methylation-based mitotic clock to be able to measure cellular proliferation, as epigenetic ageing clocks were not able to do so. Second, increased proliferative capacity (the number of times cells proliferate before replicative senescence) is not inextricably linked with retardation of epigenetic ageing since rapamycin and Y-27632 can instigate the former, but only rapamycin-treated cells exhibited retardation of epigenetic ageing. Third, epigenetic ageing is not a measure of replicative senescence since all rapamycin-treated cells eventually underwent replicative senescence and yet remained younger than the un-treated control cells; an observation that would not be made were epigenetic age a measure of senescent cells.

**Somatic cell differentiation does not drive epigenetic ageing**

Having ruled out cellular proliferation rate and proliferation capacity, as well as replicative senescence as drivers of epigenetic ageing, we considered the possible role of somatic cell differentiation in this regard. We observed that healthy primary keratinocytes in culture are heterogeneous in size and shape, but those that were growing in the presence of rapamycin were much more regular in shape and have considerably fewer enlarged cells. Staining with antibodies against p16; a marker of senescent cells (Rayess et al., 2012), and involucrin; a marker of early keratinocyte differentiation (Rice et al., 1992), showed that the enlarged cells were a mixture of senescent cells and differentiating cells, with some cells exhibiting both markers. As our previous investigations (Kabacik et al., 2018) and observations above have uncoupled cellular senescence from epigenetic ageing, we questioned whether cellular differentiation could instead be the driver and the ability of rapamycin to reduce spontaneous differentiation may be the way by which it retards epigenetic ageing.

In the experiments described thus far, primary keratinocytes were grown in a culture condition where the medium used (CnT-07) was designed with the expressed purpose of encouraging the proliferation of progenitor keratinocytes, while restricting their spontaneous differentiation; evidently not eliminating it altogether. To test the hypothesis that cellular differentiation drives epigenetic ageing, we opted to encourage spontaneous keratinocyte differentiation to see if this would cause a rise in their epigenetic age. To this end, we cultured human primary keratinocytes in a different medium, as reported by Rheinwald and Green (Rheinwald and Green, 1975), and with mouse 3T3 cells, which serve as feeder cells. Crucially, this culture condition which we term RG not only supports the proliferation of keratinocytes, it also permits their spontaneous differentiation to a much greater extent than does CnT media.

Primary keratinocytes from the same human donor (Donor D) were cultured in these two different conditions described above (CnT and RG). DNA methylation profiles from four passages of cells, with known number of population doubling were obtained and their ages were estimated by the skin & blood clock. Encouraging greater keratinocyte differentiation by culturing them in RG condition did not increase epigenetic ageing, demonstrating that contrary to the hypothesis, epigenetic ageing is not increased by greater keratinocyte differentiation and therefore the retardation of epigenetic ageing by rapamycin is not mediated through its suppression of spontaneous somatic cell differentiation. Collectively, these experiments have demonstrated that rapamycin is an effective retardant of epigenetic ageing, and that this activity is mediated independently of its effects on replicative senescence and somatic cell differentiation.

**Discussion**

It is widely assumed that extension of lifespan is a result of retardation of ageing. While there is no counter-evidence to challenge this highly intuitive association, supporting empirical evidence to confirm it is not easy to acquire. As a case in point, improvement in public health in the past century has extended life-span, but there is no directly measurable evidence that this was accompanied by a reduction in the rate of ageing. The same question could be asked of any intervention that purports to extend life. The scarcity of empirical evidence is due in part to the lack of a good measure of age that is not based on time. In this regard, the relatively recent development of epigenetic clocks is of great interest (Horvath and Raj, 2018). Despite their impressive performance, almost nothing is known about the molecular components and pathways that underpin them. At the cellular level however, more is known, but from the perspective of what epigenetic ageing is not, rather than what it is. The bringing together of rapamycin and the skin & blood clock in the experiments above have shed light on both of them. This has been significantly enhanced by comparison with the effects, or not, of the Rho kinase inhibitor, Y-27632. As a case in point, the retardation of epigenetic ageing by rapamycin could have been erroneously ascribed to the retardation of the rate of keratinocyte proliferation, were it not for the fact that Y-27632 augments proliferation rate but does not increase epigenetic ageing. This precludes a simplistic and incorrect correlation between the rate of cellular proliferation and epigenetic ageing. Recently Yang et al demonstrated that epigenetic ageing clock tracks cellular proliferation very poorly compared to the purpose-built DNA methylation-based mitotic clock (Yang et al., 2016).

The impulse to turn our attention and ascribe retardation of epigenetic ageing to reduced senescent cells is understandable since rapamycin does indeed reduce the emergence of these cells in cultures, as consistent with previous characterisation and description (Leontieva et al., 2015, Leontieva and Blagosklonny, 2016, Leontieva and Blagosklonny, 2017, Blagosklonny, 2018, Wang et al., 2017, Herranz et al., 2015). This notion however is inconsistent with our previous finding that the epigenetic age of a cellular population is not dependent on the presence of senescent cells (Kabacik et al., 2018), and this conclusion is further confirmed here, where all the rapamycin-treated cells eventually senesced, without any rise in their epigenetic age. Therefore, while rapamycin's inhibition of senescence is not in doubt, this is not the means by which it retards the progression of epigenetic age of keratinocytes.

To test whether somatic cell differentiation drives epigenetic ageing, we refrained from using chemical means to induce terminal differentiation of keratinocytes as this could introduce DNA methylation changes that might confound interpretation of the results. Instead, we exploited the propensity of keratinocytes to spontaneously differentiate, which they do significantly better in RG medium than in CnT-07 medium (Green et al., 1977). The hypothesis that differentiation drives epigenetic ageing was clearly refuted by these observations. While we still do not know what cellular feature is associated with epigenetic ageing, we can now remove somatic cell differentiation from the list of possibilities and place it with cellular senescence, proliferation and telomere length maintenance, which represent cellular features that are all not linked to epigenetic ageing.

The ability of rapamycin to suppress the progression of epigenetic ageing is very encouraging for many reasons not least because it provides a valuable point-of-entry into molecular pathways that are potentially associated with it. Evidently, the target of rapamycin, the mTOR complex is of particular interest. It acts to promote many processes including, but not limited to protein synthesis, autophagy, lipid synthesis and glycolysis (Johnson et al., 2013, Weichhart, 2018, Kim and Guan, 2019). The experiments above were not designed to identify the specific mTOR activity or activities that underpin epigenetic ageing, but they point to further experiments involving gene manipulation and drugs that could be brought to address this question. It is of great significance that we have previously identified through genome-wide association studies (GWAS), genetic variants near MLST8 coding region whose expression levels are positively correlated with epigenetic aging rates in human cerebellum (Lu et al., 2016). MLST8 is a subunit of the mTORC1 and mTORC2 complexes, and its gene expression levels increase with chronological age in multiple brain regions (Lu et al., 2016). It is pivotal for mTOR function as its deletion prevents the formation of mTORC1 and mTORC2 complexes (Kakumoto et al., 2015). The convergence of the GWAS observation with the experimental system described here is a testament of the strength of the skin & blood clock in uncovering biological features that are consistent between the human level and cellular level. It lends weight to the emerging view that the mTOR pathway may be the underlying mechanism that supports epigenetic ageing.

It is of interest to note that the experimental set-up above constitutes an *in vitro* ageing assay that is applicable not only to pure research but to screening and discovering other compounds and treatments that may mitigate or suppress epigenetic ageing. Most biological models of human diseases or conditions are derived from molecular, cellular or animal systems that rightly require rigorous validation in humans. In this regard, the epigenetic clock is distinct in being derived from, and validated at the human level. Hence *in vitro* experimental observations made with it carry a significant level of relevance and can be readily compared with an already available collection of human data generated by the epigenetic clock - the MSLT8 described above is an example in point. An added advantage of such a validated *in vitro* ageing system for human cells is the ability to test the efficacy of potential mitigators of ageing in a well-controlled manner, within a relatively short time, at a significantly low cost and with the ability to ascertain whether the effects are on life-span, ageing or both; all of which are not readily achieved with human cohort studies.

In summary, the observations above represent the first biological connection between epigenetic ageing and rapamycin. These results for human cells add to the evidence that extension of life, at least by rapamycin, is indeed accompanied by retardation of ageing. These observations also suggest that the life-extending property of rapamycin may be a resultant of its multiple actions which include, but not necessarily limited to suppression of cellular senescence (Leontieva and Blagosklonny, 2016, Leontieva and Blagosklonny, 2017, Leontieva et al., 2014, Leontieva et al., 2015) and epigenetic aging, with the possibility of augmentation of cellular proliferative potential.

## Materials and Methods

### *In vitro* cultured cell procedure

### Isolation and culture of primary keratinocytes

Primary human neonatal fibroblasts were isolated from circumcised foreskins. Informed consent was obtained prior to collection of human skin samples with approval from the Oxford Research Ethics Committee; reference 10/H0605/1. The tissue was cut into small pieces and digested overnight at 4 °C with 0.5 mg/ml Liberase DH in CnT-07 keratinocyte medium (CellnTech) supplemented with penicillin/streptomycin (Sigma) and gentamycin/amphotericin (Life Tech). Following digestion, the epidermis was peeled off from the tissue pieces and placed in 1 millilitre (ml) of trypsin-versene. After approximately 5 minutes of physical desegregation with forceps, 4 ml of soybean trypsin inhibitor was added to the cell suspension and transferred into a tube for centrifugation at 1,200 revolutions per minute for 5 minutes. The cell pellet was resuspended in CnT-07 media and seeded into fibronectin/collagen-coated plates. Cells were grown at 37 °C, with 5% $CO_2$ in a humidified incubator. Growth medium was changed every other day. Upon confluence, cells were trypsinised, counted and 100,000 were seeded into fresh fibronectin/collagen-coated plates. Population doubling was calculate using the following formula: [Log(number of harvested cells)- log(number of seeded cells)] X 3.32. Rapamycin was used at 25nM and Y-27632 at 1$\mu$M concentrations and were present in the media of treated cells for the entire duration of the experiments. RG medium was prepared by mixing three parts of F12 medium with one part DMEM, supplemented with 5% foetal calf serum, 0.4ug/ml hydrocortisone, 8.4ng/ml cholera toxin, 5ug/ml insulin, 24ug/ml adenine and 10ng/ml epidermal growth factor. 3T3-J2 cells were cultured in DMEM supplemented with 10% foetal calf serum. To prepare feeder cells, 3T3-J2 cells were irradiated at 60Gy and seeded onto fibronectin/collagen-coated plates in RG medium at least 6 hours but no more than 24 hours prior to seeding of keratinocytes. To harvest keratinocytes grown in RG media, feeder cells were first removed with squirting of the monolayer with trypsin-versene for approximately 3 minutes, after which the monolayer was rinsed with 7ml of Phosphate Buffered Saline (PBS) followed by incubation of the monolayer with 0.5ml of trypsin-versene. When all the keratinocytes have lifted off the plate, 1ml of soybean trypsin inhibitor was added to the cell suspension. Cells were counted and 100,000 were seeded into fresh plates as described above.

### *Immunofluorescence*

Cells were grown on glass coverslips that were pre-coated with fibronectin-collagen. When ready, the cells were fixed with formalin for 10 minutes, followed by three rinses with Phosphate Buffered Saline (PBS). Cell membranes were permeabilised with 0.5% TritonX-100 for 15 minutes followed by three 5 minute rinses with PBS. Primary antibodies diluted in 2% foetal calf serum in PBS were added to the cells. After 1 hour the antibodies were removed followed by three 5 minute rinsing, after which secondary antibodies (diluted in 2% foetal calf serum in PBS) was added. After 30minutes, the antibodies were removed and the cells were rinsed five times with 1ml PBS each time for five minutes followed by a final rinse in 1 ml distilled water before mounting on glass slide with Vectastain. Cells were imaged using a fluorescence microscope. Antibodies used were as follows: Anti-Involucrin (Abcam ab53112) diluted at 1:1000 and Anti-p16 (Bethyl laboratories A303-930A-T) diluted at 1:500.

*DNA methylation studies and epigenetic clock*

DNA was extracted from cells using the Zymo Quick DNA mini-prep plus kit (D4069) according to the manufacturer's instructions and DNA methylation levels were measured on Illumina 850 EPIC arrays according to the manufacturer's instructions. The Illumina BeadChips (EPIC or 450K) measures bisulfite-conversion-based, single-CpG resolution DNAm levels at different CpG sites in the human genome. These data were generated by following the standard protocol of Illumina methylation assays, which quantifies methylation levels by the $\beta$ value using the ratio of intensities between methylated and un-methylated alleles. Specifically, the $\beta$ value is calculated from the intensity of the methylated (M corresponding to signal A) and un-methylated (U corresponding to signal B) alleles, as the ratio of fluorescent signals $\beta$ = Max(M,0)/[Max(M,0)+ Max(U,0)+100]. Thus, $\beta$ values range from 0 (completely un-methylated) to 1 (completely methylated). We used the "noob" normalization method, which is implemented in the "minfi" R package (Triche et al., 2013, Fortin et al., 2017). The mathematical algorithm and available software underlying the skin & blood clock (based on 391 CpGs) is presented in Horvath et al., 2018 (Horvath et al., 2018).

## Example 2 References

BETZ, C. & HALL, M. N. 2013. Where is mTOR and what is it doing there? J Cell Biol, 203, 563-74.

BJEDOV, I., TOIVONEN, J. M., KERR, F., SLACK, C., JACOBSON, J., FOLEY, A. & PARTRIDGE, L. 2010. Mechanisms of life span extension by rapamycin in the fruit fly Drosophila melanogaster. Cell Metab, 11, 35-46.

BLAGOSKLONNY, M. V. 2018. Rapamycin, proliferation and geroconversion to senescence. Cell Cycle, 1-11.

BOCKLANDT, S., LIN, W., SEHL, M. E., SANCHEZ, F. J., SINSHEIMER, J. S., HORVATH, S. & VILAIN, E. 2011. Epigenetic predictor of age. PLoS One, 6, e14821.

CHOI, J., CHEN, J., SCHREIBER, S. L. & CLARDY, J. 1996. Structure of the FKBP12-rapamycin complex interacting with the binding domain of human FRAP. Science, 273, 239-42.

CORNU, M., ALBERT, V. & HALL, M. N. 2013. mTOR in aging, metabolism, and cancer. Curr Opin Genet Dev, 23, 53-62.

EHNINGER, D., NEFF, F. & XIE, K. 2014. Longevity, aging and rapamycin. Cell Mol Life Sci, 71, 4325-46.

EIPEL, M., MAYER, F., ARENT, T., FERREIRA, M. R., BIRKHOFER, C., GERSTENMAIER, U., COSTA, I. G., RITZ-TIMME, S. & WAGNER, W. 2016. Epigenetic age predictions based on buccal swabs are more precise in combination with cell type-specific DNA methylation signatures. Aging (Albany NY), 8, 1034-48.

FLORATH, I., BUTTERBACH, K., MULLER, H., BEWERUNGE-HUDLER, M. & BRENNER, H. 2014. Cross-sectional and longitudinal changes in DNA methylation with age: an epigenome-wide analysis revealing over 60 novel age-associated CpG sites. Hum Mol Genet, 23, 1186-201.

FORTIN, J. P., TRICHE, T. J., JR. & HANSEN, K. D. 2017. Preprocessing, normalization and integration of the Illumina HumanMethylationEPIC array with minfi. Bioinformatics, 33, 558-560.

GREEN, H., RHEINWALD, J. G. & SUN, T. T. 1977. Properties of an epithelial cell type in culture: the epidermal keratinocyte and its dependence on products of the fibroblast. Prog Clin Biol Res, 17, 493-500.

HANNUM, G., GUINNEY, J., ZHAO, L., ZHANG, L., HUGHES, G., SADDA, S., KLOTZLE, B., BIBIKOVA, M., FAN, J. B., GAO, Y., DECONDE, R., CHEN, M., RAJAPAKSE, I., FRIEND, S., IDEKER, T. & ZHANG, K. 2013. Genome-wide methylation profiles reveal quantitative views of human aging rates. Mol Cell, 49, 359-67.

HARRISON, D. E., STRONG, R., SHARP, Z. D., NELSON, J. F., ASTLE, C. M., FLURKEY, K., NADON, N. L., WILKINSON, J. E., FRENKEL, K., CARTER, C. S., PAHOR, M., JAVORS, M. A., FERNANDEZ, E. & MILLER, R. A. 2009. Rapamycin fed late in life extends lifespan in genetically heterogeneous mice. Nature, 460, 392-5.

HEILBRONN, L. K. & RAVUSSIN, E. 2003. Calorie restriction and aging: review of the literature and implications for studies in humans. Am J Clin Nutr, 78, 361-9.

HERNANDEZ, D. G., NALLS, M. A., GIBBS, J. R., AREPALLI, S., VAN DER BRUG, M., CHONG, S., MOORE, M., LONGO, D. L., COOKSON, M. R., TRAYNOR, B. J. & SINGLETON, A. B. 2011. Distinct DNA methylation changes highly correlated with chronological age in the human brain. Hum Mol Genet, 20, 1164-72.

HERRANZ, N., GALLAGE, S., MELLONE, M., WUESTEFELD, T., KLOTZ, S., HANLEY, C. J., RAGUZ, S., ACOSTA, J. C., INNES, A. J., BANITO, A., GEORGILIS, A., MONTOYA, A., WOLTER, K., DHARMALINGAM, G., FAULL, P., CARROLL, T., MARTINEZ-BARBERA, J. P., CUTILLAS, P., REISINGER, F., HEIKENWALDER, M., MILLER, R. A., WITHERS, D., ZENDER, L., THOMAS, G. J. & GIL, J. 2015. mTOR regulates MAPKAPK2 translation to control the senescence-associated secretory phenotype. Nat Cell Biol, 17, 1205-17.

HORVATH, S. 2013. DNA methylation age of human tissues and cell types. Genome Biol, 14, R115.

HORVATH, S., ERHART, W., BROSCH, M., AMMERPOHL, O., VON SCHONFELS, W., AHRENS, M., HEITS, N., BELL, J. T., TSAI, P. C., SPECTOR, T. D., DELOUKAS, P., SIEBERT, R., SIPOS, B., BECKER, T., ROCKEN, C.,

SCHAFMAYER, C. & HAMPE, J. 2014. Obesity accelerates epigenetic aging of human liver. Proc Natl Acad Sci USA, 111, 15538-43.

HORVATH, S., GARAGNANI, P., BACALINI, M. G., PIRAZZINI, C., SALVIOLI, S., GENTILINI, D., DI BLASIO, A. M., GIULIANI, C., TUNG, S., VINTERS, H. V. & FRANCESCHI, C. 2015a. Accelerated epigenetic aging in Down syndrome. Aging Cell. HORVATH, S., GURVEN, M., LEVINE, M. E., TRUMBLE, B. C., KAPLAN, H., ALLAYEE, H., RITZ, B. R., CHEN, B., LU, A. T., RICKABAUGH, T. M., JAMIESON, B. D., SUN, D., LI, S., CHEN, W., QUINTANA-MURCI, L., FAGNY, M., KOBOR, M. S., TSAO, P. S., REINER, A. P., EDLEFSEN, K. L., ABSHER, D. & ASSIMES, T. L. 2016a. An epigenetic clock analysis of race/ethnicity, sex, and coronary heart disease. Genome Biol, 17, 171.

HORVATH, S., LANGFELDER, P., KWAK, S., AARONSON, J., ROSINSKI, J., VOGT, T. F., ESZES, M., FAULL, R. L., CURTIS, M. A., WALDVOGEL, H. J., CHOI, O. W., TUNG, S., VINTERS, H. V., COPPOLA, G. & YANG, X. W. 2016b. Huntington's disease accelerates epigenetic aging of human brain and disrupts DNA methylation levels. Aging (Albany NY), 8, 1485-512.

HORVATH, S., MAH, V., LU, A. T., WOO, J. S., CHOI, O. W., JASINSKA, A. J., RIANCHO, J. A., TUNG, S., COLES, N. S., BRAUN, J., VINTERS, H. V. & COLES, L. S. 2015b. The cerebellum ages slowly according to the epigenetic clock. Aging (Albany NY), 7, 294-306.

HORVATH, S., OSHIMA, J., MARTIN, G. M., LU, A. T., QUACH, A., COHEN, H., FELTON, S., MATSUYAMA, M., LOWE, D., KABACIK, S., WILSON, J. G., REINER, A. P., MAIERHOFER, A., FLUNKERT, J., AVIV, A., HOU, L., BACCARELLI, A. A., LI, Y., STEWART, J. D., WHITSEL, E. A., FERRUCCI, L., MATSUYAMA, S. & RAJ, K. 2018. Epigenetic clock for skin and blood cells applied to Hutchinson Gilford Progeria Syndrome and ex vivo studies. Aging (Albany NY).

HORVATH, S. & RAJ, K. 2018. DNA methylation-based biomarkers and the epigenetic clock theory of ageing. Nat Rev Genet.

HORVATH, S. & RITZ, B. R. 2015. Increased epigenetic age and granulocyte counts in the blood of Parkinson's disease patients. Aging (Albany NY), 7, 1130-42.

ILAGAN, E. & MANNING, B. D. 2016. Emerging role of mTOR in the response to cancer therapeutics. Trends Cancer, 2, 241-251.

JOHNSON, S. C., RABINOVITCH, P. S. & KAEBERLEIN, M. 2013. mTOR is a key modulator of ageing and age-related disease. Nature, 493, 338-45.

KABACIK, S., HORVATH, S., COHEN, H. & RAJ, K. 2018. Epigenetic ageing is distinct from senescence-mediated ageing and is not prevented by telomerase expression. Aging (Albany NY), 10, 2800-2815.

KAKUMOTO, K., IKEDA, J., OKADA, M., MORII, E. & ONEYAMA, C. 2015. mLST8 Promotes mTOR-Mediated Tumor Progression. PLoS One, 10, e0119015.

KIM, J. & GUAN, K. L. 2019. mTOR as a central hub of nutrient signalling and cell growth. Nat Cell Biol, 21, 63-71.

KOCH, C. M. & WAGNER, W. 2011. Epigenetic-aging-signature to determine age in different tissues. Aging (Albany NY), 3, 1018-27.

LEONTIEVA, O. V. & BLAGOSKLONNY, M. V. 2016. Gerosuppression by pan-mTOR inhibitors. Aging (Albany NY), 8, 3535-3551.

LEONTIEVA, O. V. & BLAGOSKLONNY, M. V. 2017. While reinforcing cell cycle arrest, rapamycin and Torins suppress senescence in UVA-irradiated fibroblasts. Oncotarget, 8, 109848-109856.

LEONTIEVA, O. V., DEMIDENKO, Z. N. & BLAGOSKLONNY, M. V. 2014. Contact inhibition and high cell density deactivate the mammalian target of rapamycin pathway, thus suppressing the senescence program. Proc Natl Acad Sci U S A, 111, 8832-7.

LEONTIEVA, O. V., DEMIDENKO, Z. N. & BLAGOSKLONNY, M. V. 2015. Dual mTORC1/C2 inhibitors suppress cellular geroconversion (a senescence program). Oncotarget, 6, 23238-48.

LEVINE, M. E., LU, A. T., CHEN, B. H., HERNANDEZ, D. G., SINGLETON, A. B., FERRUCCI, L., BANDINELLI, S., SALFATI, E., MANSON, J. E., QUACH, A., KUSTERS, C. D., KUH, D., WONG, A., TESCHENDORFF, A. E., WIDSCHWENDTER, M., RITZ, B. R., ABSHER, D., ASSIMES, T. L. & HORVATH, S. 2016. Menopause accelerates biological aging. Proc Natl Acad Sci U S A, 113, 9327-32.

LU, A. T., HANNON, E., LEVINE, M. E., HAO, K., CRIMMINS, E. M., LUNNON, K., KOZLENKOV, A., MILL, J., DRACHEVA, S. & HORVATH, S. 2016. Genetic variants near MLST8 and DHX57 affect the epigenetic age of the cerebellum. Nat Commun, 7, 10561.

MUNOZ-ESPIN, D. & SERRANO, M. 2014. Cellular senescence: from physiology to pathology. Nat Rev Mol Cell Biol, 15, 482-96.

POWERS, R. W., 3RD, KAEBERLEIN, M., CALDWELL, S. D., KENNEDY, B. K. & FIELDS, S. 2006. Extension of chronological life span in yeast by decreased TOR pathway signaling. Genes Dev, 20, 174-84.

RAYESS, H., WANG, M. B. & SRIVATSAN, E. S. 2012. Cellular senescence and tumor suppressor gene p16. Int J Cancer, 130, 1715-25.

RHEINWALD, J. G. & GREEN, H. 1975. Serial cultivation of strains of human epidermal keratinocytes: the formation of keratinizing colonies from single cells. Cell, 6, 331-43.

RICE, R. H., QIN, Q., PILATO, A. & RUBIN, A. L. 1992. Keratinocyte differentiation markers: involucrin, transglutaminase, and toxicity. J Natl Cancer Inst Monogr, 87-91.

SHARP, Z. D., CURIEL, T. J. & LIVI, C. B. 2013. Chronic mechanistic target of rapamycin inhibition: preventing cancer to delay aging, or vice versa? Interdiscip Top Gerontol, 38, 1-16.

STANFEL, M. N., SHAMIEH, L. S., KAEBERLEIN, M. & KENNEDY, B. K. 2009. The TOR pathway comes of age. Biochim Biophys Acta, 1790, 1067-74.

TRICHE, T. J., JR., WEISENBERGER, D. J., VAN DEN BERG, D., LAIRD, P. W. & SIEGMUND, K. D. 2013. Low-level processing of Illumina Infinium DNA Methylation BeadArrays. Nucleic Acids Res, 41, e90.

WANG, R., SUNCHU, B. & PEREZ, V. I. 2017. Rapamycin and the inhibition of the secretory phenotype. Exp Gerontol, 94, 89-92.

WEICHHART, T. 2018. mTOR as Regulator of Lifespan, Aging, and Cellular Senescence: A Mini-Review. Gerontology, 64, 127-134.

WEIDNER, C. I., LIN, Q., KOCH, C. M., EISELE, L., BEIER, F., ZIEGLER, P., BAUERSCHLAG, D. O., JOCKEL, K. H., ERBEL, R., MUHLEISEN, T. W., ZENKE, M., BRUMMENDORF, T. H. & WAGNER, W. 2014. Aging of blood can be tracked by DNA methylation changes at just three CpG sites. Genome Biol, 15, R24.

YANG, Z., WONG, A., KUH, D., PAUL, D. S., RAKYAN, V. K., LESLIE, R. D., ZHENG, S. C., WIDSCHWENDTER, M., BECK, S. & TESCHENDORFF, A. E. 2016. Correlation of an epigenetic mitotic clock with cancer risk. Genome Biol, 17, 205.

ZHANG, Y., BOKOV, A., GELFOND, J., SOTO, V., IKENO, Y., HUBBARD, G., DIAZ, V., SLOANE, L., MASLIN, K., TREASTER, S., RENDON, S., VAN REMMEN, H., WARD, W., JAVORS, M., RICHARDSON, A., AUSTAD, S. N. & FISCHER, K. 2014. Rapamycin extends life and health in C57BL/6 mice. J Gerontol A Biol Sci Med Sci, 69, 119-30.

**391 CG SEQUENCES**

EP 3 802 856 B1

| SEQ ID NO | Probe ID | Sequence With CpG Marked By Brackets |
|---|---|---|

1    cg12140144

CTGCGTTTCACCTCCTTTAACGCGGAGGCGCGGAGTTGCACGTGTGGGTCTCAGTGGAGC[CG]CCACAGGTCTTATTACACAACAAAGGGCAGGGAGGGCAAGGCCAGGAGCCTCGCGGGGCG

2    cg26933021

TTCGGCAATAACAAGGAACGAAGTCCTGATTCACGCTCCACCGTGGATGAACCTCAAAAA[CG]TGATGCTCGTGAAGGAAGCCGCTCATGAACGGCCACATGCTGTAGGATTCCGTGTATATG

3    cg20822990

AATGCCTGCTTCACAGAGAACTGCTGCGAGGATCACACAAGAAAATGCTTGTCAACTGGG[CG]TGGTGGCGCATGCCTGTAATCCCAGCTACTCGGAGACTAAGCCAGGAGAATCGCTTGAAC

4    cg07312601

TCCTGCTATGACAACCAAAAACGTCTTTAAATGTTGCCAAATGTACCCGGTGAGCAAAAA[CG]TGCCTAGTAGAGAACCACTGCTCTAATGTGACCAAGCTGTCCTCACTCCTGATTTGTAGG

5    cg09993145

TTTGTGAGGCTGGCCTCAGCACGCGGCCCAAGAAACAGAACTGAAAGCGGTTGCAGTGGG[CG]TGGCCAGGAGGGTGGTTTGGCTCCTGGGTGGGAGACTCCTTCTTAATTAAGGCCAGCATT

6    cg23605843

GAGCAGCAAAGGGCCACTCTTGTCCTTTTTACCCCACGAAGTCCCACCTCCCATTCCTTA[CG]CTCAAGTTTTCATTTCTTGGAGAGCACCCCGTACGAGAGAAAGGGAAATAACTTCTGCAG

7    cg25410668

| SEQ ID NO | Probe ID | Sequence With CpG Marked By Brackets |
|---|---|---|
| | | CTAGCCTCACAGCACCGCGTGGAGTTGCTTGTTCTTTTACATAGGAGGTCACATTCTCTT[CG]TGTAATGCCACCAATGGTGCCGATTCTCCCCAGTGGGGCTGTGAGAAACCTACGCCCTCT |
| 8 | cg17879376 | TTGGCCCCTGCCTCTCTTGGGACCACAGAGGAGGTGGCAGGGCCAGGCGTGCCAGCTCCT[CG]ATCCCCTCCCCCAGCCCTGGAGCCTTGTGACAAGCTACCCTCTCCACGCCCACCCCCAGG |
| 9 | cg14962509 | ACGGGCCGGCGCCCCGCTCTGCCACACGCCGGCCGCCACAGCTGCCGCCGAATTCCAGC[CG]CCCTACTTCCCGCCGCCCTACCCGCAGCCACCGCTGCCCTACGGTCAGGCGCCCGACGCC |
| 10 | cg24375409 | CTGAAGAACCAGAAGCCCTGCGGGACAGGTGCGGGGCAGGCTCCAGGTCCCTCCCAGACA[CG]CACTCACCTTCTCGTAGTATCGGATCTCGTACTCCGTGTCATTGGCCCCAGGGGCTCCGG |
| 11 | cg22851420 | CGCCCGCGCGGCCCCGCACCTCGATGATCTCCAGCATCTCCAGGCGCGTGATGCGCCCGT[CG]CCGTCCAGGTCGTACATCTCAAAGGCCCAGTTGAGCTTCTGCTCGAAGCTGCCGCGGGAG |
| 12 | cg24107728 | AGAGGTGTGTGAAGTCACAGCCCTGGCCCCAGCTGCCGTGTGTGTAACACTGGGCCCATC[CG]TCAAACCTTCTGAGCTTGCCTCCTCCTCATCAGGAAATGGAGGTGTGGCGCTGACTAAGT |
| 13 | cg14614643 | CCATCATGACTGTGTTCCTGGTAAATTACCTATGTCTTATAAATCAAATGTTTATAATAC[CG]GCTTCCAGTAAAATTGGAGAATTTCATTTTCATTTATGGTCCTCTTCAGTTAGTAATAGT |

| SEQ ID NO | Probe ID | Sequence With CpG Marked By Brackets |
|---|---|---|

14   cg00257455

AACCTTCTCTATCTAGAGCAGATTCTGCAGAGAGGCCCCTCTTTAATTCATATTCTGA

AA[CG]TGCTTGTTATTGTTGACGTACAAAAACTTATGAATAATTCAATAAATGAATCTTGAAC

CA

15   cg23045908

TCCTGGGGTAAAAGTACCACCTTTGGATCAAGGCTTGCTGGCTGCGGCTCAGAGGATC

TG[CG]CAGAGGAAGCAGTGTGTCCTCAGGAGATCCTGAAGGAGGGGAGGGGGGACTCTTCCT

ACT

16   cg15201877

GGACAGGTACACGACGATGACGACCGGGGTGGTGAGAAGCTGCCCGACCAGGTCGG

TGAG[CG]CCAGCCAGCCGATGCACAGCAGGAAGGACTTCTTGCGCTTGCTCTCCCGGCGCCGG

TAGC

17   cg18933331

ACCTCCTGCTTGGGTTCAGCCACCTTCAAATACTGCATCAATGGCTCGTGCCTCTGCCT

G[CG]GGGCTGGGCCAGCGCGGGAGAGGCAGGCGGAGGGTTCAGGGAGCTGGGGATCTGCGG

TAT

18   cg05675373

AAGGAGGAGATGGCCAAGGGCGAGGCGTCGGAGAAGATCATCATCAACGTGGGCGG

CACG[CG]ACATGAGACCTACCGCAGCACCCTGCGCACCCTACCGGGAACCCGCCTCGCCTGGC

TGGC

19   cg19269039

CAGTAAGCCTGAGAAAGGGGCTGCTTCGGTCTCCAGCCACAACTCTGTGAAGCCAAG

CCA[CG]CGCTGTCTTCCAGAGAGGGAATAGAAGTTTCCATCCTGTGCACCCAGTGGTTGAGCA

AGA

20   cg16008966

EP 3 802 856 B1

| SEQ ID NO | Probe ID | Sequence With CpG Marked By Brackets |
|---|---|---|
| | | GAATGAATGCGGTGGTAGTGATGGTGGTGATGGTGGTGCCTGTGTATATGTGTGCATG TG[CG]TGTGTGAAAAGAGGCAGAGCAAGAATGAAAGCATCTCTAACAAAATAAGCTGCTTGA AAA |
| 21 | cg14565725 | CCAAGTTACGCCACCGGTCGAGGACGGCAGGAGACCCCCGAGTGCAGAGAAAGCTC AAAC[CG]GCAGCGAAGTCGGTCCTAGCCAAGCTGAAAAAACGTCTCGGATTTCGCGGACAGC GGCCT |
| 22 | cg05940231 | CTAGTGCCCTGGTCGAGACGGTTCTATCCTTTTGCAAAGAAGCCGGAAAGAGCTGGG TCC[CG]GGGGCGGGGGACAGACTGAGAGGCCAAGCAGGCCAGTCGTGACGACAGCCAGGCC CTTAG |
| 23 | cg03984502 | TGTGCCTATGCCTCTGAGGCTCTGATTATCCCCCGTGTTCTTTTGGTAAATGTGGGCCC A[CG]CATCCATGTATGCATCTCTGCACAGAACAGGATGTTGGTCTGTTTGGGGGCTCTGCACC T |
| 24 | cg25256723 | CAAACTAGTGACTGTTTTACTGCAGGTGAAGAAGGGGCAGAGATCAGAGGCTCTAGC AGG[CG]GGACAATGCCCAGGGATTCATGAGCCGGACAAAGCTGTATCCCTCCATTTCCACCTG CCA |
| 25 | cg16054275 | TTCATGAGCCGGACAAAGCTGTATCCCTCCATTTCCACCTGCCAACACCACGGAAGCA GT[CG]TCCGTTACCACTGACCTGAGGCCTGCCTGGGTCCAAGCTCACACTTGGAGAACCTTCT GT ] |
| 26 | cg01459453 | |

| SEQ ID NO | Probe ID | Sequence With CpG Marked By Brackets |
|---|---|---|

GCAAGTTTAAAAGTACTCACAAAATCTAATAGGCAATTCAACATAAAACTCCATGGC TAT[CG]CTGTTCCTCACTTTCTGAACCTTTACCTGCCTGACTTTACTCCATACCACTCCAACTCA C

27 cg16599143

CTGCAGAAAGCTGTGGCAAGCAAAGGATAGGCTAGAGAGAGACAGGACTAATAAAT GTGT[CG]ACTTCAGATACATTCTTGTGAAGGACAGCAAAGAATAGGTGGCCTTTTCACCTCCT AGAG

28 cg02275294

GTTTGAATGTTGCTGAAGGACGCTGGTTTTCAAACGGTAAGGAATCTCCTGATAAAGG CA[CG]AATCTTGGTGTGCAGATAAGCCAGCGATTCTTGCTTCTGGCTAGTTCTACGTTGTTCCT G

29 cg21870884

GGGCCCGCGGCGGCTGGTGGATACCTTCGTGCTGCACCTGGCGGCAGCTGACCTGGG CTT[CG]TGCTCACGCTGCCGCTGTGGGCCGCGGCGGCGGCGCTAGGCGGCCGCTGGCCGTTCG GCG

30 cg10501210

ACGTGGGGGAAGAAGGGGGTTACGCCATCAAGTCCTGAAGCCCGTCGGACCACCCAT CGC[CG]CCTGCGCAGACCCAAATCTTGGTCCCGCCGTAAGGTGCCGCAGTCCCGAATGTTCCA GAA

31 cg02901139

CCTTGGGAACCAGAAACTTAAACACAACCAGGAAGAAAAAAAATCAGCCAAAAATA AAAG[CG]AATTAAGACAGTTGGGGTCTTATTTTAGAAATATACCTTTCTAGGTTCTGGTATGTT GGG

32 cg11298786

EP 3 802 856 B1

| SEQ ID NO | Probe ID | Sequence With CpG Marked By Brackets |
|---|---|---|
| | | CTCAGGCCTCCCACCTCCACTGCACATATCCTGTGGGAGGGGGAACGGTGGCCACACTTT[CG]CCAGGGCTTGTGATCCCTCAGAGCCCTCACCAAGCAAGGATCACCCCAGTTCCGAATTAA |
| 33 | cg09809672 | CCCCAGAGAGCTTTCATCTAGAAGGTTTGACTCTGGCCAGACAACCAGCGAGCATCTTCT[CG]CAATCTGTTGCTTCTTCCATGGCAAACTCCAGAGAATTAAGAAGCCAAACTCAACATCGC |
| 34 | cg05940691 | GGAAGACTCACCCATCTGAGTAGGGAATAAATATAGGATAAATTGTTGGCAGAAAGCTTT[CG]ATCGGATGAATTTTCTCTGAGCGAAAAGCCAAGCTTTCTCTAAGTCATTTTTACCCACAT |
| 35 | cg12869659 | ATTTGACATTCAGAGATGTCGCAGCACCCTGCCCGGTACCGGTCAGCCCAGCCCGGGTCG[CG]TTACAGTTTCATCAGCTATTAGAAAAGACCCACAAACTGGCTGAAAAGTTCTCAAACTTA |
| 36 | cg23028740 | TATGGGAAATAAGCAGGGAGATGAGGATAGCTAAAGGAAGTTACTTTAAATAGGGCAGTC[CG]GGAAAATGTCTATGCAATTTTAGCTAAGCCCTGAAAAATAAGAATGAGTCATATAAAGGG |
| 37 | cg10959651 | CTGGGAAGCTCTTGAGTGTGTTCAGGCAACCTCTGAGCTCTCTGTGGAGGAGCCTGGTCC[CG]CTGTTCTGCTGGCTGAGGGCAACCTTCTGGCTGCTAGCTACCAAGAGGAGAAAGCAGCAG |
| 38 | cg00522231 | GAATTGGGGTTTTCAGCTACTCAGGAACCGCAGATAATCCCTGACAGCTTCCCTGGCCGA[CG]ATCCGGTCTCGGCTCCCAGACCGGAATACCACCTACTTCGTCTTCCCTAACGTAAGACGC |

EP 3 802 856 B1

| SEQ ID NO | Probe ID | Sequence With CpG Marked By Brackets |
|---|---|---|
| 39 | cg01752203 | GGAGAGTGTTTCCGACCGCAAGTATGAACCCTCCTCACTAACAGCTATGCTGAAGCACAG[CG]GGGAGCCAGTGCAGCACAAGCTCAGCACGACCGCGGTTGTAAGAACCCACGTGGAAGCCA |
| 40 | cg23643435 | GGAGGAGTCTGAGCCGAGTCACGCCCCTTCTCCTGTAAACTTGGGTCGCCTCTAGCTTAG[CG]AGCGCTGGAGTTTGAAGAGCGGGCAGTGGCTGCACACGCCAAACTTTCCCTATGGCTTCG |
| 41 | cg01243072 | TTCCTGCCGTGCCCTGCCCGTGCCAGCTCCTCGGTGCTCATCCCGGCTCCCTGAAATGCT[CG]CTTCCACTCAGGGCCAGCGCACTCCCTCCACGTCCCTGGCCGCAGATCTGTCCTGCTTTG |
| 42 | cg07589899 | GGAGAAGAGAAGACGTGCAGCCAGACACCTGCCGCCTTGTCAGGCCTGTGTCGCCGCCTC[CG]CAGCCCGAAATCATCCTGCCCTCCAAGGCACCGCCCTGATGCTCCAGGTGAAGGCTGAAG |
| 43 | cg10855531 | CACCACAGGCACTCCACTGTGTCTGTCCTGTCTTGGGGCACAGCGGCAGAGGCATGCCCA[CG]CAGCCGGGCTCAGCGCCTCTTCGAGAGGGACCGCTGAGTGCGGCTGTCCTCTCCAGTGGG |
| 44 | cg22943590 | TAAGACCACTTGCTGCTCCCTGGAATGATTCTAATATAGGAGGTACATTAGAGAGAGTGC[CG]TAAGAATAGCCTATATTAAAAAGAACTAGGTATGTAGCTTTTAAAGTGTGCCCATTTAGA |
| 45 | cg23398076 | |

| SEQ ID NO | Probe ID | Sequence With CpG Marked By Brackets |
|---|---|---|
| | | ATGGAGTAGGCATCCCCTCCACGATGTATGGGGACCCGCATGCAGCCAGGTCCATGCAGC[CG]GTCCACCACCTGAACCACGGGCCTCCTCTGCACTCGCATCAGTACCCGCACACAGCTCAT |
| 46 | cg12082609 | CACTGTGATCTGGAGAGTTGGAAACTTTCGGCAGTGTAGTCCATTGCACAGAACACGCAG[CG]TGCGCAATCCCAGTGCGGCCCCACAGAAGTGGGAAAACTGCAGGCGGCTCCCAGCCTTGG |
| 47 | cg01447660 | TCATCACCTTGTGGCCAGACAGGATATTGCTGTTAGAGACTCCAAGAGCCTGTTTGGGTT[CG]GAGCTATTCTGGTCAATTTTATCACCCCATGCACTGCCTCCACTTACTCATGGGCCAGGG |
| 48 | cg02085953 | AGTTTTGCCTCCAGGGAAACTGAGGCACAAGGCAGCAATGATTACTGAGGGTCCTGCCTC[CG]CTCCTCTAGGTGAGGAGCCTATTCCAGGGGCTCCAGTCTGAAAGCCTAGAGGCGAGGGGC |
| 49 | cgl5149655 | GGGTCACAGAGACCTAGAACAGCTGGAATCCTTCGCCCCCGGCGCGCAGCCTTCGCCCGC[CG]GAATCGCTGCCTTATCCACCAGCGGGATGCTTACCTCGCCCGCCCTCTCGGGTCAGGCGG |
| 50 | cg22809047 | TCACATCTGTCATCTCTCAGGTCATATCCAACACACTGGGCCACCCACGCACAGGGACGA[CG]CGACAGCCCTGTGGCTCCACCGCACAGGACAGCCACGACTGGCAATCCTGTGCCGGCCCT |
| 51 | cg06639320 | |

| SEQ ID NO | Probe ID | Sequence With CpG Marked By Brackets |
|---|---|---|

CCTTTGTTTGCCAGGGCTCCTTTCTTCGTGCCCTCCGGGTCTTGGGAGCACAGTAGTTAT[CG]GGAGCGTCGCCTCCGGCGTGGGCTCTCGGGCGCGAGTTTCGGACGAGGCCTGGGCGCGGT

| 52 | cg22454769 | |

TGCCCTCCGGGTCTTGGGAGCACAGTAGTTATCGGGAGCGTCGCCTCCGGCGTGGGCTCT[CG]GGCGCGAGTTTCGGACGAGGCCTGGGCGCGGTGGCAGGGGTCTGCCCACGCCGGGATCTC

| 53 | cg00017842 | |

ACTTTGCTACAAAACCCCAGGTTGTATCATGCCCTCTAACAATTCTGGACGTGGCCAGGA[CG]TGGTGCCAAGTCTCAGGGGCAAGACAGAAGAGGCAGAAGCACATTTAGTTCTTGTGTTTA

| 54 | cg23606718 | |

CTGACCGTGGTGCTGAGCGCGGCTCGCGCTCCGACGCGGTGCCCGAGCCTGTCGCGGCCG[CG]CCCTGCTGCACTGCGGGCCCCAGCGGTAAGTCGCCAAGGCCCCGAGAGGCTGCGTGGT

| 55 | cg22061831 | |

ATGCCCCAGCGAGTCAAGCGGGCAGACGAGTGGCGATCTCGGCACTAGCAGCAGCAGCAG[CG]CCGGGCTGTCCCCGGGCTCCGACTCGGACAGCAGCGGCGTGGTGTGTGGCGGCCGCGGAG

| 56 | cg12757011 | |

TCTTTCTTGTAATGAAACTCTTCACCTTTAGGAGACCTGGGCAGTCCTGTCAGGCAGCAG[CG]ATTCCGACCCGCCAAGTCTCGGCCTCCACATTAACCATAGGATGTTGACTCTAGAACCTG

| 57 | cg01620164 | |

EP 3 802 856 B1

| SEQ ID NO | Probe ID | Sequence With CpG Marked By Brackets |
|---|---|---|

GTGTGGTGTGTATTTAGCTCAATAGTCACCAGAGTCCAACCAGACGTGTATGTCGCGC
AA[CG]GGTCTTGTAGTTCCTCTCTTCGTATTCACATTTGTGTTAGGAGAGAGCAGTGACCACG
GC

58 cg12105450
TTAACAACCCTTCTTACTCCAGAGTCTCATGCTTGAATCTTTTCCTTTGCTTCATGGCT
T[CG]TGTTGTAGAAACTTGCAAAAACTTGTCAGCAATGGCACTATTTTTTCTTAGATTTCTTTG

59 cg00760938
CTGCCTTGGAGGGCCTCACCTTTCCTGCTGGGTCAGCTCTTGCCTGTGGCTCTGGCCTC
A[CG]GGACTCTCAACAATGCTGTGCAGGCCCCACTCTTGTCTGTGGCCCCAGGGGCTTTGTG
G

60 cg10376763
TCAGGTCTCCTTGGCAGTTCCCCTTCTGCTGTTCTTGTTGCTGCTTGGTGCTGTGTGAA
G[CG]CACCAGGGCAGAGCCCGCTGGGGGCTCACAAGTGGGAGCGGTAATTGCGATTGGCTGT
GG

61 cg23077820
CGGCCACACTCCTATTCACGTGATCGATTTCTGCATATTCCACTCGCCTGAACCGCCG
CG[CG]CTGACTGGTTCCGCCTCACCGCCCGGGTGGGTTTTATTGCTCAGCCCTGGGGACTTTTA
A

62 cg08166272
CGTGGTGGCCCTTAGCACGGTTGTGCAGCTGTAGGAGAGCCTGGTACCAGCTGTCTTC
CT[CG]CCTCGCTGTCCGCCGCGATGGCAAAGTGCTCGTCCCGGGTGTAGAGAGCCACCAGGT
GC

63 cg10523019
CTCGCTGCTTCTCCCCTAGTCTTCGGGTCCCTTGAACGCAGGTCGCTTGTTTGCCTTAC
G[CG]TAGTCAGCGGCCAGTGGCTATTTATGGCAGTAAGGAATATTATCCACATTTCACATGGA
G

64 cg23462687

EP 3 802 856 B1

| SEQ ID NO | Probe ID | Sequence With CpG Marked By Brackets |
|---|---|---|
| | | AGTTCCCCGCTCTGTGGCTTTGGCCGGCCCTGCCATGCTGACCACGGGTGACGCTCCAGT[CG]GCCCTGACACATAGTTTGTTGGCCGACATCGTGTTGTGTATTTCTCAATACAAAATAAAA |
| 65 | cg20669012 | GGTGGGAGAGCTCCTTCTGATGGGCGTCATTTCAGTTTCACAGATGAGGCATGGGAGGCT[CG]AGTGCTCCCCAAGGGTCACACATCTAGGAAGTGGTCCAGGCAGGAACTGAAGCCAGGTCT |
| 66 | cg03183882 | ATCAATCAGAGAAGGAAAACGGCTCAGGCCGGGCACCTTGGCAAGTGAGGACTCTGCACC[CG]GGGCACCGGTGCCAGCCCGCGCTGCAGGGCAACGCCCACCCGCCCACGGTGCCCGGCGCC |
| 67 | cg15910502 | AGCAGTTGTGGAAGCTTGGAGGTGGGCCAACTGAGCCAGACCTTTGTTGCCTAGGGCCAC[CG]GCTGGGGTGCGTGGCCAAGAGGGCACTGAGGAGTGCAGGAATCTTAACCTGGAGAGTGAC |
| 68 | cg12941369 | TCACATGTTTCGTTTCTAGTCCTGAAACATGGTTAAGTGCTTGCCTCCTAGGGCCTCTGC[CG]CAGGCTTTTGGTTTGGAGGCTCTCCTTTGCCACTCCACCCCTCTCCACTCTTCTCCTCTT |
| 69 | cg02244028 | AGAAGAAGCCCAGTCTCTAAAACTGAGACCCAGACATTAAGCAAGACAATAAGGCTGAGC[CG]GCTGAACTGCTGAAGTGGGATCTGCAAGTAGCAGGCAAGTGGCCACATGGCCCAAACAAG |
| 70 | cg24888989 | |

| SEQ ID NO | Probe ID | Sequence With CpG Marked By Brackets |
|---|---|---|

CGTCCGATCCAAGCGCCAAATTCAAATTTGCGGCCATCTTGAGCGGGCGGAATTCAGT

CG[CG]CGCGGTGCAGTCGGGAGGTGGAGGCACCGGCTGCATTGTTTTCGGGATCGAGGGGTG

AGG

71   cg00702638

    TCCGATCCAAGCGCCAAATTCAAATTTGCGGCCATCTTGAGCGGGCGGAATTCAGTCG

CG[CG]CGGTGCAGTCGGGAGGTGGAGGCACCGGCTGCATTGTTTTCGGGATCGAGGGGTGAG

GGC

72   cg07303143

    CGGCTTCGTTACCCTATTTTTGCCCCCAAATACAGCTGTGAAAGGATGGCAGCCTCGG

AC[CG]CCCGCAAGGTTCTTGCTAGGCATGAACTGCAGGAGCTGAGTGACCGGCGGGGACGTT

TGG

73   cg26614073

    CTTGGGCAACGTAGGAGACCTCCGTCTCCACAAGTAAAATTAATTAGCCGGCTGTGGT

GG[CG]CGCACCTGTGGTCCCAGCTACTCAGGAGGCTGAGGTAGGAGGATCACCTGAGCCCGG

GAG

74   cg15988232

    CCTTCTAGTCTCCGGGCAGCCTGGGGAGCGGCCTTTAATCCTGGTCCCTTCTCCGGGA

TA[CG]TCGTCCCCCAGGTGTCTCAGACCACCAAAACTCAGGTTCCTGGGTAGACCAGGGGGGT

CT

75   cg16933388

    GGTCAGTCGGGGCCTGCAGACCGTGACTCCGTCACGAACCCCAAATTCGCTTCTCCCC

AA[CG]CTCGGGCCTGACTGCTCAGGAGGGGCTTATGTAACCTTAACCTGGTCCCTCCGCACAG

GA

76   cg19381811

    TCCTTTTGCCTTCTTAGGAACCTGGGGCCAGTTCTCTGGGAGATGGACCACTGTTTGTC

A[CG]AAACTACGTAATAGCCAAACCAAGTGCTGTCTTAAGTTCTTTTTTGTTGTTGTTGTTAAT

| SEQ ID NO | Probe ID | Sequence With CpG Marked By Brackets |
|---|---|---|
| 77 | cg03019000 | TGAGCATAGTTGTCACCTTCCCCACCTCCCACCAAAAGTCCGGGATTTTCACGAGGGGAG[CG]TTTTATCTTTGGGCCCCTAGAAGAGTGCTTTGTAGTTTGTAGGTCCTCAGAAATTTGAGG |
| 78 | cg01844642 | CGCAGGCTCGTTGGGGTTGATCCTGGCAGCTGTCGTGGAGGTGGGGGCACTGCTGGGCAA[CG]GCGCGCTGCTGGTCGTGGTGCTGCGCACGCCGGGACTGCGCGACGCGCTCTACCTGCCGC |
| 79 | cg04474832 | CCAGCCAAGTGGCCTTGATCGTTTTCCCAATGCCCCCGAGCCTGTTTCCTGCCAGTAGAG[CG]GGTCAGATGTTGCCAACCTCTGCAGAGTAGCAATAAGCAGTAAACGCCACGCTCTGCACA |
| 80 | cg03891319 | ACCATCTCACACTGTCACATACACAATCATATCCACTGATAGACTGCACACGCAGTGGCA[CG]CTTAAACCGTCACACGTGCTCTTGTCCATGCATTCATTCCCATTCTAGGCACTGTCCGGG |
| 81 | cg03607117 | CGCTGTGGCCCCGAGCGGAACGGCCCGGAAGAGGAGACGCGTCCCCGGGAACCCAGTGCC[CG]CCCTGGCCCAGCCCCGATCCAGCCTGCGCCTCACCTCGGGTTGTAGACAGAGCGGCGGGG |
| 82 | cg22264409 | TGTTATCCAAACAAACCAGTTTTGGTTAATTGGACTACAAAGTGTTCAAATTAAACCCAA[CG]ACTGCTTTCGCGGAGGCAGAAGCGTGTAATGATTAAGACCACATAAACAACAGAGTGTCA |
| 83 | cg11205552 | |

EP 3 802 856 B1

(continued)

| SEQ ID NO | Probe ID | Sequence With CpG Marked By Brackets |
|---|---|---|

ATCCTGGAAGCCTGACAATGAGCCCAGACCATTCCTGTGCCTTGAATGGTAGGTTTTG TT[CG]ACTTTGGAATATTCTGCTCAGAGAGAAGAGCTTTTCCTTACAGCTGTTTTCTTCCTTCA G

| 84 | cgl7321954 | |

CTTTACCTTCGGCCTATCCACAGATTTCTTCTGCCCTGGAGACCACAGAACTTACCCTA T[CG]AATCTAGGATTGGCGCCGAAGCTACTCCCGCCCTTTGACGTCCCCGGGCACCCCGCCCC C

| 85 | cg00796779 | |

ACGCAGCCCCCGTGGTGCTAGGGTCAGGAGACACTTCTTTGGGTGGCGTGGGTGGGA AGC[CG]AAAAGGTGGGAGCCAGAGTGGGCTGCTGTAGGGGTGAGGGAGGCCACTGAGCTCCC GCTG

| 86 | cg18303397 | |

GAGCAGGTTACTTGTCTTTGGTTCTGTCCCTTCTGAGATCTTTCTCTGTGTAAAGCATG C[CG]TCTCTCCTCATCTCACACGGAAATCCTGAACATCCTTCAAGGCTCACGTTGGAGACGGG T

| 87 | cg09025210 | |

GAAGACCCAGCCGGCCGAGAGCCTCAGCCACCTTCCTGCAGGAGGTCCTCACACCCC AGA[CG]GTCAGAATGCTCCCCAGACTGAGGAATCAGCTGCACATCCCCCTGATGTCTCTAAAG CTG

| 88 | cg14423778 | |

GTCAGTGTTCTTTTAGTTTGCTTAAACTGTGTGGGTACTTGAGTCCTTTTAAACGATTA A[CG]CTGGGAAGAGGCACCATTTAATTAATTAATTTGTTCTGGAAGGGATCAGTGTACAATTT T

| 89 | cg15277914 | |

| SEQ ID NO | Probe ID | Sequence With CpG Marked By Brackets |
|---|---|---|

TTGCACTTAGGTCCTAGGGTAGTAAACGTTGATTGAAACAAAAGAACCCTTGGATCA ATT[CG]CCGTCTTCTAAAGAAAAGTCTCTAAAAAATGAGTTCTTCTAGTCTTGAAAACAGCCT GAC

| 90 | cg07553761 | |

AATCCGCATGGCACCGGTGGTCTGGGGGAGAGGCTGGGCCTGGCGCGGGACGAGGC GAAG[CG]CCGGTGGCCGACGGCTTCTGAGGAATTATCTTTTACTTGGCGCCACACGGGGCGGG GCCT

| 91 | cg06737494 | |

GCCAGTTGCCAGCGAATTCACAAATCCGACCGGCCCCTCCCGGCCCACCGACCTCGG GAC[CG]CCCCAGGAACATATTCAGCACTGTGGCCAGCGCCACATCCATCCTACCGCAAAGCGC CGC

| 92 | cg01059398 | |

TCATGAATTTTGGTAGTTTCTCCTATAGAACTTGGCCAATGCTGGTGACTAGACACAT GG[CG]GGTTGACGTGAGGTGCTGTGGTTATTCCAAGAATGATAATTAATACGATACGTCTCCC CC

| 93 | cg26824216 | |

AGTCTAAAATGAAGGTTGAAAAAAACAGCTCATGTCCATACACAGAAACAGAAACTG AAC[CG]AACACCGAAACTGAAACTGTTTGTCTCTTCCTGAGAAACGAGCAAACCTGAAAGCT ACTC

| 94 | cg25478614 | |

TCGGGAGCTGAGGGACCCAGAAAAGCACCAAAACTCTTTAGAAGGACTGAGCATCCC TTA[CG]TCCAAACCAATGGGGCAGGAGCAAGGCTTAGGGAGGGCTGGAGAATCCGGGAGACC TCGA

| 95 | cg07110949 | |

TCCAGGTTCTTCTCATTTCCCTGTGGTGTCTGCACACATCCTGCTTAGGATTTTCCCGC C[CG]ATACCTGTACCCCGGGTTTTGCGCTGACACATGCTCCATTGCTTCCTCGTGAGAGCTTTG

| 96 | cg23239150 | |

EP 3 802 856 B1

48

| SEQ ID NO | Probe ID | Sequence With CpG Marked By Brackets |
|---|---|---|
| | | GAACTGCTGGCACTTTGCATTTCCTCCACAGCCCTGTGGGGGCCACAGGGCCAGATTG GC[CG]GGGGAGATGACTATAAGCCAGGTGGCTTTTCCTCCTTGACCGTTTGTAAATCTGGATT CC |
| 97 | cg21254939 | CAGGAGACTGGCGTCCTGGCCACCCCACAGGCTGAAGGAAGCCTTTTTCCTCTGGAAT GC[CG]ATGGCTGGTGTACACGCCGTTGGCTCATGGGGAGAGGCGACGGCCGTCTGTCTGCGG ATT |
| 98 | cg23995914 | AGCCTCAGACCCAGCCGAGCCCCACTTCTGGGCTTAGAGCTTGACCCAACACGTTCGC AC[CG]TAGCGAGCGAGGTCCACATTTAGCCATGCCGCAGGCAAAAGAAGGATTCGGCTTCGG TCC |
| 99 | cg23836737 | CGAGCAAGCCCACTAAAGGAGTTGTTGGGGTCCCCCACACTAACACTTTGCATCTGCT GC[CG]GAGCCGTTATTGCCCTCACTGTCTCAGATTTGGCCAGCACTTAGTGGCTGCACAGGGA CA |
| 100 | cg05960024 | CAAGGAAAGTAGCAGATCATTACCCAAGTATTTTTATAATTCCTTGTCCTATGCTTCC AC[CG]GTACACTGCAAATTCCACCCAACCATGATTAAGGGAAAAGAAACAAAGATAGCATAC CTT |
| 101 | cg10699857 | CTTCTAGTGCCCGGGCCAAGAGGGCGACCCCGGAGGTGCGTAGGTGGCCCTCCGGGT TCC[CG]CTTCTCCTAGTGCCTCTGAAAATACCGTCAGGGTAAAGGGAGACAGGCAGTAAGTCT TAC |
| 102 | cg05024939 | |

49

EP 3 802 856 B1

| SEQ ID NO | Probe ID | Sequence With CpG Marked By Brackets |
|---|---|---|
| | | CAGGTCACTGAACTGCGCTTCACTGCGCCAGCCCCTCCCCTTCAGTATTTTCATCGCG C[CG]AGGAATCGGCATCCACCACAGCAGACAGAAGGCAGGGAAGATCATCCCCCAGGCCCCA AG |
| 103 | cg05106770 | GAATGAGAACCCTAACTTTCTGTAAGCTGCTAGTGCATTAATTTTCACTGCTGGTACT TT[CG]TCCAACCTTATCCTTTATGCAAAATAGACTAACAAATATTAAATCCTGTGGTTACAGTG A |
| 104 | cg06690548 | GAAGCAATTTGAGGGTGTTCCAGATCACACCAACAGCGGATGCTGCATCTGGGTAGT TCA[CG]TACCCGAACAAAAATTTTAAAAATTTGGTGTGGCCTTTGCCATCCATTCACTCCTCAA AA |
| 105 | cg02650266 | GCCCGAGAGGATCCAGGGAAAGCAGAAGGGGGTTAAGGACCATGGACAGAGCCCGT CGCG[CG]CTCGTTGCTGCCGCCTTCCCCAGCACTCTGGCGGCTCCTGAGGACAGCGGTCCCAT CTTG |
| 106 | cg01511232 | GACCGCTCAGCACAGTCTGTCTGAGTGTTGACCAGGAAAGTCCAGGCTCTTTCTAAAT CT[CG]CCGCCAGACCTGGTGACGCATTCGCATGTATTTAAGGCGTTTGCACGCAGAACGTTAT CA |
| 107 | cg25148589 | GGGTGAGTGTGTGTGAGTGCATGGGAGGGTGCTGAATATTCCGAGACACTGGGACCA CAG[CG]GCAGCTCCGCTGAAAACTGCATTCAGCCAGTCCTCCGGACTTCTGGAGCGGGGACA GGGC |
| 108 | cg24843443 | |

EP 3 802 856 B1

| SEQ ID NO | Probe ID | Sequence With CpG Marked By Brackets |
|---|---|---|
| | | TCATTAATGTTTGAAATTCGAGTTTCAACCCCAGCCCATCATGGTCTTTAGTGCTCCAGA[CG]CTTAATTCCATGACGTTATGCATGTGCAGAATATATTGAGATTCAAGGTGGTGGTGAGGG |
| 109 | cg03364683 | GATTCAAGGTGGTGGTGAGGGTGCCCCAGTAACGGCATGGGGTAATAAATGGAGAGAAAT[CG]AAACCGGAAGTTCTGTCTTCAAGAAAAGGAAAGGGTGGAAGTGACTTGTTCACAATAGAA |
| 110 | cg21815258 | CAGAGAAAGAGGTTGGAATTGCAGGGGCCGACAGAGAAACTACTCAGGGATAGGCTGCAG[CG]CCAGACCTGCTCGCCAGCCACTGCCTGTGCAGCCCCCAGCCTGCAGGTTGTATAGCAGCA |
| 111 | cg12608692 | GCATCTTTAGCAGTCCGGGCAAGGGCATCTAAGCTGACAGACACAAAAATGGGCTTTCTT[CG]GCTGGCTGGTGTTCCCAGCCTTTTATGTGGTGCGTCTCGGGCTGTGCTGCTTAATTCATT |
| 112 | cg20755989 | TCTATAAGTCGTGTGACCTTAGAAAGAGTATTTAATCCTCTAAAGTACAGTTTCCTTTTG[CG]TGCATTAAGAATAATAAAGCCACACAAATTATGATAATTATCTCAGAGCATGCGTGTTAA |
| 113 | cg12238343 | CTGACCTCCAGGAAGCTGAGCGTGGTGGATGGAACTCTACGATCTCTTTCTCTCCAAGGA[CG]GAAACCTCATCCAAGCAGTCCCAGAGGAAACGGATAAAGGTATTTGAAAGGGAGCGAGCG |
| 114 | cg02328239 | |

EP 3 802 856 B1

| SEQ ID NO | Probe ID | Sequence With CpG Marked By Brackets |
|---|---|---|

CCACGTGCGAGAACCAAGCTCTGCTCCTCAAGTGACGGGGGCTCTGCTCTGCCAGGT
GAC[CG]CGCACCATTTCTCGTGCCTGGCAAGCTGGTCCCCTTCTGGGTCCGGGACCACCACGT
CCC

115    cg21878650

ACAGATAACATGTGAAACCACAGCTTTGAATCATTTCCAACTGTGTCTTTTTGTTGGC
TC[CG]GCTTACTTTAGCTACTTACGCTGGACTGTCACAGTGTCTTAGGGATGAGGAGACGCCT
CC

116    cg2692 1969

ACCGGCTTGGAGCAAGCAAGACTCTCCACCCACAAACTGCATATTCTTTAAAGTCACT
GT[CG]CTTTAGGCTCAGATCTTAAGATTTCGGGAGCCAGTTTTCTGTGGCGGGGGAGTGGTCG
GA

117    cg10837404

CTGTTTCTAGATTTATGTTGTTGTAGTTGAACAGCAACTGTTTTTTTCCCTCAGTGTTA
A[CG]AAAGGATAAAGACTACCTGTATTGTTGGGTATGACTATCAAAGGATTTCCGGTGATTCA
T

118    cg01883408

CGCGTGGCCCTCCTCGCGCGTGCACGGCAGGCGGATGTGGCCTCCACCTGCACCCGC
GCT[CG]GGTGTTCTGAAACTGGAGGCCGGGCCCTTCCCCAGGTGTGGCCCCTCACGAGAGGCA
CGA

119    cg06448705

AGGATGTACCGCTCTCCGTGGTGCTGAAGTATAGAGCTGGTCAAGTGAGTTAAGTTGC
AA[CG]ATGTGAAAGCGCGCTCCTCTGTTCTTTGTGTTGCAGTGGTAAAAACTCGCCTTCCGAG
GC

120    cg16983159

| SEQ ID NO | Probe ID | Sequence With CpG Marked By Brackets |
|---|---|---|

CTTGGTGGGAGAGGAGGGGCACAGAGGAATGGGGGTTTGGCTCTTTGCAGGAAATGG
CCA[CG]CCTGTGACTTCTCCAAGAGAGCCTGCCGGTTTCTGCCCAGAAGGCGGTTGTGGGGAT
GAT

| 121 | cg08234504 | |

TTGTATTTCAGCCCAAAGCCTACTGGAAGTGTCAAGCTGCCAGCTCCCCTCTGCCCTC
CC[CG]TTGCTATGGCAGCCATGTCTCTGTGTGTGAATAGGTGAACCAGGCTCCAGGTTAGGAC
CT

| 122 | cg11006267 | |

GGCCGGGTATGGGGAGGGACGCTGTGTCGGGTGCGCCCTGCGCTTGCCCTGGTGGGG
GCG[CG]GGGCTGTTTCCGGCGGGCGGAGGCGCCAGCAGGCCAACTTTGCCGCGGCCCAAACA
GATG

| 123 | cg21874213 | |

CTGGGCATCTCACTGCTCTCTGGAACCAGCCTGGAGTCCCCATTATCATTTTTTCTGAA
T[CG]CCTGACTCCTCCCTCTTCCCTTTCCCACCGGCACATCTGATTAACCACCAAGTCCTACCC

| 124 | cg23500537 | |

CAGGAGTGCGGTGCAGCCACACATCCAAGGCTGACAGGGCGGGCACTCTGCCAAGTC
CTG[CG]CGCTGCTCGCCTTCCACAACACCTTCCTCAGCTTCGTCTGTATTTGAAGAGCTTAGTA
AA 5

| 125 | cg26843711 | |

ATGCAGTATTAAGTTAGGACTCTAAGCGTCGCTGTTGACCAACCTGGGCAAGAAAAT
CAA[CG]GAAACTCAAGTTACATCCTCCAACAACAAAGCAAATTAGACGGGAAAGCAGGAAAG
CTGT

| 126 | cg08587542 | |

GAAGAGAGGAGAGGTTTAGAGTCAAAGAGCCCCAAACATTAGTGAGAGTATATGTAT
GAA[CG]TTTGGTCATCTTAGAACAGTGGTTGGCATCCACAGGAGACCAGCAGAATCACATGG
GCGC

| 127 | cg10345936 | |

| SEQ ID NO | Probe ID | Sequence With CpG Marked By Brackets |
|---|---|---|

AACGGGGAAGAGGCTGAGATTGTATGACTCCCAGCCACAGTTTGCTGGGCAAGATAC

TGG[CG]CCAGGAGGTGGTGAGATTTGTCTAAGGTCACACATGAAATCCAGGATAGAACTCTG

CAGC

| 128 | cg16281600 | |

GCAAAATGACTCATGTAATTGCTCTGTGTAAGTATCCTTAGTCTTTATTGTACACCCAC

A[CG]ATTCTGATGCTATAGACTCCTGTGGAATGCAGGGAAAGAGAGAAGGGGGCCCATTTTA

AA

| 129 | cg03555227 | |

CGGCTGGCCGGCGCCGCCTCCTGGGAAGATGGCGCTGCACTTCCAGGTCAGTGTGCTC

TG[CG]CCGCGGGCCCGCGCTCCGCCACGCTGGGAACCCGGCGGGACGCGTCTGGAGACCGAG

GGC

| 130 | cg14345676 | |

AAAATGATATGAAATTTACATTTCAGTGTTCATTACTGAAGTTTTGTTGGAGTGCAGC

CA[CG]CTCTTCTGTCGGCACGTCATCTGCATAGCTGCATTCGCACTGCAAAGGCAGAGCCGAG

CC

| 131 | cg14314729 | |

AATGTCTTGTTTTTTTAACATGGCCTGGCCTAGTCTCTGACCCTGGCAGACAAAGTAA

TT[CG]TTCTTGAGGTGTGAGGACCCGTCAGACTTTCTGCCAGGAACCACAAAGTGGCTGTGCG

TG

| 132 | cg23517605 | |

CTCCAGTGCCGGCAGGTGGGAGGGCTGAGGTGGCACAGGCTGCTCCGCCACCTCGGA

CTG[CG]GCTCCTACTCGGCCACTGGCCAGAGTCCCTCCAGCCAACTGCCCCTGGTGAGACCAC

CGT

| 133 | cg01570885 | |

| SEQ ID NO | Probe ID | Sequence With CpG Marked By Brackets |
|---|---|---|
| | | GGAGGAGGGTTGGAGAGCAGGGCCGTGTTGCAAGGCTCTCTGGGTGGCCACAGCAGCTTG[CG]CTGCGCCCACATTGCTTCTGCGTGTTTACAGTTGGGCACGAGAAGGCTCAGCACGCACGC |
| 134 | cg23375552 | GCTGACCCTCTGGCCACGTAGTCAACCCGAGGATGTGTGCCCCGGGGCTCGGCCTTGCCT[CG]GGTGAGAAGGCTAGTCACCATTCAGGGTGCAGGTGTCATGGCCTGGAAATGGCAATATCT |
| 135 | cg20052760 | CTTGCGCCTCGAATGCCACGTTGAATACTCCTCATGTCTTTGGAGACATGTCCTTCCCTT[CG]AGCTGCTCCCAGTCAGGTGAGGAATAAAATGCTATGATGGCGTGAAAATTCTCCCTTGGT |
| 136 | cg16867657 | CCGCGGCGTCCCCTGCCGGCCGGGCGGCGATTTGCAGGTCCAGCCGGCGCCGGTTTCGCG[CG]GCGGCTCAACGTCCACGGAGCCCCAGGAATACCCACCCGCTGCCCAGATCGGCAGCCGCT |
| 137 | cg21572722 | GGCCGGGCGGCGATTTGCAGGTCCAGCCGGCGCCGGTTTCGCGCGGCGGCTCAACGTCCA[CG]GAGCCCCAGGAATACCCACCCGCTGCCCAGATCGGCAGCCGCTGCTGCGGGGAGAAGCAG |
| 138 | cg00194146 | TTATTGTAAACCCATTTTACCAGTGATGTGAATGAGCCGCAATGAAGGCTAAGGGACTTG[CG]CAAGGTGACATATATAAGCAACAGGCCTGCGATTGGAATCCAGGCCCCAGAGTCTGGGCA |
| 139 | cg01527307 | |

| SEQ ID NO | Probe ID | Sequence With CpG Marked By Brackets |
|---|---|---|
| | | CCCTACACCACACGTCTCGTTTCAGGAGGTGGCAGATAGTGACATTTTATGGAGAGCTTG[CG]CAGGGAACGTGTGGGAAATGAAAAGGCAACCCAGCTAATCGCACCCATAATTTCTAAGCT |
| 140 | cg22736354 | TGCGCCAGGGCGGCCACGCAGGCCAGGCAGACCACGTGGCCGCAGGACAGGTTGCGCGGG[CG]CCGCTGCTGCCGGTGGCCAAACTTCTCAAAGCACACCTTGCACTCGAGCAGGCTGATCTC |
| 141 | cg10699171 | GTGAACACTGAGCTTCTACGCGAGCACCATTGGCTGGCATCACCATATCGAGCTACCCAA[CG]TGTGCCAAATTCTGTCTGGCTGCACAAACAAACACATCTCTCTGAGTAATACTGAGAC |
| 142 | cg06493994 | GGAGAGCAAGTCAAGAAATACGGTGAAGGAGTCCTTCCCAAAGTTGTCTAGGTCCTTCCG[CG]CCGGTGCCTGGTCTTCGTCGTCAACACCATGGACAGCTCCCGGGAACCGACTCTGGGGCG |
| 143 | cg04424621 | CACCCTACTGCATGTTGCAAAGTATTCCTTTAAAATGAAGTGAGTAAAATACTGGGATGA[CG]TTATCTGGAGCCCAAGAAAGATGGCTCATTTGGAAAGGCCTAATATCCCAAGTTGCTTAC |
| 144 | cg02281167 | CCTCTTTCTCCGGCAAAGTCTTCCCTTTCTTTGCCGTCTGGAAAAAAGGTTCCTGCCTTA[CG]CTGAAAGGCTGAAGTGGGGCGCGCGAAGGGCGGCGAAGCGGAGACGGCGGCTCTCCGGGA |
| 145 | cg03771840 | |

EP 3 802 856 B1

| SEQ ID NO | Probe ID | Sequence With CpG Marked By Brackets |
|---|---|---|
| | | GCCGTCTGGAAAAAAGGTTCCTGCCTTACGCTGAAAGGCTGAAGTGGGGCGCGCGAA GGG[CG]GCGAAGCGGAGACGGCGGCTCTCCGGGATCCAGCTCCGCCCCTGGCCAGTGTGCGG CCCG |
| 146 | cg06685111 | TCACCACTTCTTTGCCAGTCTAGATCCGTCCTGGTGCCTTACTGTGCATACAGTTCTAC T[CG]TCTCAGGTGAGGAGGCCACTTAATTTGTAAAAGACTGAGGAAGGGGTAGGATCACCAC AA |
| 147 | cg06462220 | CTCAGGCCTGTCGACCCACCCTGTGATTTTGACCAGATTACAGCACTCAGGAAGAGTT CT[CG]TTTTGAAACCTGAAGACTCAATGTGTACTTCACTGCCGGGGACCTCAGTTTGCCCATCT G |
| 148 | cg08420066 | TGGGAGGCAGAGGGGTAAAAAGAAATTAAAATACATGGCGATAAGTCTTGTGATCAG AAC[CG]AGTCTTTGGGCACCTTGGGGGCAATCGAGTGAACTTCCCAGAGGAGCCCAGCAGAC TGGC |
| 149 | cg21467614 | GACAGGGGGGCCCCAGGGCTCCAGGCGGTGCTTGTTCCTCAGCCTCTTCTCCTTCCTG AT[CG]TGGCAGGCGCCACCACGCTCTTCTGCCTGCTGCACTTTGGAGTGATCGGCCCCCAGAG GG |
| 150 | cg12753631 | GGGAGGCCCGAGCTACCAATGGTGGCTTTTCTCAACTGGGCCTTGATTCCAGCTTCTG CC[CG]ATCCCCTACCTTGCTTGCCTCCTTCTATCAACACCCCATTCACACCCCAAAGGATCAAT A |
| 151 | cg18501647 | |

EP 3 802 856 B1

| SEQ ID NO | Probe ID | Sequence With CpG Marked By Brackets |
|---|---|---|
| | | CGGCCTTGAAGATGGCAATGATGCCAGTAGGCCAGAAGCAACAGATGGTGGTCAGCACCG[CG]ATGGGCATGTAGTCGTGTGGCGGGCGCCTCGGCTCCAGTAGGGCCAGCCCTGGGCCCTGG |
| 152 | cg04576021 | AGGAGCAACCTTTGTTTCCAGTTTCATTTGTCCACATATACCCCAACTGAGATTTGTTTC[CG]TGTCCTGACCAAAAAATCACAGATTGCCTCTGTGACCCAGCCTACTGCAGGTTGTTTCTC |
| 153 | cg10192196 | GTGAGTTGTGAGGCGCGCCCAGTCCCTCTGTTCCCGCCTGGCACTTGCTCTGGCCGCGCC[CG]CCCCATCTGCCACTTCGGAGAGGCCACGGCTCTGAGCTGCGGCCGCTAGTGCCCTGATGG |
| 154 | cg18468088 | TGACTTAGCCTTACCACCAGGTGGCGACACGAACACACCCACCGGGGAGGACACCGGCCC[CG]CGGAAGGTGAGGATAACTGGGAATACCAGGCATGTTACAGGACTTGGTTTTGGTTTGGTT |
| 155 | cg03894990 | TAATGACCATTTATTTCTCTTATAATCAGTAACAAAAGAAGGGAAAACTTGGTCTAAACA[CG]AATTTAGGGACTTAAACTAGACTTGGAGAAAAGCTTTCTATGCAAGATTTATTAGATACT |
| 156 | cg01740766 | ATGGGACACTAGTAAACGTCCCATAGTATATTTTGTAAGAGTAATGAAGTCTCAGGAACC[CG]GCCCTCCCCGCGGCCTCTGCTAATAAATTTCCTTGGGCGAGGGGTGAGCTGCCAGGCGCT |
| 157 | cg16255583 | CATCAAATCAGAAACCTCAGAGGCCATTGGCAAGGTTTTAGCCAGCTGAAGTGGAGCCTG[CG]AAGTGGTCGCAACAGCACGATCAACTGAAGTCGGGATTGCCAGTAATTGCCAATTCCACC |

EP 3 802 856 B1

EP 3 802 856 B1

| SEQ ID NO | Probe ID | Sequence With CpG Marked By Brackets |
|---|---|---|
| 158 | cg04642300 | TTTGTTACCCAAGCCTGGGGCAATCAGCCATAAATAACAAGGATGGTGGGGCTGCGGGGC[CG]GGGCCGTGTGGCATAAAGATGGATCAGAAGGAGGTGTGGGCATGGCTGGCTTCTCAGCAG |
| 159 | cg17266282 | TATGCTTTCTTATTACCCAACAAGAATGTTCTCGGGAGTGTTGTTGCGATGACTCGCTTG[CG]AGTGATCTGACGGAAGGAAGGGCGGCTGAGGAGGAGAGGAGGAGGGAGCAGAGCTTGCCT |
| 160 | cg07095347 | GGCTCAGCCCAGCTTGCCCTGTGTGGTTTAAGGCCTTTAACTATGAGGCAGGTCATTAAC[CG]GCTGGTGAAGCAACAGCACATTGTTCTGTTATTTTCAAACCACAACAGCCTCTGTGGAAT |
| 161 | cg00073460 | AGAAGTTCTCCTGCCTCCAGCTGAGAAGATGATCAGATTCTAGCTGCTCCTGGGGAAAGT[CG]GTACTCACAGCTGGACACAAACATAGCTTGCAGGAGGAAGAGTGTCAGAGCAAGAGACAG |
| 162 | cg16333846 | TTTTCATTGCCTGGGGATGAGAGGGAGAGACAACGTGTGTCTTACACATCTCCCAACAGC[CG]ACTTAGATGTGATCCGTTCTCCCAGAGGGAGCAGGTTTCTTTGAACTTTTCCTTTTTATG |
| 163 | cq13221458 | AGCAATACAGAGAGTCTAAAAAACATGACTATCGATTATCTTCCTTGTGCAAACCACTAA[CG]AATAAATTAAAAAGACAATACTATTTTGTAAAAAACGTTAAAACATAACATTCCCATACA |
| 164 | cg05468948 | |

(continued)

| SEQ ID NO | Probe ID | Sequence With CpG Marked By Brackets |
|---|---|---|

TTAATGACAAAGGCGCAGACATACAGGGTCTGTCACTCACCCGTGCTCAGGTGGCTG CTG[CG]CCTGGAGAACGCGCTGCTTGCGGATTCCTTTCCTTCCCTTTGAGTTTCTTTACTGATA TA

165 cg00795927

GGATTATAGCTCTTGGCAACACACGGACGGCAGCAGGCACTTTCGGAGTCTCTGGAA AAC[CG]TAATTCAAACTGAACCTGGTGCTCTTGGCATTTTGTCACCTGGCCGTCCCCCTGGACC CT

166 cg08911208

CCCAGGGCAGCAGAGCATTCCCTGGCCTTCCCTGCTGGTGCCAGCTCCTTACCACAGA GA[CG]CCGCGTGGAACTCACTACTGGCGATCGCGGACGCCCCAGGAAGGCGAGTGGCACGAC GTG

167 cg22372849

TCCCAGGCTGTCCTTCGAATAAAGTCCAGGTTGCTTATCAGACTTTCCGCAGGCTTAT CA[CG]CTGCATCTCCCCGGCCGCACCCTGCCACGCTGACCCCAGAGCTTTGCGCCCGCACCGG CC

168 cg16012294

ACCCAGAAACAATACAGATGTCCTTCAACCAGTGAACGAATAAACAAGTCCCAGAGC AGC[CG]TGCCTGGAGCATCACTCAGCAACGAAAAGCAGCGCTGCGATTCACACAGCCACGCG GTGA

169 cg22679120

AAAAAAATTACCGGGCGTAACTGCACGCGCCCGTAGTCCCAGCACTTTGGGAGGCTA AGG[CG]GAGGATCACTTGAAAGAGAGAGAAAAGCAGCTACACATCTATAGATTCGGTTCACA GATG

170 cg13931228

| SEQ ID NO | Probe ID | Sequence With CpG Marked By Brackets |
|---|---|---|
| | | GGTGTGAATCACACTGCCCGGTCGGGCCTTTGGGAAAAAATTAATGAAGGACACAGTCAG[CG]CCGTAGAACCTGCCAAATACACATCAGATCCAGTGGAGTCTGTGAAGGGGGAGGGGGAGA |
| 171 | cg27009703 | CGCAGCGGGTACAGCGTTGGCGCCCGCCGCGTGCACTGGGTTCCACGAGGCGCCAAACAC[CG]TCGCCTTGGACTGGAAGCTGCACGGGCTGAAGTCGGGGTGCTCGGCCAGCGTCGCCGCCT |
| 172 | cg11671968 | CACAGTGTCTTATATCCTGCCTACAAACTTGCCTTAGCTATGGCCTGCTGATGGCTCTGA[CG]GGTAGAAAAGGCTGCTTTTCATCCCTAAATCCCCACTCAGACCCTAGCCCAGTTTCCTCC |
| 173 | cg26312920 | CTCTAAAAAGTGACATTGATGCCAACTGCCAGAGCTGGTACCCATGCCATCTGCTAGTGA[CG]TCACAGGGCAGAGAGAGCCATGTGATCCTCTCTCTTGGGACCTTCATTCTGCACTGATCA |
| 174 | cg19663246 | AGGTAATTGTCAAAGTCACCGGAGGCTCTATGATGTGAAATGTACAATCGAATTAGAACT[CG]CCCCTTACCAACCATTCCAAATAGCTTGTCCTGTCCTTTCGAATTTGGGTTTGCCCAATG |
| 175 | cg14396995 | TGCCTTTGGAAGTTCAGGGTTTTTCTCTCCACCGGACTCGTCTGCCCTCGGGGCCAAATC[CG]CGAAGCGAGGAGGAGCTCCCACCACACAGCCTGCTGTCCCTATGGGCCACTTTATAAAAG |
| 176 | cg18442362 | |

EP 3 802 856 B1

| SEQ ID NO | Probe ID | Sequence With CpG Marked By Brackets |
|---|---|---|
| | | AGAATTAACTGTGTGTAACTGTATATTTGAGGCAAGGCAAGGGGACAGATATTTTCCTTA[CG]TTATTAGTTGTGCAACAGAAGCCAATTAAGAGATTGGAGAGATGAATAACACTAGTGATG |
| 177 | cg09748749 | CTGGCACATAGAGGTGCCTGGTACGTGTTTGTTGAATGAATGAATGAATGAGTGAATGAG[CG]AACATGCCATTTCACCTTATATATCTTGTGAACCTGCCAGGCCCGGGCCTGATGTCATAG |
| 178 | cg20692569 | CGACCCGGAGCGCGGGCGCGGGGCTGCGCCGTGCCAGGCGGTGGAGATCCCCATGTGCCG[CG]GCATCGGCTACAACCTGACCCGCATGCCCAACCTGCTGGGCCACACGTCGCAGGGCGAGG |
| 179 | cg23857078 | ATTAGAAAATCAAGTTTAGGTAAAGCATTTGGCACAGAGCTCCTAAGTACCCCTAAATGG[CG]GGTTTTGAGCTTGATGAGGAACTAATACAAATTAGGTTGTCTTATTCAGGTGGAACAACA |
| 180 | cg21743182 | GGGATTTCTGGGCTTTTTTTTTTTTGCTTGCTTATGCATCCCCCTCTCTTGGTTGTAGTA[CG]GCCGTACCATTTCAGCTTGCTAGTGCAGAAAGATGTGAATTCAGTTGCTGTATGAGCCTG |
| 181 | cg09436502 | CGCCCCCACCCCCACCGCCCCCTTTTCTTCAGAAGAGACCGGCACATGGCAGGAACTCTA[CG]TTCCTTTTGCTGAGACTTGAGGGGCTGCCCAGATACATTTACTTTTTCCTGTGGTAATAA |
| 182 | cg00503840 | CTGGAGGCATCTTCGGACCTCTGGGCGGCCCAGCCCTGCCTGGCGTCTCCCCGCCGCTTG[CG]GCCTACCGCCAAGAAGCTATGCCTTAGGCAAACCATGGAGCTCTGGCCCCAGAGGGCGCC |
| 183 | cg04084157 | |

EP 3 802 856 B1

| SEQ ID NO | Probe ID | Sequence With CpG Marked By Brackets |
|---|---|---|
| | | AGGGTGCCTGCCTCTCCCGGCCTGCGCCTGCGCGCTGGGGCCTTCGGCTGAAGGGGTGTG[CG]CTAGCGGAGCTCCGGGAAATGAATGAATGAATGAATGAATGAAATGCTGAAGCGGGCAGG |
| 184 | cg14175438 | CGCACAAAATCCCAGCCTCAAGGGCAGAACATTTTAAATGACCCACCCATCCTAGAGATG[CG]CCAGTTAGGTCATCTTATATATCTTGAGATAGCTGAGATGGTCAGATCAACCAAGGACCT |
| 185 | cg20665157 | AACTCTTTCCATTGTCAATAGAAATTGACAAACCTCATCTCCTAAATAGTGCAGCTGAGC[CG]GGCGGGATCCACGCAGCTGTAAAGGGCTCTGCTCTTGGGGCCGGGGAGCACTAACAATAG |
| 186 | cg21184711 | CATAACTAAGAGAGGAGTACCCAGTAAGGCAGTGTTGCAGGAAGACAAACCCTTCCTCTG[CG]ACAGAGCCCACAGAGGTCACTGCTGGAACAATGGGGAAAGGAGAAACTGAATCTCTCCTC |
| 187 | cg02383785 | TCACCTAGGGCGGAGGCGCAAGCTCTGCTGGGTGCTCTCCGCCCCCTTGATCGCCGCTCT[CG]GTTTTCAGCACCAGGATCCGGACAGCTCCCCACCTGGCCCTGAGGGGCCTCTTTCCTTCC |
| 188 | cg04528819 | GCAGCCCGGGAAGGGGCATTGGTGGCGCTTGGCAGCAGGTGTGACAGACCTCCTCCGGGG[CG]CCTGATCCGCGGCGGGGGCGGGGCCTGCCCCTAGGGCCCCTCCAGAGAACCCACCAGAGG |
| 189 | cg20426994 | |

EP 3 802 856 B1

| SEQ ID NO | Probe ID | Sequence With CpG Marked By Brackets |
|---|---|---|
| | | GAAGGGGCATTGGTGGCGCTTGGCAGCAGGTGTGACAGACCTCCTCCGGGGCGCCTGATC[CG]CGGCGGGGGCGGGGCCTGCCCCTAGGGCCCCTCCAGAGAACCCACCAGAGGCTGCTGGTG |
| 190 | cg08097417 | CCGGCTAAGTCATGTTTAACAGCCTCAGAAATTATCTTGTCTCCGCGTTCTTTCTTCTGC[CG]GCGAGCCAGGTAATGGTAACAGAGCGAAACTCCCCAGTCGGAACTTCTGGGTTGCAGCAG |
| 191 | cg02821342 | CTATATTAGGGCTTTGTTGCTGACAACAGTGAAAACTTGTTTGTGTCAGGAAGTGAGGTA[CG]GAGATATGACCTGGAAGGTACAGACAAAACCAAAGTGGCAGTTTTTGCATTACTTTTCTG |
| 192 | cg20397034 | CACTGGGGTCTCCTCCACACCCTTCTCTCTGGTCCCATCCCTTCTGCTGCCAAGCCCCAG[CG]TTCCTCCGGCTCGGCCTGGTCAGCTTGAGCCTCATTTTGTTCGCGTGCCCCTGGGCTGGG |
| 193 | cg03473532 | AATTAAAGACTAATTCAGAATTTTCAAGTGATAGTAAACAACTGCTATCTCAAACACATA[CG]ATATAAAATGAAACCACTGGTGCCTAACTGCCAGTTCTTTCACTCAAACCTCTGCTGTGA |
| 194 | cg08280936 | GATCCTGCTTGTCTGCTCTGGAGTCCCCCCACCCTTGCCAGGAGCTTCACAAACCAGAGA[CG]GGCTGTCAGCAAGAGCTCAGACAGGATGTGGTGCAAGTGCAGGTGCACGAGTTTAACCCT |
| 195 | cg08540945 | CCCCGAGGCGGACGCCAGAGGGCGCGCGCCCCCCACTCCTGCCCGCGTCGGGGCCGCAGC[CG]CGCTCCGCCCTTTGCCTGCAGAGCGCTGGGGGTTTAAAGTCCTGAACCCATGCACGGCTG |

EP 3 802 856 B1

| SEQ ID NO | Probe ID | Sequence With CpG Marked By Brackets |
|---|---|---|
| 196 | cg18769120 | GCGCAGAGCGCTGCCTGGCCGCAGCCCATTGCTCTGTTGTTCTGAGGGGCAAGGCCACAG[CG]ACCTACAGCAGGGAAGAGACAAACACAGATCTGGTGCAGAGATTATTCGGGTCATCGATG |
| 197 | cg26101086 | TTTGTCACTGTGAGAGAGACTCGATCCTGCTGTGTGAGTTGACACCATGGGTGCAGTATT[CG]GCACCACAGTACTCCTGCACATTGGAAACTGGGAGACTGGTGTTTTGAAGAAAGTAGCTG |
| 198 | cg19859445 | GTAAGGTGAACCCACCGGAAGGAATAACTAGGCCATCATTCTCAGCTGCCTGCTGTC̈GT[CG]TTGTGTGCAGAGCTACAGGGGTGATGCCCACCTCCCAGGTGACAGCCACCCCTCCCACGT |
| 199 | cg07502389 | CGCCTCCACGGGGCGGGGCCCTGGCCCGGGACCAGCGCCGCGGCTATAAATGGGCTGCGG[CG]AGGCCGGCAGAACGCTGTGACAGCCACACGCCCCAAGGCCTCCAAGATGAGCTACACGTT |
| 200 | cg18267374 | GGGGCCCTGGCCCGGGACCAGCGCCGCGGCTATAAATGGGCTGCGGCGAGGCCGGCAGAA[CG]CTGTGACAGCCACACGCCCCAAGGCCTCCAAGATGAGCTACACGTTGGACTCGCTGGGCA |
| 201 | cg00582628 | AAAACATGCCCCAGCTTTCCCAAGATAACCAAGAGTGCCTCCAGAAACATTTCTCCAGGC[CG]TCTATATGGACACAGTTTCTGCCCCTGTTCAGGGCTCAGAGATATAATACAGACATTCAC |
| 202 | cg16419235 | |

EP 3 802 856 B1

| SEQ ID NO | Probe ID | Sequence With CpG Marked By Brackets |
|---|---|---|
| | | CTGCGCCCTCTGCAAAGGGCTGATTTCTACAGTCGCTAGGACCTGCAGCGGCGCTGCTCC[CG]CGGGGCTCCGGCCGCGCTGCATGTCCCATTATAGTCGCTAGAGGGCAGCGCTCTCCTGCG= |
| 203 | cg23710218 | GCTGACCCCGGGGAGCGTGGACTACGAGTTGGCGCCCAAGTCCAGAATCCGCGCGCACCG[CG]GTAAGCTGCGCCTTTTGAAAAGGCTATCTGTACTCCTTGGAACAAACCACCCCGGGCAAA |
| 204 | cg07583137 | CAAACACCAGGGCAGCCCCATTTAAGGTTTTTGATACACTGAGGATCATTCAGAAAACTT[CG]GATTCCTAGTTATAGAGTTGAATCCAACCACCAACACACTCCAGAAGTCCTGACATTAGG |
| 205 | cg12402251 | GGAGGGATAATGGGATCAGGAGGCTCAGAAAAGGGCAAAGAATGGGAAGGGGCATGGAAA[CG]GGTCTTGAAACAGTTAAAAAGAGAAGATAATCACCGTCAGCGTCGAAATGGAGCCAGATC |
| 206 | cg19497517 | CAGGTCACCAGGCCGGATCCAGGAGCGCTCGGACGGCCCACTCCCCAGCTCCGCAGCCCC[CG]GCCCACCCCACAGCCCCCCGAGTCCACTGCAACGAGCCATGCTTAGAACAGCCTGTCGGA |
| 207 | cg13586038 | GAAGATACCAGGGAAAAGTCTTGTCAAGTAGCAGGCCACCGGTGTCTAGTGTAGAGGAGA[CG]ATTTCTGTCGATAGAGAGCAAAGCCAGCCAGGCAAACGAACCCGTAAGCCGCCTGAGGGA |
| 208 | cg19724470 | |

EP 3 802 856 B1

| SEQ ID NO | Probe ID | Sequence With CpG Marked By Brackets |
|---|---|---|
| | | CATTCTTATGCGACTGTGTGTTCAGAATATAGCTCTGATGCTAGGCTGGAGGTCTGGACA[CG]GGTCCAAGTCCACCGCCAGCTGCTTGCTAGTAACATGACTTGTGTAAGTTATCCCAGCTG |
| 209 | cg07211259 | TCCCATTCACAGACAAACTGCTAAAAGCAAAACCAAAACTTTCCAAATAAGCCAGGCTTT[CG]TCAGTTCCTCAGAACTAGTTCTGGTTTGACTCACTCTCATGTTACGGCAAACCTTAAGCT |
| 210 | cg07158339 | TACAGGGCTTAACTCATTTTATCCTTACCACAATCCTATGAAGTAGGAACTTTTATAAAA[CG]CATTTTATAAACAAGGCACAGAGAGGTTAATTAACTTGCCCTCTGGTCACACAGCTAGGA |
| 211 | cg24046474 | CTGTGCAAGGATTAAATAAAGGCCTAATGAAATTCAGAGAAATCCAAGAGGACAGAATGA[CG]GGGAAGCCAGCAGTTGCTCAGCAGGCATGAGACACAGCCTGCCACATTAACTGCTAGGTT |
| 212 | cg14059835 | CTGGGGTTCCCCTTTCTGGAAGACCATTCCGAAGCAGGGCAGCATTTCTAGAATGCCTTA[CG]TTTTCTCTGGAACAGTCTCCACTGAGATTGTTCTTCTCTTCCTTGGGCTGGAAAAAATAG |
| 213 | cg10570177 | TAAGCTGTCCAGACCTGGCTTGAAAACCCATCCCATGGCAAGGCAGGGATTCGCTGGCCG[CG]GTTGGCTCTATCTTGATCTGAGCAAGCCGCTGGACGTCCCTAGTTATCTTCTTCCTATCC |
| 214 | cg13649056 | |

| SEQ ID NO | Probe ID | Sequence With CpG Marked By Brackets |
|---|---|---|
| | | CGCGCAGTCGTCGGGGGATGCCGGGAGCGGCCTGGGGAGCTGTCCCTGGTGCTGACGGCT[CG]TCCGCTCTCGCCCGGGACGCGCGACCTCCTGGAGGCCTGGGGGTGCCCCACCCTGGCCG |
| 215 | cg13734401 | CTCAACTCTTCCGAAATTTGCCATCTCCTAAAGTTCTTTAATCTCTAGCCACGGGGGTTC[CG]GATTTCCTCCGGGTCTACGGGGACTCAGGGACTGCAGAGGCAGCTGTGGGGGGTGGCATG |
| 216 | cg26581729 | GAGGCTCTGAGGCTGCAACAGTCTCCCTCCTATTGAAGCTAGAACAGCACCCCGAGCCTG[CG]CCATAAGTGCCCCCAGAACTTCAGCGCCCACCATGGCGCACAAGGCCGGTGCCCAGCGCC |
| 217 | cg06231995 | GGCGCGGGGATGGGGCTGGGCCGCCCTTGGTAGCCGTCCTGGGCTGGGGGCCACCCTGGC[CG]CGTGGTCACCGGCAAGAAGCCCAGGGCCTCACCCGGGCGCGGCGTCGCGGGGGCCGAGGG |
| 218 | cg14411282 | CTTTTTTGGCACCTCCAGGTTCAACCACCAGTCTGTCTCTGCTGTGCCCAGGGTAGAGCC[CG]GGGGCTGTGAGTATGTGTGGCTCCCCTGCCCGTCATCGCTCTGGCTCAAGCTCATGCTGG |
| 219 | cg12530994 | TAAGAATAATTCCTTTTAGTTTTCGGATTTCAAAAGAATAAACCTAGTAGAAGTGAAACC[CG]TATTGGGTTGTAAGGTTCGTGTTCCTACCTTACTCTGGATGACTCACTGGTCTAGGTTTC |
| 220 | cg23754392 | |

EP 3 802 856 B1

| SEQ ID NO | Probe ID | Sequence With CpG Marked By Brackets |
|---|---|---|
| | | AAAATGCTGAAGTTTTCAAGGTGGTGTGTGTTGGGAGTCTTGGATAAGTGCTCTGAACAT[CG]CTTGGGAGGTGCTCCCTGGGAAGTGGGCATTTCAAATTTGGAGCTTTTTGTGGAGTGAAG |
| 221 | cg06908778 | TGAGTCAGAGGCAGGTGCTGCAAGGTAGGGCCGAGGCGGGCAGGTGCCCTAACTAGCTGG[CG]CCGAGGAGACCCGGGTGCGGTGGGCTCCACCGACTCTCTCTCCCGCAGTGTTCGAGCAAT |
| 222 | cg22796704 | TCCTAAGCCTCTCTGAGCTGGGCTTGGCCACCTTCCGGGGTGTGAGCGTCCACGGGACAT[CG]ACCACACCAGGCACCCAGGAGCAAGTGCTTTGAAATGCGGCTTTCTCCGGACCTTGCAGG |
| 223 | cg01560871 | GGTTTTAGCCAGAGAGAAGCGGATGGAGGCGGAACGCTGGCAGAGGACGTTGGTGGGCTG[CG]TCCCAGCTTCGTCAGCCCCACCTGGCCTGACCCCACCACACAGGGGTCGGCTTCCATGCA |
| 224 | cg04268405 | TGACGTTACGTACTGGAAGTCCCAGGAGGAATGCCCAGCAAGTGGAATCCAAGACGTTCT[CG]CCTTCTCGGGGACAGGGCCATCACCAGGATTCGGAAAGGAACAGGGAGGTTCGGTTTGTG |
| 225 | cg18738190 | ATCTTAACCTACCAAATTGTTGGCACAGCCTGCAGTTTGAGAAATGTCACTGTTGACCAG[CG]ATTTTCAAACGTTCGTGTGCATCAGACTCAACTGCAGAGTGTGCTAAAACAATCTGCTCC |
| 226 | cg04126866 | |

| SEQ ID NO | Probe ID | Sequence With CpG Marked By Brackets |
|---|---|---|

CTCCACCAACAGGAGCTCCTTGAGGCGAGGCACAGTGTCTTCTGTGTCCCTGGAGCCA
AG[CG]CATGGCTCAGCCCAGGTCACGTGTCCAGTGAATGGGTGGCATCTGAGCCTCCTGCACC
TG

227 cg25427880

CTCGCCCGCAGCCCAGCACGTGTAGAATCCAGATGTGGCTTCTGCTGGAGCCACGTGT
TC[CG]GCCTGAGCGACGTCGCACGTGGCCTCCTGGCCGCAGAGCCCATGGCGCGGGGGGCCA
CTC

228 cg09671951

GCATGGCCCAGAGAGGAGGAGCCGACCATGTGACTTCAGTTTCCACTGGCAGCTGTC
CGC[CG]GATGTGCACTGTGGGCAGGGCCAGCCTGAGTTGCCGCAAATACTGTGGCTTTAGTTT
ATT

229 cg06888746

TCTGTGTCCTGCGGCAAAGCCACCACGAGCACAGACAGGCTTGCGGCACCAGTCCTC
TCC[CG]TTGCACGCCACACAGCGCTTTCCATGCATTAACTGCTTGCGATGTCACCAAACCATG
ATC

230 cg24838825

AAATAAGCAGCAGATGCAGCAAGGCCTCTGCAGATTTAAAAAAAAAAAAAAAAGCAT
GTTG[CG]TCAGAGCACATGTCTCCCCAAAGGGTACGTGTACGAACAGCATGCAGACTTGTGA/
CTGA

231 cg13848598

CTACAACGACCCCAAGTGCTGCGACTTCGTCACCAACCGGGCCTACGCCATCGCCTCG
TC[CG]TAGTCTCCTTCTACGTGCCCCTGTGCATCATGGCCTTCGTGTACCTGCGGGTGTTCCGC
G

232 cg07906193

CTTTCCGTCCTAGGCCTGATTATGGACTGCCAAGACTTTTTGGAGAAAGCAGTTTCTT
GT[CG]CTCTTCTTTTTTCATTCTTCTTGATTTGCTTCCCTCTAACTATTGTCCCGAATCTCCTCC

EP 3 802 856 B1

(continued)

| SEQ ID NO | Probe ID | Sequence With CpG Marked By Brackets |
|---|---|---|
| 233 | cg12776156 | TTCTCCCAGTCAGCCTGGGGTCCTCCCGGGTCCCCGTGGCACCTGCCCTTGCCCTGGCCCA[CG]AGTAGGTGCTCTGAGCGGCTGCCCAGGTCACATGTGAGCTCCCTGGAGGCGCTGCACACGG |
| 234 | cg05928581 | ACTGGCCACCTCTTGGGACCATGCTGTGCCAATACCAAACCGAAGATGCTGCGTTGGTGG[CCG]TCTCTGCCTCTTGGGTCAACTCTGCAGTCTCGCCAGTCTCGGCGGGGGTTGGGCCCACCAGGAAAGGC |
| 235 | cg17627559 | GGAAGCTGGGCTGTGCGTGTATGCGTCTACCATGTGGGGGTGCCTGTGAGTGTGCTGCGG[CG]TCTGCAGTGAAGGCCTCCTGAGACCACTCCACGGAAACACCGGGAATCCCTGCAGCTGAG |
| 236 | cg23091758 | CAAAGCCGGCGAGGAGGCGGCGGCGCTGGGTGGGGACTGACCCGGCAGTCCGAGAATCCAC[CG]CGGCCTTTCACCCAACCGCCCCTCCTGCGTGGGGCCCCGCATCCCCTGGACTGGCGT |
| 237 | cg04940570 | GCGCACACACGCACACACCCTTCGGCGCGCCTTGGACGGGGTGCGCTGGGGAGCCAGAAGTTT[CG]GAGCGAGCGGGGGCGGCAGAGCCGGGCGCCTCGGAGCCCGGAGCCGGCCTGCACCCCCCT |
| 238 | cg10825530 | AAGATGTCTTTTGTTCTTTCAGGACCAGCCTGATGGAGGCAGCTTAAACAAACACCAC[CG]GAGTGGCGCAGGAGTTATAAAGTGCCATATGTGAAATGAACAAAGGGGCTATACTAAAGCC |
| 239 | cg20654468 | |

| SEQ ID NO | Probe ID | Sequence With CpG Marked By Brackets |
|---|---|---|
| | | ATAACAAGACAACAACTGCAGTAACAATCCAGTCCAAAAGTATTTGCCAAGAGTTTATTC[CG]CGGTTAGCACCAAACTCTCCATCTATTTTGCCACTGCAAACAGTGAACCCATAGTTCCCC |
| 240 | cg26552743 | CCAAGGGTATCAAAACAGGATCTCTGCAGATGGAGCTCAGTGTTATGTGTTTTGGATGCT[CG]CAATAAGATTTTCATGCACCATAAACTTTCCTGAGTATCTCAACCAGTTTTGTTGATGCC |
| 241 | cg21992250 | GTCGGGGAAGGCGGTGGCGGGCAGCAGGCCCGAGGCGGCCAGGTAGAGCAGCAGGCTGAG[CG]CGACCGCGCGGTAGCGGCCCAGGTACACGTCGGCCAGCCAGCCGCCCACGGGCGCCAGCA |
| 242 | cg15015340 | TTGGAGAGAGAGTGGGATGATGTCACTTCCTGAGGGTGGGGGGAGGAGTAGGCACGACCC[CG]GCAGGCTAGCCCGCCAGCCCGCCAGGCCACAGCTCGCCAAGTGGCTGCACCGGGGATAGG |
| 243 | cg22843803 | AACATACTGACACTGTTTGGAAATGGCAACAGGAAGATAGCAAAATGAATACTAACATTA[CG]AAAAGATGAACAGGTACATGTTCCAAGGCAGGTGGCTGTGAACTTCCTCTGAGTGAAGGC |
| 244 | cg02532488 | GGGCAGCGCTCTCTAGGGTGGCACCAAGTTGCTGGTTGCCCTCTCTCCACGCAGCCTCTG[CG]CGCACCGAGCGGGCACTGCGGTCGGGCGACACCCCTTCCACGCCCCCCTCCCCCGCCCCC |
| 245 | cg13547237 | GCAGTGCATCGAGCTGGAGCAGCAGTTTGACTTCTTGAAGGACCTGGTGGCATCTGTTCC[CG]ACATGCAGGGGGACGGGGAAGACAACCACATGGATGGGGACAAGGGCGCCCGCAGGTGGG |

(continued)

| SEQ ID NO | Probe ID | Sequence With CpG Marked By Brackets |
|---|---|---|
| 246 | cg06419846 | CACCGGGCTCACACTGCTGCTCGCACGGAGCCTGGGCACAGGGGTCCTCGCAACTGCGCC[CG]TCTGCTGCCAGCCGGAAGCCCTCAGTGCAGCGGCAGGACACGTGACCATCCACCTCCTCC |
| 247 | cg05496363 | CCAGCCGGAAGCCCTCAGTGCAGCGGCAGGACACGTGACCATCCACCTCCTCCACACATT[CG]TGTTCGCAGCCCCCGTTGTCAGGGCTGCAGCCAGTCCCCAGGCACAGGGGCCCAGCCCGT |
| 248 | cg20063906 | GTGTGCTAGTAAGCGTCTCCTTGGACTGTGGTTCTTTTTGTACCAGTTGGTAGTAGCTTT[CG]TACCAGTTCGTTGATACTTTTGTCACCTGGATTGCTGACTTTCGCTGGTTTCCAGTGCTG |
| 249 | cg12328429 | |
| 249 | | AATAGAAGTTTGCAGGTAACACAGCAGAGCCTCTCACTCTATATTAATGTCTTCTCTCTC[CG]TGGCAAATGTTCTTATTACTACATCTGTCTGAGATCTCCTGTCTTAGGAATTAATGGTTC |
| 250 | cg25969122 | GTTGGAGGAGGGTTGAGGGGGCTGGAGAGAAAATGGAGCAGGAAGAAGGTTTCTGGTGCC[CG]GGGCTGTATTTCCAGGCTCCATGAACCCACTTTGTTCAACAATCGAGGGGGATAAGGTGA |
| 251 | cg18633600 | AGCTTCAGCTGCGCAATAACAGCATCAGGACCCTGGACAGGGACCTGCTGCGGCACTCGC[CG]CTGCTCCGCCACCTGGACCTGTCCATCAACGGCCTGGCCCAGTTGCCCCCTGGTCTTTC |
| 252 | cg08622677 | |

EP 3 802 856 B1

73

| SEQ ID NO | Probe ID | Sequence With CpG Marked By Brackets |
|---|---|---|

TTTGTGCAGAGCTGGGGTGGGTAATCCTGGGGCCAGGTCTGCCCCCTGCAGTGCCTTGAC[CG]TCTCCTGCCGCTGCCTCAGCTTTACCCTTCAAGCTCGAGTCGGTTCCCGAGCTCTCCGTC

253     cg01820374

GGGAGGCTCAGTTCCTGGGCTTGCTGTTTCTGCAGCCGCTTTGGGTGGCTCCAGGTAAAA[CG]GGGATGGCGGGAGGGTTGACCTCCAGCCCCACAGGAGGGGACCAGCAGGGATCTCTGTGG

254     cg25719851

GGGTACCTCCTTCTCTGAGGAACTGGGCTGTTAGGGATTTTCCTTAGGCCCTTTGGTTTC[CG]CCTACGGAGAGGTTTCCCCCATTGGTTGCTCTTCCTCAGCCAGGGTTACTTCCTGGTCTG

255     cg13828440

CGTGAATGAGGCGTCCAAGTGGGAAACCCATCCAGTTCTACTTTTTTGAACTTTGCCTGT[CG]TGGCCAGGATAATTAGGTAGAGATCAGAAGAACAGAGTGAGACATGGAAATCCCAATTTA

256     cg26986871

TACTTTCATTAGTTGAGAAGAGCCAACAATCAACCGGCCTTTTTGGTCAGTAAGCTAACT[CG]CACTGTGGCCTCAGAAAACCCTTCTCTTCTGGTACACAGGAAAGACTTAACACGCAGCCA

257     cg00748589

CCGGTGCGCCGGGCTCTACCTCAAGGAGCTCAGGGCCATCGTGCTGAACCAACAGAGGCT[CG]TCCGCACCCAGCGCCAGAGCATCGACGAGCTGGAGCGGCGGCTGAACGAGCTGAGCGCCT

258     cg21747310

CTATCACTTTCACATCAAACTGGGGGTACTGTCCTTTGAACAGAAGACTCATGAGGAAAG[CG]CAGATTCCTTCCAGGTGGGAAGAAAGCTTTGTCCCTGCTCCATGTCTGCTGATCTGCAGG

259     cg00431549

EP 3 802 856 B1

| SEQ ID NO | Probe ID | Sequence With CpG Marked By Brackets |
|---|---|---|

TAACTGCTGGACCTGACTGTGTTACACAGGATGCTGCTCTGGTGCAGAAGTTTTGGCCAT[CG]TATGCTTGGGGACAGACCTGGGCAAAAGCCCACAGAGGAAGTTGCCACAAACACATGATC

260 cg13302154

AAGGGTTCATCAGGATGGAGATATCCGGTGCACCATGAGTTCTGTTTCCTTAATCAACAC[CG]TTGTAACTTGCCCATCCAGTTTTGTGACATTAATTCAAACCTGTGCCCTAGTCCTCTTTT

261 cg13909661

TACACCAGCCTAAATGTACAGACTTTGTAGCCGAGCCCACTCGATCGGTCTGTGCCTTCA[CG]TGACCACCATCTGTGCCTCCCTCGCTCCATCCAAATTTGTGTAGGCTGCTCCTTGGAGCT

262 cg19722847

TCTGCTTACAGCTGCTTCCAAATTAAGCATATCTGGATGGTGTGACACTTTTTGTTAGTC[CG]AGAACTGTATGGGCATCGCAACTGGGCCTGTTCCAAGATAGACTTGTTGGGACCTTCAAA

263 cg26311454

TTCAGTCTTGAACCAAGAAGACATCAAGGTCTTCAGCAGCCATAATTTTCCTGTGCTTTC[CG]GATTTGAAATCTACGTTTTCTCCTAGGTTAAATCCTCTATTTACATTCTCTGTGCCTACA

264 cg08900043

AATCATCAAGGCCATTTTCAAATCCCATTGGTCTAGCCGTCACATGGTGAGAACCGAATG[CG]CGGATAATTACGGAGCTGATATTTCCCCCCCTCCCCTTCTTTTTCCTCCCTCCCCTCCAA

265 cg00753885

CAGCCATCTCTGGAGGGTTGACCCCAATAAACTTCACATGAAAACAAATCATCCAAAAGA[CG]CAGGTGAAAGTATATACCACTTATACTGAAGTCTTTTTAAAGTAAATCACCATATAGTCA

266 cg18573383

| SEQ ID NO | Probe ID | Sequence With CpG Marked By Brackets |
|---|---|---|

GCCGTGAATGGAGTGGAGACTGGCCGCAGGTCAGGAGAGCTCACCACTTGAAGGTGA
AGT[CG]CCCTGCTCGGATTCCATCTGCAGATTTTGTTTCTCCCCCAAATCAGCCACTGCTGGAG
CT

267 cg15405572

CTGTCAGTAGTGAAAAATAGCTGGAAATCAGACAAACAACTTTATTGCTGAGATTGTT
TC[CG]GGCTAAAAGTTCTTCCAACAGCTGTTTGTTTTGGCCATTAACATGTCCATTCTTTTTATT

268 cg01528542

TGTTACAATTTAACTACTTTCTCTTTCTCTTTCTCTCTCTCTCTCTCTCTGGTAAAAA[
CG]TTAACCTCTGCTAGTGATGACCAAACCTGGTAAAGATTGTAAAGTGGGAAAAATTGGATT

269 cg08993878

TCTGCACTCACTTCCAAATATTATTTGAGACCCAAGTTTCTCATATCATTTCCAGCATT
G[CG]TCATGATTTCAGTGCTTCTTGGCATATTTTGTTTTGGGCTTGAAATATTCTAGCTCATGC

270 cg22827210

TCTGGCCCCATTAGCCAGCAACCAGGGAAATGTAGCTGCAGGAAAATCACCTCGTTT
CCT[CG]GGATGTTTTTTCTTAGGCTGGTTTCCTTTACAAGCTGCAATTATGTTCCATCCCACGC
AA

271 cg03670162

GGGGCCTAAACAGCCACAAACACTGCAGAGATGAGCACCAGACTTAAGTTGGAGATA
CAC[CG]ATTCTCCTGTTTCTGGGGAAGGATTCTCAGAAGGTGGCTCATATGAGTAAAAATCAT
TTT

272 cg04596060

ACTTGGAATGAACATGTTGGAAATAAACGCTCTCATTTTGCAGGCAGATAAACTGGG
AAT[CG]TGCGTGTAAAGCAGCTTGCTCAAAGTCTTATAACTATGAATTGGAAAGTCAGATTCG
AGC

273 cg21907579

| SEQ ID NO | Probe ID | Sequence With CpG Marked By Brackets |
|---|---|---|
| | | AAATATTACTGTTTATTACCAGGCATACCCCAGTAAAATAAAGAGGCAACCAGGCGATAG[CG]ACTATCTCACCAGCCGCTGCACCTATAGGACTTGGAGACGTCACGAGTCACGCAACCGGC |
| 274 | cg10281002 | TTGGGATGCGATAACTCAGTGCCCTCTTGCAGACTTGCATAGAAATAATTACTGGGTTGT[CG]TGGAGGGGACACGAGACAGAGGGAGTTCTCCGTAATGTGCCTTGCGGAGAGAAAGGTCCA |
| 275 | cg07172885 | CAACCGTTGAGCCATTGGTGTCAAGTATTTTAATTCTCTTTAAAATTTAAAACCTGCAAG[CG]CGGGAGCTCAGGGACCTGGCCAGGAAGGCCTGAGCTTCCGGGTCATCTTAGCACGCCCCC |
| 276 | cg20404336 | ACCGTTGAGCCATTGGTGTCAAGTATTTTAATTCTCTTTAAAATTTAAAACCTGCAAGCG[CG]GGAGCTCAGGGACCTGGCCAGGAAGGCCTGAGCTTCCGGGTCATCTTAGCACGCCCCCTC |
| 277 | cg18582260 | TTAAACATAAATCTGCGGTCTGTTCTTAGCACCTGCGGCTGCGTGGGAGGTATGGAAAGG[CG]CACTTTGGGTTTCGTGAAATCTCAAGTCAGGTCTTGACTTCTCTCTTTCCACAGATTCAT |
| 278 | cg22179082 | AACACAGGGTAGGACTTCAAAACACCAGCGTGAGCGAGGCAGGCACACACGGACTCGCGG[CG]GTCTGTTTGCAACAGCGCTGGGAATGCACATTGGAAAATCACATCTTGCATGCTGAAAAC |
| 279 | cg06648759 | |

EP 3 802 856 B1

| SEQ ID NO | Probe ID | Sequence With CpG Marked By Brackets |
|---|---|---|

AGGATTATCTACAAGCAGTAGCTGTAGGACTTGGCACTCTGCCAGCCAGCAAGAACACTC[CG]GTGTCCCTCTCCATGCCAGCTGGGCCAGTGGCTCACACAGGTTCTGGGCAAGCTGTGTGT

280    cg23357533

GGTATGTGAAACAAGAAGTTCTGGGTCCTTTCATCATAAGGGAGAAGCTTCAGAAAGTTC[CG]AGGACCTGCTAAAATCAGCTACTAGAATCTGCTGCCAGAGGGGACAAAGACGTGCACTCA

281    cg20102280

TACATGTTGGCCAAGCATGATTTCAAACCGGAAAGAAAATTACACAGCAATAAAATATAG[CG]GCATGAGAACTTACATTTGTCTTCAGGGTCCACACATGAGATACATTTGTTATTCTGTGA

282    cg13767001

TCTCTCTGTCTAGCTTCTTTGGCTGCTTGCCTGAGATCTTTATCAGTGCAGTCAGCATTG[CG]TCAATGAAGCTTTTGTTATAATTTCTCTTCCATTGCATTTTCAGTTTCTTTAGCCCAGGG

283    cg23389651

CCTAGAGGTCACAAAGAGAGTTGCCCCGCTATCTGCAGGTGCACAGTTCACCAATTAGCA[CG]CTGCTACTGTGGAGACCTCCAGCATCACACAGAGAGCAAGGCCTCCATATTTTCTCCTCT

284    cg09646392

TCACTATTCTTAGTCCACAGGGGAGTAGTGACTACCCAGGGCTTGGTAAGTGCTCAGTAA[CG]TTTGTTGAAAGATGAATCAATATTTCAATGCTGGGGCAAAGCAGTGAAAAACTGGGGAAT

285    cg00593462

TCCTCTGCCATCATTTGATCCTCTACCCGCTAAAAAGCGGGTTTTCCTTCTGGGACTTGG[CG]CAAGCGCTCCTAGGCCAGGCGCGCGCTTAGGTCTGAGACCGGCCGAGGAGCAGGGGCGCC

| SEQ ID NO | Probe ID | Sequence With CpG Marked By Brackets |
|---|---|---|
| 286 | cg07115626 | GAAGGAAAAGCCTTGCACTAGAGCTCTCTATTAGTCCGAGGCTGCGCACCCGGCTTAGAG[CG]CGCTGAGTGTCCGTTGGGGCCCCTGCTCTTGGGGGCGCCTGGGGCTCTGCGCGCCCGCAG |
| 287 | cg06738602 | ACTTCATTGTTTGGTGAGTTGCTTTGCTTTGCTCGTTGCCCCGATCTTCTGTGTATTCTC[CG]CAGACCCCGCAAGTGCTCCTGCACTCCCTCCCAGCCCTCTGCTGGGGCTTAACGCTTCCC |
| 288 | cg03032497 | CACACCACTCGTATCTAACTCAACCCCTTTAGATATTCTTCCAGGTGGAATTATTGGATT[CG]GTCAGAATGGGGGAGGGGCCACTATGCCCTTAAGAGGCTCAGAAGTGCCTACCTGGCTAA |
| 289 | cg18771300 | TGCCGTGGGGAAAACCTGCCTGCTGATGAGCTACGCCAACGACGCCTTCCCAGAGGAATA[CG]TGCCCACTGTGTTTGACCACTATGCAGGTAAGAAAAAGTGGGAAACTCTCTGCATCCAGA |
| 290 | cg24058132 | GGGCCATGAGTGGCCCTACCATGGCTCTTCCCCAGCATCTCAGGGAGTATCTACCTCGTG[CG]AGGACCAGGCTTGGACACCAGGTCCCGATTCCATTGTCATCTTGGTGGAATCACTTTGCT |
| 291 | cg13027206 | TACCCGCAAGAGACGCCCCAACCTTCAGGCTGCTTTATGTCTCCAAAGCTCTGCAGGTGC[CG]CTCTTCCCCAGAGGACACCCCTTCCCTGCCAGTCAACCCCACCAGCCGCAGGCAGACATT |
| 292 | cg15480367 | AAGGGGGCGGCACCGCTGACGTCATTTCCGGGGTCGGGGTATATAAGCGGGGCGCGAGGG[CG]CTGCTGCTGCCACCGCTCCTGCCACTGCAGTGCTCGAGCCCCGTGCAGGGGAGCTTGCGG |

EP 3 802 856 B1

| SEQ ID NO | Probe ID | Sequence With CpG Marked By Brackets |
|---|---|---|
| 293 | cg12177001 | CCTTGCTGGCTCTGTCTGCTGAGGTTTTACCCAAGTGACTCCATTTTGAATCTTACAACT[CG]CACACTACTCATGTGGAAGATTTAAATGTACATTCCAGGACCTGGTGCTTTCTCTTCCGC |
| 294 | cg23709172 | GGACAGCAGAGCACCAGAACGAAAGTGCTCCCAGGCCTGCCAGGGGCTGCCTGAGGGGGC[CG]GGCAGAAGCCCAGCAGGTCTGGCCAATTCATAGCTCAGAGAGCCCAGGCCTCCACGGAGC |
| 295 | cg14334310 | GTCGTGGGGAGCTGGGCTTGCCCTCTGGTGGGTGTCCCTGGCTAGGCCTCTCCCTCCTAA[CG]GCTCCCCACCCGGCCTTCTCCTGACCCCAGACCAACCTCGTGACCCAGGCTTCTTTGAGA |
| 296 | cg04875128 | CGGCGCGCGCCGGGCTGTAGCTCTGCGACGACAGCGAGCGGTTCTGCTGCGGGTACGTGG[CG]CACGGCCGCAGCGCCCCCACGGCCGGCGCGCACGCCTCGTCCCGCGCGCCCGACGCCTGC |
| 297 | cg21296230 | GGTGCGTTGTTCGCGGGGGTGAATTGTGAAGAACCATCGCGGGGTCCTTCCTGCTGAGGC[CG]CGGACACCGTGACCTCGCTGCTCTGGGTCTGCAGGGAAACGTAGGAAAAAAGTTGTCAG |
| 298 | cg02071305 | TGCCTGATGGATAATCCATCACTTGCTTTTCTAGTATGAATGGTCTATTTACGGGTCCAG[CG]CCCCTGCTGGCTTACGACCTTTTCCAGGGCGGGGAGGGGCTGTCCTCATCTCTGTGACCC |
| 299 | cg03361973 | AACACAGATTTACTGTTTTGCTACATATCCAGATAGGAATTTACTTAAGGCTTAGTTTGT[CG]CTTATTTGGATCGTGGTGATGTAGGGTGATTACTCTAGCAAAAAGCAAGAAGGCTGGTAC |

EP 3 802 856 B1

| SEQ ID NO | Probe ID | Sequence With CpG Marked By Brackets |
|---|---|---|
| 300 | cg16717122 | TCTTCCTGTTTTTACTCCTCCTTTTCATTCATAACAAAAGCTACAGCTCCAGGAGCCCAG[CG]CCGGGCTGTGACCCAAGCCGAGCGTGGAAGAATGGGGTTCCTCGGGACCGGCACTTGGAT |
| 301 | cg04858164 | AGATATCCTCAGGAAATTGGAAAAGAGAGGAAAGAGCTTGGGAAAGAGACCTCGTGAAGT[CG]TATACAGACACCTTGGGCTCATGAATCTGATCTTAATTAGCATATTTTAAAAAGACTTTA |
| 302 | cg25005357 | AATTGATGCTGAGTTCAGTATTTGATAGTGTGTTTTCTCAGCATTTTATTGTTGTCACTG[CG]GGGGCTGAAATGAAGTTCTGCTCTACTTGTACCTTGACTGGAATTTGATGTGCAGGGCTG |
| 303 | cg08454546 | ATGTGCACGAGGTTTATGTGTGTGTGTGCGCGCGTGTGTGTGTGTGCGTGTGTGCGCGTG[CG]CTCTCGTCCTCAGCTCAAAGTCTGCGTACGGCAGTGTTGGAAATTATTTCATAGGAGTAA |
| 304 | cg21801378 | CCACGAAGAGCTTGATGGCGTCGTGGTCCTTCATGGGTACGGCGGGACCGGGGTTTAGCC[CG]CTCATGCCGACGCCGCTGTCCGCGGTGCTGAAACCCAGGCGCGGGCCGGGGCCAGCGGGC |
| 305 | cg15188939 | CATACATTTCTTTCATGACACTATTTTTATACAAGATTACTTTAAATCTGTAGCTAACTA[CG]GTCTGCTTGTGGTGAAGTAAAGTGGTTTTAGTCCACAGAGATCTTTTTTGGAAGGTTATT |
| 306 | cg20540209 | TGGCCTCCTTCTCCGCAGGGCTTGCTCTCAGCTGGCGGCCGGTCCCCAAGGGACACTTTC[CG]ACTCGGAGCACGCGGCCCTGGAGCACCAGCTCGCGTGCCTCTTCACCTGCCTCTTCCCGG |

81

EP 3 802 856 B1

| SEQ ID NO | Probe ID | Sequence With CpG Marked By Brackets |
|---|---|---|
| 307 | cg05542681 | CCTCGCGCTACTCAATGACGAGGCAGCGGGGCAGGTGCTGCGAGAAATACTTGAAGAGCT[CG]GGGGTGGCCCCGGGGCAGTTGGTCAGCTCCAGCTCCTCCAGCTCCTGCAGCTGCACCAGG |
| 308 | cg08949164 | CGCGCCACGGGGAGGGCGCGCGCGGCCAGGCGGGGTCCCGAGGCAGCCAAGCCCGCTCCC[CG]TCCCGCAGCCACCTGTGGGTTGACTCACAGCCCCGCATCCCGGGGGAGGGGGCTCCGGCC |
| 309 | cg09183146 | GGCGCACCCGGTCCCCGCGGCTCCTGGCTACGAGCTGGGCGGGCAAGTGGGCGCGGGCAG[CG]GGGGCCAGAGGTCTTCAGGCAGAAAGCCCCAGAGCTGCCCCGCTGCCCGGCTCCTCCTGC |
| 310 | cg00454305 | GCAAGTGGGCGCGGGCAGCGGGGGCCAGAGGTCTTCAGGCAGAAAGCCCCAGAGCTGCCC[CG]CTGCCCGGCTCCTCCTGCCCTGCCCACCTGCACCTGCAGCTGCTCCGGGCGGACTCAGGT |
| 311 | cg02871659 | CCAAGCGCTGCTTCTCTTTCAGTTCTTTCGAAATGAATTCGCTGCGAATGTGGGAAGATG[CG]CTGAAATGCCTTTTGTGGCTCTGGCTTCGCTCAGGTATCCATCCAACCTCTAAGTGGAAT |
| 312 | cg00991848 | TCCTCCAACCCCAGCCCAAATGACTCCGGGGTCGCACTTGCTCAACGCCCCAACGACCGA[CG]CGTACCTTAATAGGCAGGGAGAAGAGATAGATCTCCTCCAGGGACTTGATCTTCATGTCC |
| 313 | cg02331561 | |

| SEQ ID NO | Probe ID | Sequence With CpG Marked By Brackets |
|---|---|---|

CAGCGGCGGTAGCCGAGCGAGGGCGCGGTGGCCTCTGACAGGAATGACTCTGCGCAC GTG[CG]TTTCGCAGCAGTGGAAGTCTTCACACCCGGAAACTCGACTTTGGCCGTTTCTCCATTT CT

| 314 | cg26974444 | |

GAATCTCTGTCACTGGGGCAAGAAATTACAGCTGTGAACCTGCTGGTTAGTGTTCTGT GC[CG]AGGCCTTGAACTTATGATTAACGTGGTTGACGTTCTGTCCAGCTCATCCCATGCTCAG T

| 315 | cg06112560 | |

TTGATTGAACAAAAGTTTGATTCAGAACTCCTTTTAAACAGGTGAAAGCTTCAGTAA TA[CG]AGGACAGCATTTTCTTAGGGCAGCCCTTTGCTGGGCTGGAAAACAGCTGTCCCCTTCA GA

| 316 | cg10917602 | |

AAGAGGGCCCCTCCAGGCCAGTCTGGGCACCCTGGGATAGCGGCTGCAGGTAGGCAG AGG[CG]CTGCCAGTGCCCAGGTGGCCTTTCCCTCCATCCGGCCCTTCCCACCTTCCTATAACCT TC

| 317 | cg09155044 | |

GTTGTAAGAATTGCAGCATCCGGGACCTAGAGACCAGCGGATCAGGGGATCCAGCGA ATA[CG]GCGATCCGATTCGGGAACCAAGCATTTCCCCTGAAACTATTTCAGGCACCATTCGGG CTG

| 318 | cg04031656 | |

TGACACTTTCACAGTCATATATGGATGCACGTAGAATAGTGAAAAAGTTGAGTCACC CAA[CG]TGCACATTTCCAGCCAAGGGCAAACAGGAGGACACGCTGCTTTCTGGTTTCAGCTCT TGT

| 319 | cg03746976 | |

TATCGCCTGGCACACATCCCTGTACCATCCTAGGTCCGTTTCCTGATTTGAATACAATT G[CG]ATAGTAATACTTGCTAAAGCGTAGGGAGAGCCTTTTCATTCATCCAGTAAATATTCACT G

EP 3 802 856 B1

| SEQ ID NO | Probe ID | Sequence With CpG Marked By Brackets |
|---|---|---|

320 cg18693704

GACCCAGGCGACTGACATGTTCCTCTCCTCTCAGCTGAAAAGCTTTGCTAGCTCTGTC
TA[CG]CATAAAGTAAGGTTAAACACAGATTTTGCCCCGAAGGGCATTAATTAGGGACCAATTT
AC

321 cg00658652

AGAATAAGATATAATATTCGACATCATTTTAAATACTTAACTCACAGGAAAGTGTACC
CA[CG]TACCAAAACCAAACAAGAAACTAAAACCAGAGGCATTGGTTTGGACTGAGGACCTCA
GCT

322 cg03991512

AGTTGCCACAGGGTAAGCCCAGTGCCCTTTTGCCCAAGGTCAGGTCACTTGGTGCTGG
GG[CG]TCACAGAGCCCAGGAAACTTGGGATCAGAACCCCCTGCTCCCCGCTCCCCACCTCATC
CC

323 cg22947000

TAGCTATGACACATGGCTTGGAAATTAACCTTTAACCAAACATCTTATAAGTAACGCC
AG[CG]CAGCTTCCCTTGTGAATGTAAAGAGATCCAGGGCTCTTGGAGAGGGACAAGTGAGAG
CCA

324 cg07082267

GCTCCTCATGTGAGAAGGACCATAGGAATCTCCCGTTTCACAGGTGGGCACACCAAG
GCC[CG]ACAATGGGTCCAGGCTGCCAAGGGTGGAGCCGAGATGCAAAGGGGCACCTCAGAGC
CTGC

325 cg03486383

AGGGGGTCCCGGTGCCCAGGCGGGGGCGGCAGGCTCCACTGGGCACTTGCTGAGAGC
TTG[CG]GCTTGAGCAGCCGCTGGTCAGTGAAGCCGTGTCCGGCTTCGTTGGCTCCCGGCTTGG
GAC

326 cg02228185

AGGAACCCATGGGAATGAGCTAACCGGAGTATTTCTGGTTAAGCATTGGCTAGAGAA

EP 3 802 856 B1

| SEQ ID NO | Probe ID | Sequence With CpG Marked By Brackets |
|---|---|---|
| | | TGG[CG]CTGAGATTCAGAGAACAGGGCTGGAGGTAAAACCATTTATTACTAACCCCAGAGCAGTGA |
| 327 | cg23668631 | AGAGGGAACTCAGCAGGACAGTGAGGTGACCTTCGCTGTGGCTGTTCCTGGGGACTCTGC[CG]CCACCTCTTCCCCTAACGCCTCCGCGTGTGAATCCTCTGGCACCACCACTTGCCCCATAT |
| 328 | cg14522800 | CCAGTCCCTCCGAGTGCCAGCCTTCTTCACCGAGAGCAGCGAGTACAGCTGTGTGATGGA[CG]GCCAGACCATGGCGGTGGCCACTGGAGGGGGCACCAGCCCTCCCCAGCCCAACCCCTTCC |
| 329 | cg25135555 | AAACCCCTTTCCCACGTATATAGGTTTGGCATTTGCTGAGTAGGAGCAGCTGTACGACTC[CG]GGAATCTGGGAGAGTTAGCTCAGCCTGCTGACTCAGAAACTCCGGGGTCTCTAAGGACAT |
| 330 | cg13029847 | GGCCCCTGCCCTTCTGCGCTGCCCACCCCCAGCCAAGCATGCCACCCTCTTTCCCGTTAA[CG]GCCTGCTAAGGAACCTCAATTAATAGCTCACTGTAGCCTTCTGATTCTCCATGAGAAAGA |
| 331 | cg06144905 | CTGACCTCACCACCCACCAGGGAGGTGGGTCTTATTCTGGGCATCGTGCCAAGTTCTTAG[CG]GGGCCCTCTAGAATCTCTAAAGCAAATCAGGCTGAAGAGGGGAAAACCAGCAGGGGGAGG |
| 332 | cg11896923 | TCGTCGGGGAGTGAAAGCAGGCGCAGGGAAATAAAAAGAAGGAAAGGGAGACAGACCAGG[CG]CCTAACAGATGGGGACCAAGAAACAAGAGATAGCTGAGAGGTGCAAACAGAAGAGAAAAA |
| 333 | cg06874016 | |

(continued)

| SEQ ID NO | Probe ID | Sequence With CpG Marked By Brackets |
|---|---|---|
|  |  | CAGCCTCTCAGGAGCTGACAGGTCCTCTTTCGGGGCTCAGGAGGGTGGGCACACACCCAG[CG]GCCTGCAGAGTAAGCTTATTACCCACAACTGTGCCCGCTTTGTGCTTCTAAGGTGCACAC |
| 334 | cg25809905 | ACTTGATTCTGGTTGGGGGCTTTGCCTAGGGGAGCCTTCCCTGACTCCTCAGGCTGGCCG[CG]TGGGCTAACACACGTAGGCACAGCATTGAGCACACTGTTTACTCTTGGTCCGTTCACAGG |
| 335 | cg04267101 | TCAGCTTCCTCCTTGGTCAACCTTGACTCGTTGGTCAAGGCACCCCAGGTTGCAAAGACC[CG]GAACCCCTTCCTGACAGGTAAGATATGCCCTTGTCCCTCAACCCAGGGGCTCCTGCTTC |
| 336 | cg22507023 | CCACCCCCACAGAGGCTTAGCAAGGGCCTCTCTGTGCAGTCAGCTCCGGCCAAGCCTCCT[CG]AGCCACAGAGAACGTGAACATGAGGATTGCGGTGAGGGCATGTGTGGCACGTTATTTTTC |
| 337 | cg02867102 | CTCCTGGAGTGGGTGCTCCTGGGATGCTTCAGGTTTAGACACCGGGGTTACGGCAGCTGC[CG]AGGAAGGCTAAAGCCAGCGTCCTGGATTCAGACAGACCTTTTAGCCATTAAATCCACTAA |
| 338 | cg13093111 | ATTCATGAACATTTACCAGATTCTCCAAAGGGCGTGGGTTGCCAAATGCTTAGGAACTTC[CG]CTTTCAGTGTTTAGCAGGTTAGCGGGGAAATATTAGTCCCATTATTTAAGCTGAGATTTG |
| 339 | cg13683374 | GCTGCATTCCCAGAGGACAGGCCATGGGAGGTGATTCCAGGGGGGCCTGAGCTCTCCTCC[CG]AGACCGTCCACGGGGCATCCATGTCCCGGGTACCTGCTTGTGGGCTGCTCTGGTCACTCT |

| SEQ ID NO | Probe ID | Sequence With CpG Marked By Brackets |
|---|---|---|
| 340 | cg10644544 | GCATCTGCAAACAGCCGGCTCACCCTCTCCCGCTCTGCTACGGCTGCACTCTGCACAGGA[CG]ACAGTAGAGGGGGCAATGAGGGCAAAGAACCGTCCCGGACTGTACCCCCCTCCTTCCCTG |
| 341 | cg03643998 | CAATTGATCGCTGCGTGTGTTTCCAGGACTGTCATTGCCTTTAACAGAGGGCAGGGGGCT[CG]TTCGGTAGTGAGGATCCCAGAGTGGGCCGTGAGCCCACCAGCGTGAACACAGAGCCTTGT |
| 342 | cg11620135 | GCCGCAGGTGTCTGAAGGGTCGATCCACTCTGGAAAGGCTTTGCCCTGGTGACAGGCTTG[CG]CTGCTGCTGCTGCACACAGGCGGGGTTGGTTTTGTAGGTAGGTGGCTCCTGCTGCCACTC |
| 343 | cg25436157 | CAAAAAGTGCCAGTGTTCTCTGCCTGTAGAAACACACAGATTTTCAGAAACACCCACACA[CG]GGGAAACCATCACTCTTAAGCCACAGCAAAAGTCCTCCTGGCTCTCGGTGCTGGAGCAGT |
| 344 | cg24217948 | CCCCCACCTCCTGCCAACTATCCACACCTTTCCCCTTAAAGTTTAGTTGGAGTCCACTGG[CG]TTGATTGTTTTTCTCTTACATCTTTTTCCTTCTTTTTCTTTTCATTCCTTCACTCTTCAT |
| 345 | cg17589341 | CCAGGGGACCAGTTCCTTGGTGTTGCTTTGGCATTGATGCCTGAAGTGGGAGGAGAAAGC[CG]AGCCCACAAACACACAGAGCAGAGTGGGGCTCTGAGTATATAACTGTTAGGTGCCTCCCT |
| 346 | cg17243289 | TGGGCGCGCCCTATGCAAATGAGCGGGCGGGGCCCTCGTGTTGCTGAACGAGGGCGGGTT[CG]CGATGTAAATAAGCCCAGAGGTGGGGTCTTTGGAGAGCACTTAGGGCCCGGGTAGGGGAT |

EP 3 802 856 B1

(continued)

| SEQ ID NO | Probe ID | Sequence With CpG Marked By Brackets |
|---|---|---|
| 347 | cg12459502 | ATTTTTCTTTCTTTCTTTAAAATCTGAAACGGGAGCTTCCGCATTTATTATTTGCA[CG]TGGTTTTGAGGCGCACTCCTGGCCACATCACAGCTATGTTCTCTTGCCTCTGGGAAATAC |
| 348 | cg19283806 | TCCGTAGTATTGTCTCTGGCTTTGAACGCTGTTGAGGGAGGGAATGTTTGCACTCAT CC[CG]CATCCTTTTTGGCTGCTATCTTTGCGGGGATTGTTCAAGGAGAAATCCATCCTGACTG G |
| 349 | cg10052840 | CTGTTGACCCGCAGGACTCGCTGGATGTTGAGGTCGTCAGCACCTTCTGCGGGGGTCA GG[CG]TCCGGCGGCCCGCGCTGCCCACAAACACGGGATAGTGTTCAGGTCTGAGTGAGGGGGTGG AGA |
| 350 | cg26005082 | AGCTCTCCACCGACCGAAGGAGGAGGAGAATGCTATTTATTTCAGCACCAAATATCCGGA CAG[CG]CCTCTCTCGGGAGGTCCGAGAAGAGAACCGCGATCTGTTCAGCACCGGGGCTCAGGA CAGT |
| 351 | cg11766468 | CCACTCTCTGGGCCTCCCCCTGCGGGAGGCCTTGGGTGGGAGCCGAGGG TCA[CG]GCCTCCCCCTGCCCCCCTGTCCTCGCTGTTCTCAGGGGCAAGTGACACGGGCGCAGGA GGC |
| 352 | cg10586358 | ACTCCGTTCCGGCCACGCGCCATGTGTGGAAATCAGACCCGTCAGTGCGTCAGTCAG GGC[CG]GGTTCAGTCAGTCAGGAAATTTGAGGCCAGGCCTGATGAGAGGGAGCCCCAATGGC AAAG |
| 353 | cg14556683 | |

| SEQ ID NO | Probe ID | Sequence With CpG Marked By Brackets |
|---|---|---|

ACCAGCGCCACCGAGAACACCAGGCTCCACATGAAGGCGCGCAGCAGCTTCAGCGAC AGG[CG]CGACGGCGCCAGCAGCGCGGTCACCACCAGCTCCGGCATGTCGCCGCGCTCCGGGA CCAC

| 354 | cg26842024 | |

CGACGACGACCTCAACAGCGTGCTGGACTTCATCCTGTCCATGGGGCTGGATGGCCTG GG[CG]CCGAGGCCGCCCCGGAGCCGCCGCCGCCGCCCCCGCCGCCTGCGTTCTATTACCCCGA AC

| 355 | cg18335931 | |

GGAAGGGGGGAGGACGCCTGTGGATCGAGGTGTCCCCTGGGGTCCCTGGCACCCTCC TTT[CG]CCCCTCGTTCCCTGGACTGGGGTGTCTGTCCGCCAGCGTCGCAGCTGGGGTGGTGAC AGA

| 356 | cg17861230 | |

GCGGACTTGTCCGGATCCGAATAGAAGCGCTGTTGGATGCGGATGGGGCGCCGGGGT TGC[CG]CCACAGGTGCTTCGGGGCTCTGGTCATGCTGTGGCGGCCGCGAGAGCGACTCAACCT GCT

| 357 | cg10498798 | |

GGTGGCCGGCGGGGCCCTCCTCACGCTGCTGCTCTGCGTAGGACCCTACAACGCCTCC AA[CG]TGGCCAGCTTCCTGTACCCCAATCTAGGAGGCTCCTGGCGGAAGCTGGGGCTCATCAC GG

| 358 | cg27212234 | |

ATCACCAGCAAGTGTCGCGGGTCCCGCGGGTCCTCCAGCGTATGGATGGACAGCTGT GGG[CG]GGGGGGAGAGGCGAGGCTGTGGACGGGGGAACGGGGCGGGGCTGTGGACGAGGGA ACGGG

| 359 | cg17110586 | |

EP 3 802 856 B1

89

| SEQ ID NO | Probe ID | Sequence With CpG Marked By Brackets |
|---|---|---|
| | | CCCAAGCCTGCTTGGGCTGCTGGGAAACAGGCATGTTGTCTCAGAGGGCACCGCGCTCGG[CG]AAGACTCAGCGAGACTGGACGCTGACCATGGTTCTGAACACACTGTGCTGCGGGACCTGG |
| 360 | cg21944491 | AATGTTAGGCGGAGCGGGAGGTGGGCCGGGCCTTCGGACGCCCTGTCCCGCAGACGTTGA[CG]AGTGCAGCGAGGAGGACCTTTGCCAGAGCGGCATCTGTACCAACACCGACGGCTCCTTCG |
| 361 | cg21940708 | TGGGGAGGGCACATCGTGACTGTGTTTTTCATAACTTATGTTTTTATATGGTTGCATTTA[CG]CCAATAAATCCTCAGCTGGGGTCTGGCTTTGTTTCCTGGGGGCAAAGGAGGTTTGGGGTT |
| 362 | cg15811427 | AGAGGGCAGGGCTGTATTCCGCTACTGGGTCCTATGCACCATGCAGAACCAGTGTCTTCA[CG]TGGAGACTCATCACTGATCCGAAAGGTGACTGCTTCTGTATTACACTCATTTCCCCATGA |
| 363 | cg06458239 | TGACCCTAGTTTGATGGGTTTTTTCCTTTGTCCTCTCTTTCTTGGATTGAGTCCTCACAC[CG]CGGCGGACTGCGGCGTGGTAGGAACTACACCACCCAGAATACTGTGCGCCGAGCGTGCCG |
| 364 | cg10729426 | GATGGGTTTTTTCCTTTGTCCTCTCTTTCTTGGATTGAGTCCTCACAGCGCGGCGGACTG[CG]GCGTGGTAGGAACTACACCACCCAGAATACTGTGCGCCGAGCGTGCCGGGGCCTTAGACC |
| 365 | cg21911021 | |

EP 3 802 856 B1

| SEQ ID NO | Probe ID | Sequence With CpG Marked By Brackets |
|---|---|---|
| | | AGGGCTCAGGTCAGAGCAGGAGCAGCCGGGGGCGCGGCCCCCACGTGGCCTCCCGGGACA[CG]TGCCCACAGCGCGACACCTAAGTCGCTCCTTTCACAGAATAGCCTTGGCCCCGGCACGGC |
| 366 | cg24834740 | GGGATGAGGATGGGGCGGGGAGGTGGTCCCAGCCTGCTATCACCTAGCTGGGGGCCGGGG[CG]CTTTGGCCAAGGGACGATAGCTTGAGATAAATGGGAGTGTGGGGACTCTGGAAAGACGGG |
| 367 | cg19702785 | CTCGTCGAGCACGTGCAGGTGGCCAGTGCGGTAGAAGTGCAGCAGGCTCAGGAAGAAGCC[CG]GGTGCCGGTCGAAGTAGAATTCGCGCGCCGCCTCGTCGTAGTCGTCGCACAGGCGCCGCG |
| 368 | cg07547549 | TTGCAGCCTGGAGCTCAGCTCCATTGGAATGCTCCGGGCGCTGTCCAAGGTGCTGGAATG[CG]CCGCGCCCGGGGGCAGAGCTGCGGGCCGGGGGATTATCGCTGCCCACGGCTTCGGGCTGA |
| 369 | cg12303084 | AAACTTGGGAAAGGGGCCCCCACACGCACTTCTCCTGCACCCTGGCTAGATTTCCCGGCA[CG]GGCCAGCCAGGGCAGCCAGCCTGACCTGCTCCAGGAAAGCCTGAGGCCCGGAGGTCCCTG |
| 370 | cg27544190 | GAACCCTCGACTGGGGGCAGCCGCACCAGTGGACACGGCGGGGTAGGATTAAAGTTGAGG[CG]TGCTCACAGACACTTGTCTGGTGTGAGCCCTTGGCATATAGATGGCTGCGAGTGAAGTGG |
| 371 | cg01262913 | |

EP 3 802 856 B1

| SEQ ID NO | Probe ID | Sequence With CpG Marked By Brackets |
|---|---|---|
| | | GTTCCAAGAAATCTGCCACCAGCTCCAAGCCTCATGTCCTGAAGTGCCACCTCATTCC CG[CG]GGGTGAGCCAGCAGCCTCTGAAAAGAGGAAGCCATTGAACAGATCACACTGTGCCTC CCG |
| 372 | cg17274064 | AAAATAATAATTAAAACTCCCTCAACTTTTAAGGCCGAGCAACATAATCTATTAATTG GT[CG]CTATTAACATGCAGTTTTATTGACCATAGCACACAGAAGTCTGATTGTGAGGGAGGAG TG |
| 373 | cg09428349 | TTCAGATCTCACTGTGCCCTTTCACTTTCCTTTTCAATTAAGCTTCCTGTACAGCTGCC T[CG]GCTCCTTCTCTTAGAACACTCTAGAGAACTGGAAATCATGTAATTACTTTTGTCTCCAAA |
| 374 | cg10636297 | CCTCTGGCCTGTGGCTCACTGCATGCAGCCCCTGGCGTGCAATACTAGTGCTCCACGG CG[CG]ATGTGCTTCTAGCCCTTGCACTGCACCTAGGCTCAGGGTTCAAACGGCCAGCCCGAAA AG |
| 375 | cg12373771 | TGGCGATCCAGGAGCACCAGTACAGGTCGGTGACGGCGATGAGGTACAGGTCCAGCA GGC[CG]CCCTGCGCCAGCAGCAGCACCACGGACAGCGCCTGGTAGCCCCAGCGGCACCTGGG ACTG |
| 376 | cg05442902 | GCCAGGTCACCCTCTCACTCTGTGCCTCTTAGTTATCTTGCATGCTCTGGTCTTTGCAT A[CG]CTGCTCCCTGCACCAGGAACCTCCATCCCCATCTTTGTCTGCTTGTCGAACTTCAGAAAT |
| 377 | cg16612562 | GCCGCCCGGGGTCCGAATTGGGGGGGGGCGGCTGTGTGACCTTGGGCGAATCGCCGCA CTG[CG]CTGGGTCTGCGCTCCGCATCCATCACAGGCAGACTCCTCAAGAGGCTCCAACCTTTT CTT |
| 378 | cg19015086 | |

| SEQ ID NO | Probe ID | Sequence With CpG Marked By Brackets |
|---|---|---|

TGTCACCAGAGTCACACCACCTCCTTTTTATCAGCTATAAAACAGGACTACTGCTGAA
CT[CG]TAGAGTTGGGGGAAAGAGTGAGATAACATATAGATTACCAACCCAGTGCTGCGACAC
ACA

379   cg21205978

CAGAGTTATTAGCCCTTTAATGCTGTGCACCTCATAGGGTTGTTACCCACATCAGCGT
CA[CG]TAAGATGCTGTGGAGGAAAGCAGTTTCAGAACAATCAGTGATGACAGCTACTGTGAA
TCC

380   cg01949403

TCCTCCCCACAAACCCCATAAAAGCACCTTAAACCCTGTAAAGAGGGGCTTATTTCAC
TT[CG]CAGAAATCATTCCGCTCTCCCTCTGAGAGTATATTACTGTGCTTCAATACACTTTGCCT
T

381   cg08415592

AGTATGTCAGTGGCAGGTCTTTCTCCTTGAGACCACAGCAGACCCCCAGCCCTGAGGA
TG[CG]AGGCAGGTGGGTTGGATGAGAGGGATCTGGATGTCTGGTCTCAGGCTGCTCCTCTAAG
GG

382   cg19853760

AAAAGGGTGGGAGCGTCCGGGGGCCCATCTCTCTCGGGTGGAGTCTTCTGACAGCTG
GTG[CG]CCTGCCCGGGAACATCCTCCTGGACTCAATCATGGCTTGTGTGAGTGTGGGGACCCC
CCC

383   cg23124451

TCAGTCTCCCCATATTTACAATAAAAGGGGAGCGAGGTGGGATGGCGCTGAGGATCC
CTA[CG]TCCGATCCTAATCTCCAGCTCAGGCAGGCTCGGCCGCCACTAGCATCCTGGAGCGAC
AAC

384   cg25459323

GAGGGATGGTTGTCCTCACCCCTGTGAGGCAATATGCTGTCCATTAGTATCCACTGAA
TG[CG]TGAAATTTTTTTCTAATGGGCAAACTGAGGCTCAGAGAAGTTCCTGTCTGGCTCAAGG
TT

EP 3 802 856 B1

| SEQ ID NO | Probe ID | Sequence With CpG Marked By Brackets |
|---|---|---|
| 385 | cg27187881 | CCTGTCTTCAGCAGCATCGCTCTGGACTCAGCTTCCGAGGACCTGACCAGATCTGGTCTG[CG]TGTATCAGCTGTATGTGTTGGGCTCTGGAAGCTAAGAAACGTCTGAAAAGCACTGGGGTC |
| 386 | cg00343092 | CGGTCCCAGGAGTGGCCGACGCTCCCTCTCCTGCCCATTCCGCGGATGGGCAATCCCAGG[CG]GAACTCCCTTGAGGGTCTCAGAATATCTGGGAGACCTCGGGCTCTTGATCTCCGAGACAC |
| 387 | cg00347775 | TAAACACAACTCCTCGAGCAGCATACTCATTTGGAGAGAGCTGCTGTTGAAATGTCATTG[CG]TTGTTTTTAAGAGTTTTGAGCCTGGTAAAACCATTCACCTGGGGAGGCAACGTGTAGTGG |
| 388 | cg27131176 | CGAGCCGCGGCCACAGGGCCAGCCGCACAGTCGGAGGAAGGGCCGGAGCGAGGCGGGGCC[CG]GGGCTGTCAAGGAGAAAAACATCCCAAGGCCTGCAAATTGCTGCTCTCAGCTTTTTTCCC |
| 389 | cg22982767 | AATGGAAAAAAATTTAAAAGATTGGGGACAACAGGAAACACATTGGATCCCCAGGGGAAA[CG]GCCTGGAAGCTACAGTAGAGACATGGGTGACCCAAGGGCTCTGTTCAAGTCCTGGGGCTG |
| 390 | cg07016730 | AGCCTGAGTGCCAGTCCCAGCCTCTCTGAGCCGGCTCAGGCCAGGCAGTCAGCTTATCCG[CG]CCTAACTTCTCTACAGATGGGGGCAACACCAGGGCAACCCCGGTGGGCTGCTGGGAGGAA |
| 391 | cg01892695 | |

EP 3 802 856 B1

94

| SEQ ID NO | Probe ID | Sequence With CpG Marked By Brackets |
|---|---|---|
| | | TTTAGTTCAAACCTAGGCCTGGGTTTGGGTACAAACCCAGACCAAAGGGGCATCTAA TCC[CG]TTTAAGGCAATTTAAGAAGTATTTCCCTAGGCCACTAGATAAATGTATTCTTTAAAGT AT |

EP 3 802 856 B1

All publications mentioned herein are to disclose and describe the methods and/or materials in connection with which the publications are cited (e.g. U.S. Patent Publication 20150259742). Publications cited herein are cited for their disclosure prior to the filing date of the present application. Nothing here is to be construed as an admission that the inventors are not entitled to antedate the publications by virtue of an earlier priority date or prior date of invention. Further, the actual publication dates may be different from those shown and require independent verification.

CONCLUSION

This concludes the description of the preferred embodiment of the present invention. The foregoing description of one or more embodiments of the invention has been presented for the purposes of illustration and description. It is not intended to be exhaustive or to limit the invention to the precise form disclosed. Many modifications and variations are possible in light of the above teaching. Nonetheless, the appended claims define the scope of the invention.

**Claims**

1.   A method of observing biomarkers in human skin and/or blood cells that correlate with an age of an individual, the method comprising:

   (a) obtaining genomic DNA from human skin cells and/or blood cells that have been derived from the individual;
   (b) observing the individual's genomic DNA cytosine methylation status in all 391 methylation markers of SEQ ID NO: 1-SEQ ID NO: 391;
   wherein said observing comprises performing a bisulfite conversion process on the genomic DNA so that cytosine residues in the genomic DNA are transformed to uracil, while 5-methylcytosine residues in the genomic DNA are not transformed to uracil;
   (c) comparing the CG locus methylation observed in (b) to the CG locus methylation observed in genomic DNA from human skin and/or blood cells derived from a group of individuals of known ages; and
   (d) correlating the CG locus methylation observed in (b) with the CG locus methylation and known ages in the group of individuals;

   so that biomarkers in human skin and/or blood cells that correlate with an age of an individual are observed.

2.   The method of claim 1, further comprising using the observations to estimate the age of the individual; and, optionally,

   comparing the estimated age with the actual age of the individual so as to obtain information on life expectancy of the individual; or
   the estimate of the age of the individual comprises use of a regression analysis.

3.   The method of claim 1, wherein the skin and blood cells are human fibroblasts, keratinocytes, buccal cells, endothelial cells, lymphoblastoid cells, and/or cells obtained from blood skin, dermis, epidermis or saliva.

4.   The method of claim 3, wherein the age of the individual is estimated using a weighted average of methylation markers within the set of 391 methylation markers.

5.   The method of claim 1, wherein methylation is observed by a process comprising hybridizing genomic DNA obtained from the individual with 391 complementary sequences couple to a substrate and disposed in an array; or wherein methylation is observed in at least 100, 200 or 300 methylation markers.

6.   A tangible computer-readable medium comprising computer-readable code that, when executed by a computer, causes the computer to perform operations comprising:

   a) receiving information corresponding to methylation levels of a set of methylation markers in a biological sample, wherein the set of methylation markers comprises 391 methylation markers of SEQ ID NO: 1-SEQ ID NO: 391;
   b) determining an epigenetic age by applying a statistical prediction algorithm to methylation data obtained from the set of methylation markers; and
   c) determining an epigenetic age using a weighted average of the methylation levels of the 391 methylation markers.

7. A method of observing the effects of a test agent on genomic methylation associated epigenetic aging of human cells, the method comprising:

(a) combining the test agent with human cells that have been isolated;
(b) observing methylation status in all 391 methylation markers of SEQ ID NO: 1-SEQ ID NO: 391 in genomic DNA from the human cells;
(c) comparing the observations from (b) with observations of the methylation status in at least 10 of the 391 methylation markers of SEQ ID NO: 1-SEQ ID NO: 391 in genomic DNA from control human cells not exposed to the test agent such that effects of the test agent on genomic methylation associated epigenetic aging in the human cells is observed.

8. The method of claim 7, wherein a plurality of test agents are combined with the human cells.

9. The method of claim 7, wherein the test agent is an inhibitor of cellular senescence.

10. The method of claim 7, wherein the cells are primary keratinocytes from multiple donors.

11. The method of claim 7, wherein the method observes human cells *in vitro.*

12. The method of claim 11, wherein the human cells differentiate *in vitro.*

13. The method of claim 8, wherein the test agent is a compound having a molecular weight less than 3,000, 2,000, 1,000 or 500 g/mol; or

a polypeptide; or
a polynucleotide.


**Patentansprüche**

1. Ein Verfahren zum Beobachten von Biomarkem in menschlichen Haut- und/oder Blutzellen, die mit einem Alter eines Individuums korrelieren, wobei das Verfahren Folgendes beinhaltet:

(a) Erhalten genomischer DNA aus menschlichen Hautzellen und/oder Blutzellen, die von dem Individuum stammen;
(b) Beobachten des Methylierungsstatus des Cytosins der genomischen DNA des Individuums in allen 391 Methylierungsmarkem der SEQ ID NO: 1 bis SEQ ID NO: 391;
wobei das Beobachten das Durchführen eines Bisulfit-Konversionsprozesses an der genomischen DNA beinhaltet, sodass Cytosinreste in der genomischen DNA zu Uracil umgewandelt werden, während 5-Methylcytosinreste in der genomischen DNA nicht zu Uracil umgewandelt werden;
(c) Vergleichen der in (b) beobachteten CG-Locusmethylierung mit der CG-Locusmethylierung, die in genomischer DNA aus menschlichen Haut- und/oder Blutzellen, die von einer Gruppe von Individuen bekannten Alters stammen, beobachtet wurde; und
(d) Korrelieren der in (b) beobachteten CG-Locusmethylierung mit der CG-Locusmethylierung und bekannten Altem in der Gruppe von Individuen;

sodass Biomarker in menschlichen Haut- und/oder Blutzellen beobachtet werden, die mit einem Alter eines Individuums korrelieren.

2. Verfahren gemäß Anspruch 1, femer beinhaltend das Verwenden der Beobachtungen zum Schätzen des Alters des Individuums; und optional

das Vergleichen des geschätzten Alters mit dem tatsächlichen Alter des Individuums, um Informationen über die Lebenserwartung des Individuums zu erhalten; oder
die Schätzung des Alters des Individuums beinhaltet die Verwendung einer Regressionsanalyse.

3. Verfahren gemäß Anspruch 1, wobei die Haut- und Blutzellen menschliche Fibroblasten, Keratinozyten, Bukkalzellen, Endothelzellen, lymphoblastoide Zellen und/oder aus Blut, Haut, Dermis, Epidermis oder Speichel erhaltene Zellen sind.

4. Verfahren gemäß Anspruch 3, wobei das Alter des Individuums unter Verwendung eines gewichteten Mittelwerts von Methylierungsmarkem innerhalb des Satzes von 391 Methylierungsmarkem geschätzt wird.

5. Verfahren gemäß Anspruch 1, wobei Methylierung durch einen Prozess beobachtet wird, der das Hybridisieren genomischer DNA, die von dem Individuum erhalten wurde, mit 391 komplementären Sequenzen, die an ein Substrat gekoppelt und in einem Array angeordnet sind, beinhaltet; oder
wobei Methylierung in mindestens 100, 200 oder 300 Methylierungsmarkern beobachtet wird.

6. Ein physisches computerlesbares Medium, das computerlesbaren Code beinhaltet, der bei Ausführung durch einen Computer bewirkt, dass der Computer Vorgänge durchführt, die Folgendes beinhalten:

   a) Empfangen von Informationen, die Methylierungsgraden eines Satzes Methylierungsmarker in einer biologischen Probe entsprechen, wobei der Satz Methylierungsmarker 391 Methylierungsmarker der SEQ ID NO: 1 bis SEQ ID NO: 391 beinhaltet;
   b) Bestimmen eines epigenetischen Alters durch Anwenden eines statistischen Vorhersagealgorithmus auf Methylierungsdaten, die aus dem Satz Methylierungsmarker erhalten wurden; und
   c) Bestimmen eines epigenetischen Alters unter Verwendung eines gewichteten Mittelwerts der Methylierungsgrade der 391 Methylierungsmarker.

7. Ein Verfahren zum Beobachten der Wirkungen eines Testmittels auf mit genomischer Methylierung assoziierte epigenetische Alterung menschlicher Zellen, wobei das Verfahren Folgendes beinhaltet:

   (a) Vereinen des Testmittels mit menschlichen Zellen, die isoliert wurden;
   (b) Beobachten des Methylierungsstatus in allen 391 Methylierungsmarkern der SEQ ID NO: 1 bis SEQ ID NO: 391 in genomischer DNA aus den menschlichen Zellen;
   (c) Vergleichen der Beobachtungen von (b) mit Beobachtungen des Methylierungsstatus in mindestens 10 der 391 Methylierungsmarker der SEQ ID NO: 1 bis SEQ ID NO: 391 in genomischer DNA aus menschlichen Kontrollzellen, die dem Testmittel nicht ausgesetzt waren, sodass Wirkungen des Testmittels auf mit genomischer Methylierung assoziierte epigenetische Alterung in den menschlichen Zellen beobachtet werden.

8. Verfahren gemäß Anspruch 7, wobei eine Vielzahl von Testmitteln mit den menschlichen Zellen vereint werden.

9. Verfahren gemäß Anspruch 7, wobei das Testmittel ein Hemmer von Zellseneszenz ist.

10. Verfahren gemäß Anspruch 7, wobei die Zellen primäre Keratinozyten von mehreren Spendern sind.

11. Verfahren gemäß Anspruch 7, wobei das Verfahren menschliche Zellen in vitro beobachtet.

12. Verfahren gemäß Anspruch 11, wobei sich die menschlichen Zellen in vitro differenzieren.

13. Verfahren gemäß Anspruch 8, wobei das Testmittel eine Verbindung mit einem Molekulargewicht von weniger als 3000, 2000, 1000 oder 500 g/mol; oder

   ein Polypeptid; oder
   ein Polynukleotid ist.

## Revendications

1. Un procédé d'observation de biomarqueurs dans des cellules cutanées et/ou sanguines humaines qui sont en corrélation avec un âge d'un individu, le procédé comprenant :

   (a) le fait d'obtenir de l'ADN génomique à partir de cellules cutanées et/ou de cellules sanguines humaines qui sont issues de l'individu ;
   (b) le fait d'observer le statut de méthylation de la cytosine de l'ADN génomique de l'individu dans la totalité des 391 marqueurs de méthylation de SEQ ID NO : 1 à SEQ ID NO : 391 ;

dans lequel ledit fait d'observer comprend le fait de réaliser un processus de conversion du bisulfite sur l'ADN génomique de sorte que des résidus de cytosine dans l'ADN génomique sont transformés en uracile, tandis que des résidus de 5-méthylcytosine dans l'ADN génomique ne sont pas transformés en uracile ;

(c) le fait de comparer la méthylation du locus CG observée en (b) à la méthylation du locus CG observée dans de l'ADN génomique provenant de cellules cutanées et/ou sanguines humaines issues d'un groupe d'individus d'âges connus ; et

(d) le fait de mettre en corrélation la méthylation du locus CG observée en (b) avec la méthylation du locus CG et des âges connus dans le groupe d'individus ;

de sorte que des biomarqueurs dans des cellules cutanées et/ou sanguines humaines qui sont corrélés à un âge d'un individu sont observés.

2. Le procédé de la revendication 1, comprenant en outre le fait d'utiliser les observations afin d'estimer l'âge de l'individu ; et, facultativement,

le fait de comparer l'âge estimé à l'âge réel de l'individu de sorte à obtenir des informations sur l'espérance de vie de l'individu ; ou

l'estimation de l'âge de l'individu comprend l'utilisation d'une analyse de régression.

3. Le procédé de la revendication 1, dans lequel les cellules cutanées et sanguines sont des fibroblastes, des kératinocytes, des cellules buccales, des cellules endothéliales, des cellules lymphoblastoïdes humain(e)s, et/ou des cellules humaines obtenues à partir de sang, de peau, de derme, d'épiderme ou de salive.

4. Le procédé de la revendication 3, dans lequel l'âge de l'individu est estimé à l'aide d'une moyenne pondérée de marqueurs de méthylation au sein de l'ensemble des 391 marqueurs de méthylation.

5. Le procédé de la revendication 1, dans lequel une méthylation est observée par un processus comprenant le fait d'hybrider de l'ADN génomique obtenu auprès de l'individu avec 391 séquences complémentaires couplées à un substrat et disposées en un réseau ; ou

dans lequel une méthylation est observée dans au moins 100, 200 ou 300 marqueurs de méthylation.

6. Un support lisible par ordinateur tangible comprenant un code lisible par ordinateur qui, lorsqu'il est exécuté par un ordinateur, amène l'ordinateur à réaliser des opérations comprenant :

a) le fait de recevoir des informations correspondant à des niveaux de méthylation d'un ensemble de marqueurs de méthylation dans un échantillon biologique, dans lequel l'ensemble de marqueurs de méthylation comprend 391 marqueurs de méthylation de SEQ ID NO : 1 à SEQ ID NO : 391 ;

b) le fait de déterminer un âge épigénétique par application d'un algorithme de prédiction statistique à des données de méthylation obtenues à partir de l'ensemble de marqueurs de méthylation ; et

c) le fait de déterminer un âge épigénétique à l'aide d'une moyenne pondérée des niveaux de méthylation des 391 marqueurs de méthylation.

7. Un procédé d'observation des effets d'un agent d'essai sur un vieillissement épigénétique de cellules humaines associé à une méthylation génomique, le procédé comprenant :

(a) le fait de combiner l'agent d'essai à des cellules humaines qui ont été isolées ;

(b) le fait d'observer un statut de méthylation dans la totalité des 391 marqueurs de méthylation de SEQ ID NO : 1 à SEQ ID NO : 391 dans de l'ADN génomique provenant des cellules humaines ;

(c) le fait de comparer les observations provenant de (b) à des observations du statut de méthylation dans au moins 10 des 391 marqueurs de méthylation de SEQ ID NO : 1 à SEQ ID NO : 391 dans de l'ADN génomique provenant de cellules humaines témoin non exposées à l'agent d'essai de telle sorte que des effets de l'agent d'essai sur un vieillissement épigénétique dans les cellules humaines associé à une méthylation génomique sont observés.

8. Le procédé de la revendication 7, dans lequel une pluralité d'agents d'essai sont combinés aux cellules humaines.

9. Le procédé de la revendication 7, dans lequel l'agent d'essai est un inhibiteur de sénescence cellulaire.

10. Le procédé de la revendication 7, dans lequel les cellules sont des kératinocytes primaires provenant de donneurs multiples.

11. Le procédé de la revendication 7, dans lequel le procédé observe des cellules humaines *in vitro.*

12. Le procédé de la revendication 11, dans lequel les cellules humaines se différencient *in vitro.*

13. Le procédé de la revendication 8, dans lequel l'agent d'essai est un composé ayant une masse moléculaire inférieure à 3 000, 2 000, 1 000 ou 500 g/mol ; ou

un polypeptide ; ou
un polynucléotide.

FIG. 1

FIG. 2

Globular head domain

1A

Flexible linker

Heptad repeat

α Helical coiled coil domain

1B 2A 2B1 2B2

NLS

Globular tail domain

N

C

Δ50

p.Met540Thr

Non-classical HGPS/ Mandibuloacral dysplasia

Perturbation of globular domain

c.1824C>T

Classical HGPS

High progerin

c.1968+1G>A c.1968+2T>C

Non-classical HGPS

High progerin

c.1968G>A c.1968+5G>A

Atypical Werner syndrome

Low progerin

Age 5.4          Age 11.5          Age 37

FIG. 3

FIG. 4

FIG. 5

Effects of Oestrogen on Neonatal Dermal Fibroblasts

B

DNAmAge (SkinClock)

Oestrogen Concentration (nM)

A  Effects of Y-27632 and Rapamycin on Neonatal Keratinocytes

Control   Y-27632   Rapa   Y-27632 + Rapa

DNAmAge (Skin Clock)

Cumulative Population Doublings

FIG. 6

106

FIG. 7

FIG. 8

FIG. 9

FIG. 10

# AgeAccelSkinClock

Mortality P=9.6e-07, Het. P=0.42

| Cohort | N | Deaths | | HR [95% CI] |
|---|---|---|---|---|
| WHI BA23 White | 998 | 418 | | 1.01 [ 0.98 , 1.04 ] |
| WHI EMPC White | 1096 | 317 | | 1.05 [ 1.02 , 1.08 ] |
| InChianti | 924 | 209 | | 1.04 [ 0.99 , 1.10 ] |
| WHI BA23 AfricanA | 676 | 229 | | 1.01 [ 0.98 , 1.05 ] |
| WHI EMPC AfricanA | 558 | 141 | | 1.05 [ 1.00 , 1.09 ] |
| JHS AfricanA | 1746 | 281 | | 1.04 [ 1.01 , 1.07 ] |
| WHI BA23 Hispanic | 433 | 118 | | 1.07 [ 1.02 , 1.12 ] |
| WHI EMPC Hispanic | 318 | 47 | | 1.05 [ 0.97 , 1.14 ] |
| FE model | | | | 1.03 [ 1.02 , 1.05 ] |

0.90   1.00   1.10   1.20

Hazard Ratio

## FIG. 11

FIG. 12

FIG. 13

FIG. 14

## REFERENCES CITED IN THE DESCRIPTION

### Patent documents cited in the description

- WO 2018027228 A
- US 2016222448 A
- US 20150259742 A
- US 025185
- US 119145
- US 6214556 B
- US 5786146 A
- US 6017704 A
- US 6265171 B
- US 6200756 B
- US 6251594 B
- US 5912147 A
- US 6331393 B
- US 6605432 B
- US 6300071 B
- US 20030148327
- US 20030148326 A
- US 20030143606 A
- US 20030082609 A
- US 20050009059 A
- US 4683202 A
- US 4683195 A
- US 4800159 A
- US 4965188 A
- US 5333675 A
- US 6300070 B
- WO 04096825 A1

### Non-patent literature cited in the description

- **FRAGA et al.** *Trends in Genetics.*, 2007, vol. 23 (8), 413-418
- **RAKYAN et al.** *Genome research.*, 2010, vol. 20 (4), 434-439
- **TESCHENDORFF et al.** *Genome research.*, 2010, vol. 20 (4), 440-446
- **JUNG et al.** *BMC biology.*, 2015, vol. 13 (1), 1-8
- **ZHENG et al.** *Epigenomics.*, 2016, vol. 8 (5), 705-719
- **BOCKLANDT et al.** *PLoS One.*, 2011, vol. 6 (6), e14821
- **GARAGNANI et al.** *Aging Cell.*, 2012, vol. 11 (6), 1132-1134
- **HANNUM et al.** *Mol Cell.*, 2013, vol. 49 (2), 359-367
- **HORVATH.** *Genome Biol.*, 2013, vol. 14 (10), R115
- **WEIDNER et al.** *Genome Biol.*, 2014, vol. 15 (2), R24
- **LIN et al.** *Aging (Albany NY).*, 2016, vol. 8 (2), 394-401
- **HORVATH et al.** *Nat Rev Genet.*, 2018
- **HANNUM et al.** *Mol Cell*, 2013, vol. 49 (2), 359-367
- **HORVATH et al.** *Proc Natl Acad Sci U S A.*, 2014, vol. 111 (43), 15538-15543
- **MARIONI et al.** *Genome Biol.*, 2015, vol. 16 (1), 25
- **MARIONI et al.** *Int J Epidemiol.*, 2015, vol. 44 (4), 1388-1396
- **HORVATH S ; GARAGNANI P ; BACALINI MG ; PIRAZZINI C ; SALVIOLI S ; GENTILINI D ; DI BLASIO AM ; GIULIANI C ; TUNG S ; VINTERS HV.** Accelerated epigenetic aging in Down syndrome. *Aging Cell*, 2015, vol. 14 (3), 491-495
- **HORVATH et al.** *J Infect Dis.*, 2015, vol. 212 (10), 1563-1573
- **LEVINE et al.** *Aging (Albany NY)*, 2015, vol. 7 (9), 690-700
- **LEVINE et al.** *Aging (Albany NY).*, 2015, vol. 7 (12), 1198-1211
- **LEVINE et al.** *Proc Natl Acad Sci U S A.*, 2016, vol. 113 (33), 9327-9332
- **CHEN et al.** *Aging (Albany NY).*, 2016, vol. 8 (9), 1844-1865
- **QUACH et al.** *Aging (Albany NY).*, 2017, vol. 9 (2), 419-446
- **DUGUE et al.** *Int J Cancer.*, 2017
- **SIMPKIN et al.** *Int J Epidemiol.*, 2017, vol. 46 (2), 549-558
- **MAIERHOFER et al.** *Aging (Albany NY).*, 2017, vol. 9 (4), 1143-1152
- **BOCKLANDT et al.** *PLoS One.*, 2011
- **HANNUM.** *Mol Cell*, 2013
- **HORVATH.** *Genome Biol.*, 2013, vol. 14 (R115)
- **WEIDNER.** *Genome Biol.*, 2014
- **CHRISTIANSEN et al.** *Aging Cell.*, 2015
- **PERNA et al.** *Clinical Epigenetics.*, 2016, vol. 8 (1), 1-7

- **HANNUM et al.** Genome-Wide Methylation Profiles Reveal Quantitative Views Of Human Aging Rates. *Molecular Cell*, 2013, vol. 49 (2), 359-367
- **MATSUYAMA et al.** Epigenetic clock analysis of human fibroblasts in vitro: effects of hypoxia, donor age, and expression of hTERT and SV40 largeT. *AGING*, 2019, vol. 11, 1-11
- Technical Note: Epigenetics. CpG Loci Identification ILLUMINA Inc., 2010
- **OAKELEY, E. J.** *Pharmacology & Therapeutics*, 1999, vol. 84, 389-400
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.*, 1991, vol. 19, 4967
- **ECKERT et al.** *PCR Methods and Applications*, 1991, vol. 1, 17
- PCR. IRL Press
- **OLEK et al.** *Nuc. Acids Res.*, 1994, vol. 24, 5064-6
- **SYVANEN**. *Nature Rev. Gen.*, 2001, vol. 2, 930-942
- **FRAGA MF ; ESTELLER M**. Epigenetics and aging: the targets and the marks. *Trends in Genetics.*, 2007, vol. 23 (8), 413-418
- **RAKYAN VK ; DOWN TA ; MASLAU S ; ANDREW T ; YANG TP ; BEYAN H ; WHITTAKER P ; MCCANN OT ; FINER S ; VALDES AM**. Human aging-associated DNA hypermethylation occurs preferentially at bivalent chromatin domains. *Genome research.*, 2010, vol. 20 (4), 434-439
- **TESCHENDORFF AE ; MENON U ; GENTRY-MAHARAJ A ; RAMUS SJ ; WEISENBERGER DJ ; SHEN H ; CAMPAN M ; NOUSHMEHR H ; BELL CG ; MAXWELL AP**. Age-dependent DNA methylation of genes that are suppressed in stem cells is a hallmark of cancer. *Genome research.*, 2010, vol. 20 (4), 440-446
- **JUNG M ; PFEIFER GP**. Aging and DNA methylation. *BMC biology*, 2015, vol. 13 (1), 1-8
- **ZHENG SC ; WIDSCHWENDTER M ; TESCHEN-DORFF AE**. Epigenetic drift, epigenetic clocks and cancer risk. *Epigenomics*, 2016, vol. 8 (5), 705-719
- **BOCKLANDT S ; LIN W ; SEHL ME ; SANCHEZ FJ ; SINSHEIMER JS ; HORVATH S ; VILAIN E**. Epigenetic predictor of age. *PLoS One*, 2011, vol. 6 (6), e14821
- **GARAGNANI P ; BACALINI MG ; PIRAZZINI C ; GORI D ; GIULIANI C ; MARI D ; DI BLASIO AM ; GENTILINI D ; VITALE G ; COLLINO S**. Methylation of ELOVL2 gene as a new epigenetic marker of age. *Aging Cell.*, 2012, vol. 11 (6), 1132-1134

- **HANNUM G ; GUINNEY J ; ZHAO L ; ZHANG L ; HUGHES G ; SADDA S ; KLOTZLE B ; BIBIKOVA M ; FAN JB ; GAO Y**. Genome-wide methylation profiles reveal quantitative views of human aging rates. *Mol Cell.*, 2013, vol. 49 (2), 359-367
- **HORVATH S**. DNA methylation age of human tissues and cell types. *Genome Biol.*, 2013, vol. 14 (10), R115
- **WEIDNER CI ; LIN Q ; KOCH CM ; EISELE L ; BEIER F ; ZIEGLER P ; BAUERSCHLAG DO ; JOCKEL KH ; ERBEL R ; MUHLEISEN TW**. Aging of blood can be tracked by DNA methylation changes at just three CpG sites. *Genome Biol.*, 2014, vol. 15 (2), R24
- **LIN Q ; WEIDNER CI ; COSTA IG ; MARIONI RE ; FERREIRA MR ; DEARY IJ ; WAGNER W**. DNA methylation levels at individual age-associated CpG sites can be indicative for life expectancy.. *Aging (Albany NY).*, 2016, vol. 8 (2), 394-401
- **HORVATH S ; RAJ K**. DNA methylation-based biomarkers and the epigenetic clock theory of ageing.. *Nat Rev Genet.*, 2018
- **NWANAJI-ENWEREM JC ; WEISSKOPF MG ; BACCARELLI AA**. Multi-tissue DNA methylation age: Molecular relationships and perspectives for advancing biomarker utility. *Ageing Res Rev.*, 2018, vol. 45, 15-23
- **HORVATH S ; ERHART W ; BROSCH M ; AMMERPOHL O ; VON SCHONFELS W ; AHRENS M ; HEITS N ; BELL JT ; TSAI PC ; SPECTOR TD**. Obesity accelerates epigenetic aging of human liver.. *Proc Natl Acad Sci U S A.*, 2014, vol. 111 (43), 15538-15543
- **MARIONI R ; SHAH S ; MCRAE A ; CHEN B ; COLICINO E ; HARRIS S ; GIBSON J ; HENDERS A ; REDMOND P ; COX S**. DNA methylation age of blood predicts all-cause mortality in later life. *Genome Biol.*, 2015, vol. 16 (1), 25
- **HORVATH S ; LEVINE AJ**. HIV-1 Infection Accelerates Age According to the Epigenetic Clock. *J Infect Dis.*, 2015, vol. 212 (10), 1563-1573
- **HORVATH S ; MAH V ; LU AT ; WOO JS ; CHOI OW ; JASINSKA AJ ; RIANCHO JA ; TUNG S ; COLES NS ; BRAUN J**. The cerebellum ages slowly according to the epigenetic clock. *Aging (Albany NY).*, 2015, vol. 7 (5), 294-306
- **LEVINE ME ; HOSGOOD HD ; CHEN B ; ABSHER D ; ASSIMES T ; HORVATH S.** DNA methylation age of blood predicts future onset of lung cancer in the women's health initiative. *Aging (Albany NY).*, 2015, vol. 7 (9), 690-700
- **LEVINE ME ; LU AT ; CHEN BH ; HERNANDEZ DG ; SINGLETON AB ; FERRUCCI L ; BANDINELLI S ; SALFATI E ; MANSON JE ; QUACH A**. Menopause accelerates biological aging.. *Proc Natl Acad Sci U S A.*, 2016, vol. 113 (33), 9327-9332

- **CHEN BH** ; **MARIONI RE** ; **COLICINO E** ; **PETERS MJ** ; **WARD-CAVINESS CK** ; **TSAI PC** ; **ROETKER NS** ; **JUST AC** ; **DEMERATH EW** ; **GUAN W**. DNA methylation-based measures of biological age: meta-analysis predicting time to death. *Aging (Albany NY).*, 2016, vol. 8 (9), 1844-1865
- **QUACH A** ; **LEVINE ME** ; **TANAKA T** ; **LU AT** ; **CHEN BH** ; **FERRUCCI L** ; **RITZ B** ; **BANDINELLI S** ; **NEUHOUSER ML** ; **BEASLEY JM**. Epigenetic clock analysis of diet, exercise, education, and lifestyle factors. *Aging (Albany NY).*, 2017, vol. 9 (2), 419-446
- **DUGUE PA** ; **BASSETT JK** ; **JOO JE** ; **JUNG CH** ; **MING WONG E** ; **MORENO-BETANCUR M** ; **SCHMIDT D** ; **MAKALIC E** ; **LI S** ; **SEVERI G**. DNA methylation-based biological aging and cancer risk and survival: Pooled analysis of seven prospective studies. *Int J Cancer.*, 2017
- **SIMPKIN AJ** ; **HOWE LD** ; **TILLING K** ; **GAUNT TR** ; **LYTTLETON O** ; **MCARDLE WL** ; **RING SM** ; **HORVATH S** ; **SMITH GD** ; **RELTON CL**. The epigenetic clock and physical development during childhood and adolescence: longitudinal analysis from a UK birth cohort. *Int J Epidemiol.*, 2017, vol. 46 (2), 549-558
- **MAIERHOFER A** ; **FLUNKERT J** ; **OSHIMA J** ; **MARTIN GM** ; **HAAF T** ; **HORVATH S**. Accelerated epigenetic aging in Werner syndrome. *Aging (Albany NY).*, 2017, vol. 9 (4), 1143-1152
- **WALKER RF** ; **LIU JS** ; **PETERS BA** ; **RITZ BR** ; **WU T** ; **OPHOFF RA** ; **HORVATH S**. Epigenetic age analysis of children who seem to evade aging. *Aging (Albany NY)*, 2015, vol. 7 (5), 334-339
- **MERIDETH MA** ; **GORDON LB** ; **CLAUSS S** ; **SACHDEV V** ; **SMITH AC** ; **PERRY MB** ; **BREWER CC** ; **ZALEWSKI C** ; **KIM HJ** ; **SOLOMON B**. Phenotype and course of Hutchinson-Gilford progeria syndrome. *N Engl J Med.*, 2008, vol. 358 (6), 592-604
- **GORDON LB** ; **KLEINMAN ME** ; **MASSARO J** ; **D'AGOSTINO RB, SR.** ; **SHAPPELL H** ; **GERHARD-HERMAN M** ; **SMOOT LB** ; **GORDON CM** ; **CLEVELAND RH** ; **NAZARIAN A**. Clinical Trial of the Protein Farnesylation Inhibitors Lonafamib, Pravastatin, and Zoledronic Acid in Children With Hutchinson-Gilford Progeria Syndrome. *Circulation.*, 2016, vol. 134 (2), 114-125
- **ERIKSSON M** ; **BROWN W** ; **GORDON L** ; **GLYNN M** ; **SINGER J** ; **SCOTT L** ; **ERDOS M** ; **ROBBINS C** ; **MOSES T** ; **BERGLUND P**. Recurrent de novo point mutations in lamin A cause Hutchinson-Gilford progeria syndrome.. *Nature*, 2003, vol. 423 (6937), 293-298
- **MOULSON CL** ; **FONG LG** ; **GARDNER JM** ; **FARBER EA** ; **GO G** ; **PASSARIELLO A** ; **GRANGE DK** ; **YOUNG SG** ; **MINER JH**. Increased progerin expression associated with unusual LMNA mutations causes severe progeroid syndromes. *Hum Mutat.*, 2007, vol. 28 (9), 882-889
- **BAR DZ** ; **ARLT MF** ; **BRAZIER JF** ; **NORRIS WE** ; **CAMPBELL SE** ; **CHINES P** ; **LARRIEU D** ; **JACKSON SP** ; **COLLINS FS** ; **GLOVER TW**. A novel somatic mutation achieves partial rescue in a child with Hutchinson-Gilford progeria syndrome.. *J Med Genet.*, 2017, vol. 54 (3), 212-216
- **AHMAD Z** ; **ZACKAI E** ; **MEDNE L** ; **GARG A**. Early onset mandibuloacral dysplasia due to compound heterozygous mutations in ZMPSTE24. *Am J Med Genet A.*, 2010, vol. 152A (11), 2703-2710
- **BARROWMAN J** ; **WILEY PA** ; **HUDON-MILLER SE** ; **HRYCYNA CA** ; **MICHAELIS S**. Human ZMPSTE24 disease mutations: residual proteolytic activity correlates with disease severity. *Hum Mol Genet.*, 2012, vol. 21 (18), 4084-4093
- **BAI S** ; **LOZADA A** ; **JONES MC** ; **DIETZ HC** ; **DEMPSEY M** ; **DAS S**. Mandibuloacral Dysplasia Caused by LMNA Mutations and Uniparental Disomy. *Case Rep Genet.*, 2014, vol. 2014, 508231
- **VERSTRAETEN VL** ; **BROERS JL** ; **VAN STEENSEL MA** ; **ZINN-JUSTIN S** ; **RAMAEKERS FC** ; **STEIJLEN PM** ; **KAMPS M** ; **KUIJPERS HJ** ; **MERCKX D** ; **SMEETS HJ**. Compound heterozygosity for mutations in LMNA causes a progeria syndrome without prelamin A accumulation. *Hum Mol Genet.*, 2006, vol. 15 (16), 2509-2522
- **HISAMA FM** ; **LESSEL D** ; **LEISTRITZ D** ; **FRIEDRICH K** ; **MCBRIDE KL** ; **PASTORE MT** ; **GOTTESMAN GS** ; **SAHA B** ; **MARTIN GM** ; **KUBISCH C**. Coronary artery disease in a Werner syndrome-like form of progeria characterized by low levels of progerin, a splice variant of lamin A. *Am J Med Genet A.*, 2011, vol. 155A (12), 3002-3006
- **BARTHELEMY F** ; **NAVARRO C** ; **FAYEK R** ; **DA SILVA N** ; **ROLL P** ; **SIGAUDY S** ; **OSHIMA J** ; **BONNE G** ; **PAPADOPOULOU-LEGBELOU K** ; **EVANGELIOU AE**. Truncated prelamin A expression in HGPS-like patients: a transcriptional study. *Eur J Hum Genet.*, 2015, vol. 23 (8), 1051-1061
- **GORDON LB** ; **MASSARO J** ; **D'AGOSTINO RB, SR.** ; **CAMPBELL SE** ; **BRAZIER J** ; **BROWN WT** ; **KLEINMAN ME** ; **KIERAN MW**. Impact of farnesylation inhibitors on survival in Hutchinson-Gilford progeria syndrome. *Circulation*, 2014, vol. 130 (1), 27-34
- **LU AT** ; **XUE L** ; **SALFATI EL** ; **CHEN BH** ; **FERRUCCI L** ; **LEVY D** ; **JOEHANES R** ; **MURABITO JM** ; **KIEL DP** ; **TSAI PC**. GWAS of epigenetic aging rates in blood reveals a critical role for TERT.. *Nat Commun.*, 2018, vol. 9 (1), 387

- **LOWE D** ; **HORVATH S** ; **RAJ K**. Epigenetic clock analyses of cellular senescence and ageing. *Oncotarget*, 2016, vol. 7 (8), 8524-8531
- **CAO K** ; **GRAZIOTTO JJ** ; **BLAIR CD** ; **MAZZULLI JR** ; **ERDOS MR** ; **KRAINC D** ; **COLLINS FS**. Rapamycin reverses cellular phenotypes and enhances mutant protein clearance in Hutchinson-Gilford progeria syndrome cells.. *Sci Transl Med.*, 2011, vol. 3 (89), 89-58
- **PARK SK** ; **SHIN OS**. Metformin alleviates ageing cellular phenotypes in Hutchinson-Gilford progeria syndrome dermal fibroblasts. *Exp Dermatol.*, 2017, vol. 26 (10), 889-895
- **KUBBEN N** ; **ZHANG W** ; **WANG L** ; **VOSS TC** ; **YANG J** ; **QU J** ; **LIU GH** ; **MISTELI T**. Repression of the Antioxidant NRF2 Pathway in Premature Aging.. *Cell*, 2016, vol. 165 (6), 1361-1374
- **FRIEDMAN J** ; **HASTIE T** ; **TIBSHIRANI R**. Regularization Paths for Generalized Linear Models via Coordinate Descent. *Journal of Statistical Software.*, 2010, vol. 33 (1), 1-22
- **TRICHE TJ** ; **WEISENBERGER DJ** ; **VAN DEN BERG D** ; **LAIRD PW** ; **SIEGMUND KD**. Low-level processing of Illumina Infinium DNA Methylation BeadArrays.. *Nucleic Acids Research*, 2013, vol. 41 (7), e90-e90
- **HUB CJ** ; **ZHANG B** ; **VICTOR MB** ; **DAHIYA S** ; **BATISTA LF** ; **HORVATH S** ; **YOO AS**. Maintenance of age in human neurons generated by microRNA-based neuronal conversion of fibroblasts. *Elife*, 2016, vol. 5, e18648
- **LEVINE ME** ; **LU AT** ; **QUACH A** ; **CHEN BH** ; **ASSIMES TL** ; **BANDINELLI S** ; **HOU L** ; **BACCARELLI AA** ; **STEWART JD** ; **LI Y**. An epigenetic biomarker of aging for lifespan and healthspan. *Aging (Albany NY)*, 2018
- **HOUSEMAN E** ; **ACCOMANDO W** ; **KOESTLER D** ; **CHRISTENSEN B** ; **MARSIT C** ; **NELSON H** ; **WIENCKE J** ; **KELSEY K**. DNA methylation arrays as surrogate measures of cell mixture distribution. *BMC Bioinformatics*, 2012, vol. 13 (1), 86
- **HORVATH S** ; **GURVEN M** ; **LEVINE ME** ; **TRUMBLE BC** ; **KAPLAN H** ; **ALLAYEE H** ; **RITZ BR** ; **CHEN B** ; **LU AT** ; **RICKABAUGH TM**. An epigenetic clock analysis of race/ethnicity, sex, and coronary heart disease. *Genome Biol.*, 2016, vol. 17 (1), 171
- **HORVATH S** ; **RITZ BR**. Increased epigenetic age and granulocyte counts in the blood of Parkinson's disease patients.. *Aging (Albany NY).*, 2015, vol. 7 (12), 1130-1142
- **ROJAS D** ; **RAGER JE** ; **SMEESTER L** ; **BAILEY KA** ; **DROBNA Z** ; **RUBIO-ANDRADE M** ; **STYBLO M** ; **GARCIA-VARGAS G** ; **FRY RC**. Prenatal arsenic exposure and the epigenome: identifying sites of 5-methylcytosine alterations that predict functional changes in gene expression in newborn cord blood and subsequent birth outcomes. *Toxicol Sci.*, 2015, vol. 143 (1), 97-106
- **KNIGHT AK** ; **CRAIG JM** ; **THEDA C** ; **BAKVAD-HANSEN M** ; **BYBJERG-GRAUHOLM J** ; **HANSEN CS** ; **HOLLEGAARD MV** ; **HOUGAARD DM** ; **MORTENSEN PB** ; **WEINSHEIMER SM**. An epigenetic clock for gestational age at birth based on blood methylation data.. *Genome Biology.*, 2016, vol. 17 (1), 206
- **VIECHTBAUER W**. Conducting Meta-Analyses in R with the metafor Package. *J Statistical Software.*, 2010, vol. 36 (3), 1-48
- **BETZ, C.** ; **HALL, M. N.** Where is mTOR and what is it doing there?. *J Cell Biol*, 2013, vol. 203, 563-74
- **BJEDOV, I.** ; **TOIVONEN, J. M.** ; **KERR, F.** ; **SLACK, C.** ; **JACOBSON, J.** ; **FOLEY, A.** ; **PARTRIDGE, L.** Mechanisms of life span extension by rapamycin in the fruit fly Drosophila melanogaster. *Cell Metab*, 2010, vol. 11, 35-46
- **BLAGOSKLONNY, M. V**. Rapamycin, proliferation and geroconversion to senescence. *Cell Cycle*, 2018, 1-11
- **BOCKLANDT, S.** ; **LIN, W.** ; **SEHL, M. E.** ; **SANCHEZ, F. J.** ; **SINSHEIMER, J. S.** ; **HORVATH, S.** ; **VILAIN, E.** Epigenetic predictor of age. *PLoS One*, 2011, vol. 6, e14821
- **CHOI, J.** ; **CHEN, J.** ; **SCHREIBER, S. L.** ; **CLARDY, J.** Structure of the FKBP12-rapamycin complex interacting with the binding domain of human FRAP.. *Science*, 1996, vol. 273, 239-42
- **CORNU, M.** ; **ALBERT, V.** ; **HALL, M. N.** mTOR in aging, metabolism, and cancer.. *Curr Opin Genet Dev*, 2013, vol. 23, 53-62
- **EHNINGER, D.** ; **NEFF, F.** ; **XIE, K.** Longevity, aging and rapamycin. *Cell Mol Life Sci*, 2014, vol. 71, 4325-46
- **EIPEL, M.** ; **MAYER, F.** ; **ARENT, T.** ; **FERREIRA, M. R.** ; **BIRKHOFER, C.** ; **GERSTENMAIER, U.** ; **COSTA, I. G.** ; **RITZ-TIMME, S.** ; **WAGNER, W.** Epigenetic age predictions based on buccal swabs are more precise in combination with cell type-specific DNA methylation signatures. *Aging (Albany NY)*, 2016, vol. 8, 1034-48
- **FLORATH, I.** ; **BUTTERBACH, K.** ; **MULLER, H.** ; **BEWERUNGE-HUDLER, M.** ; **BRENNER, H.** Cross-sectional and longitudinal changes in DNA methylation with age: an epigenome-wide analysis revealing over 60 novel age-associated CpG sites. *Hum Mol Genet*, 2014, vol. 23, 1186-201
- **FORTIN, J. P.** ; **TRICHE, T. J., JR.** ; **HANSEN, K. D.** Preprocessing, normalization and integration of the Illumina HumanMethylationEPIC array with minfi. *Bioinformatics*, 2017, vol. 33, 558-560
- **GREEN, H.** ; **RHEINWALD, J. G.** ; **SUN, T. T.** Properties of an epithelial cell type in culture: the epidermal keratinocyte and its dependence on products of the fibroblast. *Prog Clin Biol Res*, 1977, vol. 17, 493-500

- **HANNUM, G.** ; **GUINNEY, J.** ; **ZHAO, L.** ; **ZHANG, L.** ; **HUGHES, G.** ; **SADDA, S.** ; **KLOTZLE, B.** ; **BIBIKOVA, M.** ; **FAN, J. B.** ; **GAO, Y.** Genome-wide methylation profiles reveal quantitative views of human aging rates. *Mol Cell*, 2013, vol. 49, 359-67
- **HARRISON, D. E.** ; **STRONG, R.** ; **SHARP, Z. D.** ; **NELSON, J. F.** ; **ASTLE, C. M.** ; **FLURKEY, K.** ; **NADON, N. L.** ; **WILKINSON, J. E.** ; **FRENKEL, K.** ; **CARTER, C. S.** Rapamycin fed late in life extends lifespan in genetically heterogeneous mice.. *Nature*, 2009, vol. 460, 392-5
- **HEILBRONN, L. K.** ; **RAVUSSIN, E.** Calorie restriction and aging: review of the literature and implications for studies in humans. *Am J Clin Nutr*, 2003, vol. 78, 361-9
- **HERNANDEZ, D. G.** ; **NALLS, M. A.** ; **GIBBS, J. R.** ; **AREPALLI, S.** ; **VAN DER BRUG, M.** ; **CHONG, S.** ; **MOORE, M.** ; **LONGO, D. L.** ; **COOKSON, M. R.** ; **TRAYNOR, B. J.** Distinct DNA methylation changes highly correlated with chronological age in the human brain. *Hum Mol Genet*, 2011, vol. 20, 1164-72
- **HERRANZ, N.** ; **GALLAGE, S.** ; **MELLONE, M.** ; **WUESTEFELD, T.** ; **KLOTZ, S.** ; **HANLEY, C. J.** ; **RAGUZ, S.** ; **ACOSTA, J. C.** ; **INNES, A. J.** ; **BANITO, A.** mTOR regulates MAPKAPK2 translation to control the senescence-associated secretory phenotype.. *Nat Cell Biol*, 2015, vol. 17, 1205-17
- **HORVATH, S.** DNA methylation age of human tissues and cell types. *Genome Biol*, 2013, vol. 14, R115
- **HORVATH, S.** ; **ERHART, W.** ; **BROSCH, M.** ; **AMMERPOHL, O.** ; **VON SCHONFELS, W.** ; **AHRENS, M.** ; **HEITS, N.** ; **BELL, J. T.** ; **TSAI, P. C.** ; **SPECTOR, T. D.** Obesity accelerates epigenetic aging of human liver.. *Proc Natl Acad Sci USA*, 2014, vol. 111, 15538-43
- **HORVATH, S.** ; **GARAGNANI, P.** ; **BACALINI, M. G.** ; **PIRAZZINI, C.** ; **SALVIOLI, S.** ; **GENTILINI, D.** ; **DI BLASIO, A. M.** ; **GIULIANI, C.** ; **TUNG, S.** ; **VINTERS, H. V.** Accelerated epigenetic aging in Down syndrome. *Aging Cell*, 2015
- **HORVATH, S.** ; **GURVEN, M.** ; **LEVINE, M. E.** ; **TRUMBLE, B. C.** ; **KAPLAN, H.** ; **ALLAYEE, H.** ; **RITZ, B. R.** ; **CHEN, B.** ; **LU, A. T.** ; **RICKABAUGH, T. M.** An epigenetic clock analysis of race/ethnicity, sex, and coronary heart disease. *Genome Biol*, 2016, vol. 17, 171
- **HORVATH, S., LANGFELDER, P., KWAK, S., AARONSON, J., ROSINSKI, J.,VOGT, T. F., ESZES, M., FAULL, R. L., CURTIS, M. A., WALDVOGEL, H. J., CHOI,O. W., TUNG, S., VINTERS, H. V., COPPOLA, G. & YANG, X. W.** Huntington's disease accelerates epigenetic aging of human brain and disrupts DNA methylation levels. *Aging (Albany NY)*, 2016, vol. 8, 1485-512
- **HORVATH, S.** ; **MAH, V.** ; **LU, A. T.** ; **WOO, J. S.** ; **CHOI, O. W.** ; **JASINSKA, A. J.** ; **RIANCHO, J. A.** ; **TUNG, S.** ; **COLES, N. S.** ; **BRAUN, J.** The cerebellum ages slowly according to the epigenetic clock. *Aging (Albany NY)*, 2015, vol. 7, 294-306
- **HORVATH, S.** ; **OSHIMA, J.** ; **MARTIN, G. M.** ; **LU, A. T.** ; **QUACH, A.** ; **COHEN, H.** ; **FELTON, S.** ; **MATSUYAMA, M.** ; **LOWE, D.** ; **KABACIK, S.** Epigenetic clock for skin and blood cells applied to Hutchinson Gilford Progeria Syndrome and ex vivo studies. *Aging (Albany NY)*, 2018
- **HORVATH, S.** ; **RAJ, K.** DNA methylation-based biomarkers and the epigenetic clock theory of ageing.. *Nat Rev Genet.*, 2018
- **HORVATH, S.** ; **RITZ, B. R.** Increased epigenetic age and granulocyte counts in the blood of Parkinson's disease patients.. *Aging (Albany NY)*, 2015, vol. 7, 1130-42
- **ILAGAN, E.** ; **MANNING, B. D.** Emerging role of mTOR in the response to cancer therapeutics. *Trends Cancer*, 2016, vol. 2, 241-251
- **JOHNSON, S. C.** ; **RABINOVITCH, P. S.** ; **KAEBERLEIN, M.** mTOR is a key modulator of ageing and age-related disease.. *Nature*, 2013, vol. 493, 338-45
- **KABACIK, S.** ; **HORVATH, S.** ; **COHEN, H.** ; **RAJ, K.** Epigenetic ageing is distinct from senescence-mediated ageing and is not prevented by telomerase expression. *Aging (Albany NY)*, 2018, vol. 10, 2800-2815
- **KAKUMOTO, K.** ; **IKEDA, J.** ; **OKADA, M.** ; **MORII, E.** ; **ONEYAMA, C.** mLST8 Promotes mTOR-Mediated Tumor Progression. *PLoS One*, 2015, vol. 10, e0119015
- **KIM, J.** ; **GUAN, K. L.** mTOR as a central hub of nutrient signalling and cell growth.. *Nat Cell Biol*, 2019, vol. 21, 63-71
- **KOCH, C. M.** ; **WAGNER, W.** Epigenetic-aging-signature to determine age in different tissues.. *Aging (Albany NY)*, 2011, vol. 3, 1018-27
- **LEONTIEVA, O. V.** ; **BLAGOSKLONNY, M. V.** Gerosuppression by pan-mTOR inhibitors. *Aging (Albany NY)*, 2016, vol. 8, 3535-3551
- **LEONTIEVA, O. V.** ; **BLAGOSKLONNY, M. V.** While reinforcing cell cycle arrest, rapamycin and Torins suppress senescence in UVA-irradiated fibroblasts. *Oncotarget*, 2017, vol. 8, 109848-109856
- **LEONTIEVA, O. V.** ; **DEMIDENKO, Z. N.** ; **BLAGOSKLONNY, M. V.** Contact inhibition and high cell density deactivate the mammalian target of rapamycin pathway, thus suppressing the senescence program.. *Proc Natl Acad Sci U S A*, 2014, vol. 111, 8832-7
- **LEONTIEVA, O. V.** ; **DEMIDENKO, Z. N.** ; **BLAGOSKLONNY, M. V.** Dual mTORC1/C2 inhibitors suppress cellular geroconversion (a senescence program). *Oncotarget*, 2015, vol. 6, 23238-48

- **LEVINE, M. E.** ; **LU, A. T.** ; **CHEN, B. H.** ; **HERNANDEZ, D. G.** ; **SINGLETON, A. B.** ; **FERRUCCI, L.** ; **BANDINELLI, S.** ; **SALFATI, E.** ; **MANSON, J. E.** ; **QUACH, A.** Menopause accelerates biological aging.. *Proc Natl Acad Sci U S A*, 2016, vol. 113, 9327-32
- **LU, A. T.** ; **HANNON, E.** ; **LEVINE, M. E.** ; **HAO, K.** ; **CRIMMINS, E. M.** ; **LUNNON, K.** ; **KOZLENKOV, A.** ; **MILL, J.** ; **DRACHEVA, S.** ; **HORVATH, S.** Genetic variants near MLST8 and DHX57 affect the epigenetic age of the cerebellum.. *Nat Commun*, 2016, vol. 7, 10561
- **MUNOZ-ESPIN, D.** ; **SERRANO, M.** Cellular senescence: from physiology to pathology.. *Nat Rev Mol Cell Biol*, 2014, vol. 15, 482-96
- **POWERS, R. W., 3RD** ; **KAEBERLEIN, M.** ; **CALDWELL, S. D.** ; **KENNEDY, B. K.** ; **FIELDS, S.** Extension of chronological life span in yeast by decreased TOR pathway signaling. *Genes Dev*, 2006, vol. 20, 174-84
- **RAYESS, H.** ; **WANG, M. B.** ; **SRIVATSAN, E. S.** Cellular senescence and tumor suppressor gene p16. *Int J Cancer*, 2012, vol. 130, 1715-25
- **RHEINWALD, J. G.** ; **GREEN, H.** Serial cultivation of strains of human epidermal keratinocytes: the formation of keratinizing colonies from single cells.. *Cell*, 1975, vol. 6, 331-43
- **RICE, R. H.** ; **QIN, Q.** ; **PILATO, A.** ; **RUBIN, A. L.** Keratinocyte differentiation markers: involucrin, transglutaminase, and toxicity.. *J Natl Cancer Inst Monogr*, 1992, 87-91
- **SHARP, Z. D.** ; **CURIEL, T. J.** ; **LIVI, C. B.** Chronic mechanistic target of rapamycin inhibition: preventing cancer to delay aging, or vice versa?. *Interdiscip Top Gerontol*, 2013, vol. 38, 1-16

- **STANFEL, M. N.** ; **SHAMIEH, L. S.** ; **KAEBERLEIN, M.** ; **KENNEDY, B. K.** The TOR pathway comes of age. *Biochim Biophys Acta*, 2009, vol. 1790, 1067-74
- **TRICHE, T. J., JR.** ; **WEISENBERGER, D. J.** ; **VAN DEN BERG, D.** ; **LAIRD, P. W.** ; **SIEGMUND, K. D.** Low-level processing of Illumina Infinium DNA Methylation BeadArrays.. *Nucleic Acids Res*, 2013, vol. 41, e90
- **WANG, R.** ; **SUNCHU, B.** ; **PEREZ, V. I**. Rapamycin and the inhibition of the secretory phenotype.. *Exp Gerontol*, 2017, vol. 94, 89-92
- **WEICHHART, T.** mTOR as Regulator of Lifespan, Aging, and Cellular Senescence: A Mini-Review. *Gerontology*, 2018, vol. 64, 127-134
- **WEIDNER, C. I.** ; **LIN, Q.** ; **KOCH, C. M.** ; **EISELE, L.** ; **BEIER, F.** ; **ZIEGLER, P.** ; **BAUERSCHLAG, D. O.** ; **JOCKEL, K. H.** ; **ERBEL, R.** ; **MUHLEISEN, T. W.** Aging of blood can be tracked by DNA methylation changes at just three CpG sites. *Genome Biol*, 2014, vol. 15, R24
- **YANG, Z.** ; **WONG, A.** ; **KUH, D.** ; **PAUL, D. S.** ; **RAKYAN, V. K.** ; **LESLIE, R. D.** ; **ZHENG, S. C.** ; **WIDSCHWENDTER, M.** ; **BECK, S.** ; **TESCHENDORFF, A. E.** Correlation of an epigenetic mitotic clock with cancer risk. *Genome Biol*, 2016, vol. 17, 205
- **ZHANG, Y.** ; **BOKOV, A.** ; **GELFOND, J.** ; **SOTO, V.** ; **IKENO, Y.** ; **HUBBARD, G.** ; **DIAZ, V.** ; **SLOANE, L.** ; **MASLIN, K.** ; **TREASTER, S.** Rapamycin extends life and health in C57BL/6 mice. *J Gerontol A Biol Sci Med Sci*, 2014, vol. 69, 119-30